# EUROPEAN PATENT SPECIFICATION

(11) **EP 3 548 510 B1**
(45) Date of publication and mention of the grant of the patent: **02.10.2024**
(21) Application number: 17818367.9
(22) Date of filing: 30.11.2017
(51) Int. Cl.: C07K 14/705

(54) **COMPOUNDS USEFUL FOR THE TREATMENT AND/OR CARE OF THE SKIN, HAIR, NAILS AND/OR MUCOUS MEMBRANES**
VERBUNDSTOFFE, DIE ZUR BEHANDLUNG UND/ODER PFLEGE DER HAUT, HAARE UND/ODER SCHLEIMHÄUTE NÜTZLICH SIND
COMPOSÉS UTILES AU TRAITEMENT ET/OU AU SOIN DE LA PEAU, DES CHEVEUX, DES ONGLES ET OU DES MUQUEUSES

(30) Priority: 30.11.2016 EP 16382581
(43) Date of publication of application: 09.10.2019
(73) Proprietor: Lubrizol Advanced Materials, Inc., Cleveland, OH 44141-3247 (US)
(72) Inventor: VAN DEN NEST, Wim, 08850 Barcelona (ES); RODRIGUEZ BIELSA, Katalina, 08850 Barcelona (ES); LAPORTA, Olga, 08850 Barcelona (ES); GARCIA SANZ, Nuria, 08850 Barcelona (ES); ALMIÑANA DOMÈNECH, Nuria, 08850 Barcelona (ES)
(74) Representative: Bradley, Josephine Mary
(86) International application number: PCT/US2017/063871
(87) International publication number: WO 2018/102508

(56) References cited:
- EP-A1- 2 123 673
- US-A1- 2011 206 752
- US-A1- 2016 206 543
- BLANES-MIRA C ET AL: "A SYNTHETIC HEXAPEPTIDE (ARGIRELINE) WITH ANTIWRINKLE ACTIVITY", INTERNATIONAL JOURNAL OF COSMETIC SCIENCE, KLUWER ACADEMIC PUBLISHERS, DORDRECHT, NL, vol. 24, no. 5, 1 January 2002 (2002-01-01), pages 303 - 310, XP009045291, ISSN: 0142-5463, DOI: 10.1046/J.1467-2494.2002.00153.X

## Description

### FIELD OF THE INVENTION

The invention relates to the use of compounds which are effective in maintaining and/or improving the physical and immunological barrier functions of the skin. The compounds are useful in the treatment and/or care of the skin, hair, nails and/or mucous membranes. In particular, the compounds are useful for maintaining and/or improving the hydration of the skin and maintaining the microbial equilibrium of the skin.

### BACKGROUND OF THE INVENTION

The major function of the skin is to form a barrier between the inside of the host and the environment. The skin must protect the organism from chemicals, ultraviolet light, mechanical insults, and pathogenic microorganisms. Most importantly, the skin must provide an efficient permeability barrier that prevents the loss of water and electrolytes. The skin is composed of epidermis, dermis and hypodermis. The epidermis consists of 4 layers: the undifferentiated basal layer, where cell proliferation occurs, the stratum spinosum, the stratum granulosum (SG), and the acellular layers of the stratum corneum. The permeability barrier localizes primarily to the stratum corneum, but also to the stratum granulosum¹.

In the stratum corneum (SC), three physiological parameters contribute to the quality of the barrier function: the stratum corneum thickness, the intercellular lipid content, and the lipid organization. All parameters are closely related to skin topography or micro-relief and to water content. In the stratum corneum, corneocytes and lamellar bodies are essential in providing this permeability barrier. Corneocytes are keratinocytes that have undergone terminal differentiation with degradation of the nucleus, loss of DNA, and formation of a unique cornified envelope that serves as a platform for a neutral lipid extracellular matrix that contain ceramides, cholesterol, and free fatty acids¹. The thickness of the stratum corneum depends on the balance between desquamation and replenishing of the corneocytes. The stratum corneum intercellular lipids are derived from the layer below the stratum corneum, the stratum granulosum, as part of the keratinocyte differentiation process. Lamellar bodies are membrane bilayer secretory organelles in the epidermis that play a key role in delivering the polar precursor lipids and proteins, such as antimicrobial peptides, from stratum granulosum keratinocytes into the extracellular spaces of the stratum corneum¹. This lipid-based "mortar" is considered to be the main pathway for the diffusion of substances into the body. It consists of an approximately equimolar mixture of ceramides (Cer), cholesterol (Chol) and free fatty acids (FFA). In addition, minor amounts of cholesterol sulfate (ChoIS) are present. This unusual composition with high ceramide content is essential for maintaining the skin barrier function. Moreover, lower levels of ceramides and altered profiles of ceramides are found in prominent skin diseases such as atopic dermatitis and psoriasis. In the human stratum corneum, 11 major subclasses of ceramides based on four sphingoid bases and three acyl chain types have been identified. The acyl chain length has been reported to be very important. Specifically, its shortening is believed to probably decrease diffusion resistance of the skin due to the fact that the skin ceramides possess exclusively very long acyl chains. The most abundant species is Cer NS. Although short-chain ceramides have been used as ceramide mimics with increased aqueous solubility in various experiments, recent studies showed substantial differences between the native long-chain ceramides and their short-chain analogues in cell membranes and the skin. The activity of amphiphilic transdermal permeation enhancers, which influence the permeability of the stratum corneum ceramide membranes, is highly dependent on the chain length². Also, lipid chains can take conformations (trans or gauche), which could affect the compactness of the intercellular lipid layers, which may influence water transport in the stratum corneum. Moreover, the lateral ordering of stratum lipids is known to be important. There is a balance between orthorhombic and hexagonal packing. Both organizations are lamellar but an orthorhombic organization is necessary to let water to circulate through the lipid phase, while the percentage of the orthorhombic phase reduces the rate of water flux through the tissue³. It has been reported that long acyl chains in ceramides are essential for the formation of tightly packed impermeable lipid lamellae⁴.

Lines in the skin surface constitute what is known as skin microrelief or cutaneous microrelief. The different aspects of skin microrelief are generally classified according to the orientation and depth of the lines. Primary and secondary lines are discrete and shallow, reaching up to about 200 µm in depth. They form a criss-cross pattern and are readily identifiable on the wrists, for example. The microrelief characteristics of the primary lines are related to factors such as the skin softness and its velvety aspects. The orientation and depth of skin microrelief lines is influenced by aging and a variety of environmental factors. For example, the skin surface pattern becomes rough under dry conditions. The state of hydration of the stratum corneum is an important factor affecting skin microrelief: the skin surface morphology can be altered under different humidity conditions and can be regulated by the application of a humectant or by hydration^{5a}. By way of contrast, wrinkles are more readily visible structures, which are more complex, deeper and less numerous that the lines on the skin surface. Wrinkles are predominantly caused by muscle contractions under the skin, which is why they show up when we make facial expressions, like squinting, frowning and smiling.

The stratum corneum forms a very important part of the skin barrier and was thought for decades to be the only barrier present in the epidermis, even though a secondary epidermal barrier (tight junction barrier) was postulated a long time ago. Tight junctions (TJs) are intercellular junctions that are formed by multiprotein complexes presents in the stratum granulosum (SG). They consist of (i) integral membrane proteins including occludin (OCLN), junctional adhesion molecules (JAMs) and claudins (CLDNs); (ii) scaffolding proteins that anchor the membrane components to the actin cytoskeleton, e.g. the ZO isoforms; and (iii) cytoplasmic proteins including signaling molecules and transcription factors. Tight junctions are responsible principally for intercellular sealing. The tight junctions' seal prevents free diffusion of molecules between cells through the paracellular pathway by forming a physical barrier at the apical end of the junctional complex. The seal created by tight junctions is not absolute; tight junctions behave as though they are perforated by pores, allowing selective passage of ions and small uncharged molecules through the paracellular pathway. This is crucial for epithelial function. The tight junctions' structure is congruent with the secondary epidermal barrier function⁶.

Claudin-1 (CLDN1), a main component of tight junctions in the epidermis, is reported to be indispensable for this barrier function. Abnormalities in CLDN1 cause human skin diseases. Recent evidence indicates that atopic dermatitis (AD), which is a common chronic inflammatory skin disease, is associated with decreased CLDN1 expression levels in humans. Atopic dermatitis appears in -20% of children and 3% of adults, and its symptoms include itching and eczema, which decrease patients' quality of life. Although immunological imbalances were previously proposed to explain the features of atopic dermatitis, such as increased immunoglobulin E (IgE) levels and the acquired immune response, recent studies showed that epidermal barrier functions critically influence the pathological features of atopic dermatitis. A recent study of the in vivo response to decreased CLDN1 expression levels in mouse revealed that CLDN1 plays a critical role in atopic dermatitis, several features of which were related to the CLDN1 expression level. These results provide strong evidence that CLDN1-induced changes in barrier function have potential roles in the pathogenesis, severity, and natural course of atopic dermatitis and suggest that the immunological features of atopic dermatitis depend on the external environment. CLDN1 orchestrates the features of atopic dermatitis in a time and dose-dependent manner in vivo which provides insight into the etiology of atopic dermatitis and suggests a potential target for future therapies⁷.

The skin is colonized by a diverse collection of microorganisms - including bacteria, fungi and viruses - as well as mites. The set of microbial communities present in the skin is named skin microbiota. Many of these microorganisms are harmless and in some cases provide vital functions that the human genome has not evolved. Symbiotic microorganisms occupy a wide range of skin niches and protect against invasion by more pathogenic or harmful organisms. Human skin is comprised of different areas with variable temperature, pH, humidity, antimicrobial peptide (AMP) composition, and lipid content. Consequently, these diverse areas provide unique and variable areas for the growth of microorganisms⁸. The skin microbiota provides positive effects and contributes to host immune defense through different mechanisms. The skin has a complex relationship with the diverse microorganisms. Skin keratinocytes discriminate the presence of commensals from the presence of pathogenic microorganisms by differential induction of antimicrobial peptides and activation of distinct signaling pathways to induce an adapted and fast innate immune response. Physiological and psychological stressors can modulate communication between the host and microbiota thus affecting the immune response and promoting infection⁹.

The skin immune response is vital in wounding and infection and also modulates the commensal microbiota that colonizes the skin. A major part of innate protection is the recognition of pathogen-associated molecular patterns (PAMPs) by pattern recognition receptors (PRRs) that in humans comprise toll like receptors (TLRs), nucleotide-binding oligomerization domain (NOD)-like receptors (NLRs), retinoic acidinducible gene I (RIGI)-like receptors (RLRs) and C-type lectin receptors (CLRs). The ligation of PRRs by PAMPs results in an inflammatory response leading to efficient destruction of the invading pathogens. To accomplish these functions in skin, resident immune system cells (e.g. epidermal Langerhans cells (LCs), dermal dendritic cells (DDCs) and mast cells) as well as nonimmune system cells (e.g. keratinocytes, melanocytes, endothelial cells of the microvasculature, stromal cells such as fibroblasts and adipocytes) are endowed with an array of PRRs. Pathogenic micro-organisms in the skin can also be killed through antimicrobial peptides such as defensins and cathelicidins, which are either constitutively produced by keratinocytes or induced after an inflammatory response or a direct activation of pattern recognition receptors by microbial components. Toll like receptors recognize pathogens at the cell surface (TLRs 1, 2, 4-6, 10) or within the endosome (TLRs 3, 7-9), whereas (NOD)-like receptors and (RIGI)-like receptors act as intracellular surveillance molecules¹⁰. Toll like receptors are members of a larger superfamily of interleukin-1 receptors (IL-1Rs) that share significant homology in their cytoplasmic regions. The IL-1R and TLR family signals via shared downstream signaling molecules. They include, among others, the adaptor molecule MyD88¹¹.

Viral infections are common in human skin, with keratinocytes as viral targets. Therefore, the capacity to induce a sufficient antiviral response seems to be crucial during the earliest phases of the innate immune response. Keratinocytes *in vitro* respond very well to a synthetic mimicking viral double-stranded RNA analog, which occurs as a metabolite during viral infection. The corresponding receptors are TLR3 and MDA5, which are expressed in multiple cell types including keratinocytes. Activation of TLR3 either directly leads to NF-κB activation and upregulation of NF-κB-responsive proteins such as CXCL8/IL8, or trigger TRIF activation that ultimately stimulates, independently of MyD88, the IRF3 transcription factor through TANK-binding kinase-1 (TBK1), followed by the production of type I interferons (IFNs) and the expression of proinflammatory cytokines. Thus, keratinocytes are equipped with a broad antiviral defense program enabling them to efficiently target viral infections of the skin. Prenatal skin already expresses the same spectrum of toll like receptors as adult skin. Strikingly, many toll like receptors are significantly higher expressed in prenatal (TLRs 1-5) and infant and child (TLRs 1 and 3) skin than in adult skin¹².

Physical and immunological barriers are maintained in the epidermis, and the interaction between these barriers is known. Toll like receptors activation functionally alters tight junctions in cultured keratinocytes, which suggests that the immediate enhancement of the tight junctions function prevents further invasion of pathogens and/or their secreted products and maintains cutaneous barrier homeostasis. For instance, it is known that the infiltration of pathogens into the epidermis enhances tight junctions function via TLR2 signaling (trough TLR2 or TLR adaptor MyD88)¹³.

In addition to intrapersonal anatomical variations in the skin microbiota, the diversity and abundance of the cutaneous microbial flora varies between gender, age, seasons, ethnicity as well as various stressors, including physiological injury and psychological anxiety, promoting endocrine and metabolic changes within the cutaneous microenvironments that directly impact the metabolic requirements and pathogenicity of various microorganisms. The impact of environmental factors such as climate, including temperature and UV exposure but also of lifestyle, including alcohol intake or nutrition on microbial communities remains to be elucidated.

Frequent washing has also been reported to disturb the skin barrier resulting in skin irritation and in changes in the microbiome on hand skin. Cosmetics, hygiene products, makeup and moisturizers have also been implicated in modifying the skin microbiota.

The overuse of antibiotics, which were initially and still are an important milestone in the treatment of all kind of bacterial infections, has become a general health issue leading to a certain number of antibiotic-resistant strains of pathogenic microorganisms making the treatment of infections almost impossible and hence permanently unbalance the gut and skin microbiota. Radiotherapy and chemotherapy used to treat cancer may also impact the microbiota.

However, there are not only extrinsic factors that imbalance the healthy skin microbiota. Intrinsic factors such as a sebum overproduction, e.g. during puberty, enhance the over-colonization by *Propionibacterium acnes* (*P. acnes*) potentially leading to acne and to an imbalanced skin microbiota¹⁴. The overgrowth of P. acnes, a commensal skin bacterium, has been associated with the progression of acne vulgaris. Skin microorganisms can mediate fermentation of glycerol, which is naturally produced in skin, to enhance their inhibitory effects on P. acnes growth. The skin microorganisms, most of which have been identified as *Staphylococcus epidermidis* (*S. epidermidis*)*,* in the microbiome of human fingerprints can ferment glycerol and create inhibition zones to repel a colony of overgrown *P. acnes*¹⁵.

Increasing evidence demonstrates that commensal microorganisms in the human skin microbiome help fight pathogens and maintain homeostasis of the microbiome. These microorganisms maintain biological balance when one of them overgrows. The growth of some commensal bacteria, protect the host from pathogenic bacteria both directly and indirectly. Direct effects include bacteriocin production, production of toxic metabolites, induction of a low reduction oxidation potential, depletion of essential nutrients, prevention of adherence of competing bacteria, inhibition of translocation, and degradation of toxins. Commensal bacteria compete for nutrients, niches, and receptors. For example, Staphylococcus epidermidis bind keratinocyte receptors and inhibit adherence of virulent S. aureus. Commensals can release speciesspecific antibiotic substances known as bacteriocins. Indirectly, bacteria can induce the host to enhance antibody production, stimulate phagocytosis and clearance mechanisms, and augment interferon and cytokine production¹⁶.

The skin microbial flora can enter a state of dysbiosis, defined as a change in relative composition of the different microbes compared to normal, during a disease state. This is most well described in atopic dermatitis. It was first observed in the 1970s that there was increased S. aureus colonization on atopic dermatitis lesions. Besides increased S. aureus colonization of the skin in atopic dermatitis, 16S rRNA DNA sequencing has revealed increased S. epidermidis colonization. This seems to have a protective role against infections. S. epidermidis has also been shown in both murine and keratinocyte models to have a protective role through the amplification of endogenous AMPs. Finally, there is evidence that S. epidermidis can prevent S. aureus biofilm formation in the nasal cavities as well as produce its own AMPs to prevent other pathogens from colonizing the skin. Therefore, increased S. epidermis colonization in skin affected by atopic dermatitis may represent a way for the skin to naturally prevent increased S. aureus colonization in atopic dermatitis¹⁷.

There is a need for new methods of treating the skin so as to maintain and/or improve the physical and/or the immunological barrier functions of the skin. There is a need for new methods of treating the skin so as to prevent and/or alleviate one or more adverse effects to the skin associated with an impaired barrier function.
EP2123673A1 discloses the use of a peptide of a general formula (I) R₁-AA-R₂ as defined therein in the preparation of a cosmetic or pharmaceutical composition for the treatment of those conditions in mammals requiring neuronal exocytosis regulation.
US 2011/0206752A1 discloses the use of a peptide of a general formula (I) R₁-AA-R₂ as defined therein for the treatment of pain and/or inflammation.
US 2016/206543A1 discloses compositions containing a combination of hexapeptides and Blanes-Mira C et al ("A synthetic hexapeptide (Argireline) with antiwrinkle activity", International Journal of Cosmetic Science, Kluwer Academic Publishers, Dordrecht, NL, vol 24, no. 5, 1 January 2002, pages 303 to 301) relates to the hexapeptide, Argireline^{®}.

### SUMMARY OF THE INVENTION

The invention is defined in the appended claims. Employed in the invention is a compound represented by formula (I)

R₁-Wₘ-Xₙ-AA₁-AA₂-AA₃- AA₄-AA₅-AA₆-(AA₇)ₐ-(AA₈)_{b}-Yₚ-Z_{q}-R₂ (I),

its stereoisomers and/or its cosmetically or pharmaceutically acceptable salts, wherein:
AA₁ is Glu; AA₂ is Glu; AA₃ is Met; AA₄ is Gln; AA₅ is Arg; AA₆ is Arg; AA₇ is selected from the group consisting of Ala, Ser, Gly, Thr, Pro, Glu, Lys and Val; AA₈ is selected from the group consisting of Asp, Glu, Asn and Gln; and
up to 2 of AA₁, AA₂, AA₃, AA₄, AA₅ and AA₆ are replaced providing that:
   when AA₁ is replaced, it is replaced by an amino acid selected from the group formed by Asp, Gln and Asn;
   when AA₂ is replaced, it is replaced by an amino acid selected from the group formed by Asp and Gln;
   when AA₃ is replaced, it is replaced by an amino acid selected from the group formed by Leu and Ile;
   when AA₄ is replaced, it is replaced by an amino acid selected from the group formed by Glu, Asn and Asp;
   when AA₅ is replaced, it is replaced by Lys; and
   when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids.

It has been found that the compounds of the invention are effective in maintaining and improving the physical and immunological barriers provided by the skin. As a result, the compounds of the invention are useful in the protection, treatment and/or care of the skin, hair, nails and/or mucous membranes. In particular, the compounds of the invention are useful in non-therapeutic and therapeutic treatments of conditions of the skin associated with an impaired physical barrier function of the skin and/or an impaired immunological barrier function of the skin.

Disclosed is a cosmetic or pharmaceutical composition comprising a compound of formula (I), its stereoisomers and/or its cosmetically or pharmaceutically acceptable salts, together with at least one cosmetically or pharmaceutically acceptable excipient or adjuvant.

Disclosed is the use of a compound of formula (I), its stereoisomers and/or its cosmetically or pharmaceutically acceptable salts, or a composition comprising a compound of formula (I), its stereoisomers and/or its cosmetically or pharmaceutically acceptable salts, for the treatment and/or care of the skin, hair, nails and/or mucous membranes.

In one aspect, the invention provides the cosmetic, non-therapeutic use of a compound of formula (I), its stereoisomers and/or its cosmetically acceptable salts, or a composition comprising same, for the cosmetic, non-therapeutic treatment and/or care of the skin, hair, nails and/or mucus membranes, as defined in the appended claims. In particular, it has been found that the compounds of the invention are effective at maintaining and/or improving skin hydration.

In one aspect, the invention provides a compound of formula (I), its stereoisomers and/or its pharmaceutically acceptable salts, or a pharmaceutical composition comprising same, for use as a medicament, as defined in the appended claims. In particular, the invention provides a compound of formula (I), its stereoisomers and/or its pharmaceutically acceptable salts, or a pharmaceutical composition comprising same, for use in the treatment or prevention of a disease or disorder which is associated with impairment of the barrier functions of the skin. In particular, the invention relates to the compound of formula (I), its stereoisomers and/or its pharmaceutically acceptable salts, or a pharmaceutical composition comprising same, for use in the treatment of atopic dermatitis.

Disclosed is the use of the compound of formula (I), its stereoisomers and/or its pharmaceutically acceptable salts for the manufacture of a medicament for the treatment or prevention of a disease or disorder. The disease or disorder is associated with impairment of the barrier functions of the skin and includes atopic dermatitis.

Disclosed is a method of treatment and/or care of the skin, hair, nails and/or mucous membranes of a subject comprising administering a compound of formula (I), its stereoisomers and/or its cosmetically or pharmaceutically acceptable salts, or a composition comprising same, to the subject. Typically the compound of the invention will be administered topically.

In one aspect, the invention provides a cosmetic, non-therapeutic method of treatment and/or care of the skin, hair, nails and/or mucous membranes of a subject comprising administering a compound of formula (I), its stereoisomers and/or its cosmetically acceptable salts, or a cosmetic composition comprising same, to the subject, as defined in the appended claims.

Disclosed is a method of treatment of disease or disorder comprising administering a compound of formula (I), its stereoisomers and/or its pharmaceutically acceptable salts, or a pharmaceutical composition comprising same, to the subject. The disease or disorder includes diseases or disorders of the skin, hair, nails and/or mucous membranes associated with impairment of the barrier functions of the skin.

### DETAILED DESCRIPTION OF THE INVENTION

### Definitions

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes, mast cells, neurones and/or adipocytes among others. The term "skin" also comprises the scalp. The term "skin" includes the skin of mammals and includes human skin. Likewise the terms "hair, nails and mucous membranes" include the hair, nails and mucous membranes of mammals, for example humans.

The term "treatment", covers therapeutic methods including methods directed to the administration of a compound according to the invention to alleviate or eliminate a disease or disorder, or to reduce or eliminate one or more symptoms associated with said disease or disorder. The term "treatment" also covers methods of therapy directed to alleviating or eliminating physiological consequences of the disease or disorder.

When the terms "treatment" and "care" are accompanied by the qualifications "cosmetic" and/or "non-therapeutic", it means that the treatment or care is such and, for example, has the aim of improving or maintaining the aesthetic appearance of the skin, hair, nails and/or mucous membranes. In particular, the treatment can have the aim of improving cosmetic properties of the skin, hair, nails and/or mucous membranes such as, for example and not restricted to, the level of hydration, elasticity, firmness, shine, tone or texture, which properties affect the aesthetic appearance of the skin, hair, nails and/or mucous membranes. The term "care" in the context of this specification refers to the maintenance of properties of the skin, hair, nails and/or mucous membranes. Said properties are subject to being improved or maintained by cosmetic treatment and/or care of the skin, hair, nails and/or mucous membranes both in healthy subjects (for example, in the cosmetic, non-therapeutic treatment and/or care of skin which is not affected by inflammation, such as when the subject does not suffer inflammation of the skin; in the cosmetic, non-therapeutic treatment and/or care of skin which is not affected by atopic dermatitis, such as when the subject does not suffer atopic dermatitis, in the cosmetic, non-therapeutic treatment and/or care of skin which is not affected by contact dermatitis, such as when the subject does not suffer contact dermatitis, in the cosmetic, non-therapeutic treatment and/or care of skin which is not affected by exczema, such as when the subject does not suffer exczema, and/or in the cosmetic, non-therapeutic treatment and/or care of skin which is not affected by psoriasis, such as when the subject does not suffer psoriasis) as well as in those have sensitive skin and those which present diseases and/or disorders of the skin, hair, nails and/or mucous membranes such as, for example and not restricted to, ulcers and injuries to skin, psoriasis, dermatitis, acne or rosacea, among others.

The term "prevention", as used in this invention, refers to the ability of a compound of the invention to prevent, delay or hinder the appearance or development of a disease or disorder, or to prevent, delay or hinder the change in a cosmetic property of the skin, mucous membranes and/or hair. The term "prevention", as used in this invention, is interchangeable with the term "inhibition", i.e. it refers to the ability of a compound of the invention to inhibit the appearance or development of a disease or disorder, or to inhibit the change in a cosmetic property of the skin, hair, nails and/or mucous membranes.

In the context of this invention, the term "aging" refers to the changes experienced by the skin with age (chronoaging) or through exposure to the sun (photoaging) or to environmental agents such as tobacco smoke, extreme climatic conditions of cold or wind, chemical contaminants or pollutants, and includes all the external visible and/or perceptible changes through touch, such as and not restricted to, the development of discontinuities on the skin such as wrinkles, fine lines, expression lines, stretch marks, furrows, irregularities or roughness, increase in the size of pores, loss of hydration, loss of elasticity, loss of firmness, loss of smoothness, loss of the capacity to recover from deformation, loss of resilience, sagging of the skin such as sagging cheeks, the appearance of bags under the eyes or the appearance of a double chin, among others, changes to the color of the skin such as marks, reddening, bags or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and it presents the same physical characteristics as aging, such as and not restricted to, flaccidity, sagging, changes to the color or irregularities in the pigmentation, abnormal and/or excessive keratinization. The sum of various environmental factors such as exposure to tobacco smoke, exposure to pollution, and climatic conditions such as cold and/or wind also contribute to the aging of the skin.

In this description the abbreviations used for amino acids follow the rules of IUPAC-IUB Commission of Biochemical Nomenclature specified in Eur. J. Biochem., (1984), 138, 9-37*.* Thus, for example, Gly represents NH₂-CH₂-COOH, Gly- represents NH₂-CH₂-CO-, -Gly represents -NH-CH₂-COOH and -Gly- represents -NH-CH₂-CO-. Therefore, the hyphen, which represents the peptide bond, eliminates the OH in the 1-carboxyl group of the amino acid (represented here in the conventional non-ionized form) when situated to the right of the symbol, and eliminates the H of the 2-amino group of the amino acid when situated to the left of the symbol; both modifications can be applied to the same symbol (see Table 1).

| **Table 1. Structures of the amino acid residues, their nomenclature in three-letter code and nomenclature for the amino acids in one letter code** | | | |
|---|---|---|---|
| Name | Residue | Symbol | Residue |
| Glutamyl -GluE | 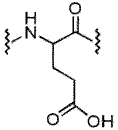 | Aspartyl -Asp-D | 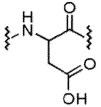 |
| Glutaminyl -Gln-Q | 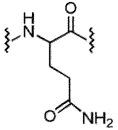 | Asparaginyl -Asn-N | 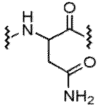 |
| Tyrosyl -Tyr-Y | 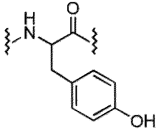 | Phenylalanyl -Phe-F | 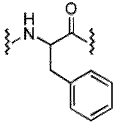 |
| Methionyl -Met-M | 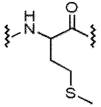 | Leucyl -Leu-L | 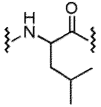 |
| Isoleucyl -Ile-I | 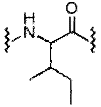 | Arginyl -Arg-R | 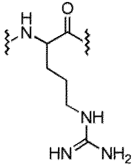 |
| Lysyl -Lys-K | 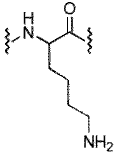 | Histidyl -His-H | 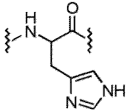 |
| Alanyl -Ala-A | 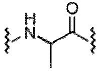 | Seryl -Ser-S | 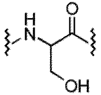 |
| Glycyl -Gly-G | 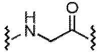 | Threonyl -Thr-T | 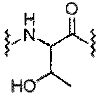 |
| Prolyl -ProP | 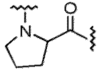 | Valyl -Val-V | 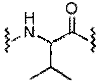 |

As used herein, the term "non-cyclic aliphatic group"" includes linear (i.e. straight and unbranched) or branched, saturated or unsaturated hydrocarbyl groups such as alkyl, alkenyl and alkynyl. The non-cyclic aliphatic group may be substituted (mono- or poly-) or unsubstituted.

As used herein, the term "alkyl" includes both saturated linear and branched alkyl groups, which may be substituted (mono- or poly-) or unsubstituted. The alkyl group is bound to the rest of the molecule by a single bond. The alkyl group has from 1 to 24, preferably from 1 to 16, more preferably from 1 to 14, even more preferably from 1 to 12, yet more preferably 1, 2, 3, 4, 5 or 6 carbon atoms. The term "alkyl" includes, for example, methyl, ethyl, isopropyl, isobutyl, tert-butyl, 2-methylbutyl, heptyl, 5-methylhexyl, 2-ethylhexyl, octyl, decyl, dodecyl, lauryl, hexadecyl, octadecyl and amyl.

As used herein, the term "alkenyl" refers to a group containing one or more double carbon-carbon bonds and which may be linear or branched and substituted (mono- or poly-) or unsubstituted. Preferably it has 1, 2 or 3 double carbon-carbon bonds. If more than one double carbon-carbon bond is present, the double bonds may be conjugated or not conjugated. Preferably the alkenyl group has from 2 to 24, preferably from 2 to 16, more preferably from 2 to 14, even more preferably from 2 to 12, yet more preferably 2, 3, 4, 5 or 6 carbon atoms. The alkenyl group is bound to the rest of the molecule by a single bond. The term "alkenyl" includes, for example, vinyl (-CH₂=CH₂), allyl (-CH₂-CH=CH₂), prenyl, oleyl, linoleyl groups and similar.

The term "alkynyl" refers to a group containing one or more triple carbon-carbon bonds and which may be linear or branched, and substituted (mono- or poly-) or unsubstituted. Preferably the alkynyl group has 1, 2 or 3 triple carbon-carbon bonds. The triple bonds may be conjugated or not conjugated The alkynyl group has from 2 to 24, preferably from 2 to 16, more preferably from 2 to 14, even more preferably from 2 to 12, yet more preferably 2, 3, 4, 5 or 6 carbon atoms. The alkynyl group is bound to the rest of the molecule by a single bond. The term "alkynyl" includes, for example and not restricted to, ethynyl, 1-propynyl, 2-propynyl, 1-butynyl, 2-butynyl, 3-butynyl, pentynyl, such as 1-pentynyl, and similar. The alkynyl group can also contain one or more double carbon-carbon bonds, and alkynyl groups include, for example and not restricted to, but-1-en-3-ynyl and pent-4-en-1-ynyl groups, and similar.

The term "alicyclyl" is used herein to cover, for example and not restricted to, aliphatic cyclic (alicyclic) groups such as cycloalkyl or cycloalkenyl or cycloalkynyl groups. The term "alicyclyl" refers to a monoradical that contains one or more rings of carbon atoms, the rings may be saturated (e.g., cyclohexyl) or unsaturated (e.g., cyclohexenyl) provided that they are not aromatic. More specifically alicylic groups contain three or more, from 3 to 24, from 3 to 12, or from 6 to 12, ring carbon atoms. The alicyclic group may be a monocyclic, bicyclic, or tricyclic ring system and the rings may be, for example, fused or linked by a single bond or a linking group such as a methylene or other alkylene group. The alicyclic group may be substituted (mono- or poly-) or unsubstituted. In one embodiment, the alicyclyl group is a 6 to 12 membered ring system which consists of carbon atoms and optionally contains one or two double bonds.

The term "cycloalkyl" refers to a saturated mono- or polycyclic alkyl group which may be substituted (mono- or poly-) or unsubstituted. The cycloalkyl group has from 3 to 24, preferably from 3 to 16, more preferably from 3 to 14, even more preferably from 3 to 12, yet even more preferably 3, 4, 5 or 6 carbon atoms. The cycloalkyl group is bound to the rest of the molecule by a single bond, Cycloalkyl groups include, for example and not restricted to, cyclopropyl, cyclobutyl, cyclopentyl, cyclohexyl, cycloheptyl, methyl cyclohexyl, dimethyl cyclohexyl, octahydroindene, decahydronaphthalene, dodecahydrophenalene and similar.

The term "cycloalkenyl" refers to a non-aromatic mono- or polycyclic alkenyl group which may be substituted (mono- or poly-) or unsubstituted. The cycloalkenyl group has from 5 to 24, preferably from 5 to 16, more preferably from 5 to 14, even more preferably from 5 to 12, yet more preferably 5 or 6 carbon atoms. The cycloalkenyl group is bound to the rest of the molecule by a single bond. Preferably the cycloalkenyl group contains 1, 2 or 3 double carbon-carbon bonds. If more than one double carbon-carbon bond is present, the double bonds may be conjugated or not conjugated. Cycloalkenyl groups include, for example and not restricted to, the cyclopent-1-en-1-yl group and similar.

The term "cycloalkynyl" refers to a non-aromatic mono- or polycyclic alkynyl group which may be substituted (mono- or poly-) or unsubstituted. The cycloalkynyl group has from 8 to 24, preferably from 8 to 16, more preferably from 8 to 14, even more preferably from 8 to 12, yet even more preferably 8 or 9 carbon atoms and is bound to the rest of the molecule by a single bond. Preferably the cycloalkynyl group contains 1, 2 or 3 triple carbon-carbon bonds, conjugated or not conjugated. Cycloalkynyl groups include, for example and not restricted to, the cyclooct-2-yn-1-yl group and similar. Cycloalkynyl groups can also contain one or more double carbon-carbon bonds, including, for example and not restricted to, the cyclooct-4-en-2-ynyl group and similar.

As used herein, the term "heterocyclyl" or "heterocyclic" refers to a hydrocarbon ring system of 3 to 10 members, wherein one or more of the atoms in the ring or rings is a heteroatom (i.e. not a carbon atom). Thus "heterocyclyl" or "heterocyclic" refers a cyclic group in which the ring atoms consist of carbon and one or more heteroatoms. To satisfy valence, the heteroatom may be bonded to H or substituent groups. Preferably from 1, 2 or 3 of the ring carbon atoms are heteroatoms. Each heteroatom can be independently selected from the group consisting of O, N, S, P and B, or the group consisting of O, N, and S. The heterocyclyl group may be substituted (mono- or poly-) or unsubstituted. The heterocyclyl group may be a monocyclic, bicyclic, or tricyclic ring system and the rings may be, for example, fused or linked by a single bond or a linking group such as a methylene or other alkylene group. Nitrogen, carbon or sulfur atoms present in the heterocyclyl radical may be optionally oxidized and the nitrogen atom may be optionally quaternized. The heterocyclyl radical may be unsaturated or partially or fully saturated. The heterocyclyl radical may be aliphatic or aromatic. In one embodiment, the heterocyclyl is aliphatic (also known as heteroalicyclyl) and is a 3 to 10 membered ring system where the atoms of the ring or rings consist of carbon atoms and from 1 to 4, or 1, 2 or 3 heteroatoms. In one embodiment, the heterocyclyl group is a 6 to 10 membered ring system where the atoms of the ring or rings consist of carbon atoms and from 1 to 4 heteroatoms and where the ring system optionally contains one or two double bonds. In one embodiment, the heterocyclyl is aromatic (also known as heteroaryl) and is a 6 to 10 membered ring system where the atoms of the ring or rings consist of carbon atoms and from 1 to 4, or 1, 2 or 3 heteroatoms. The greatest preference is for the term heterocyclyl to refer to a ring of 5 or 6 members. Examples of saturated heteroalicyclyl groups are dioxane, piperidine, piperazine, pyrrolidine, morpholine and thiomorpholine. Examples of aromatic heterocyclyl groups are pyridine, pyrrol, furan, thiophene, benzofuran, imidazoline, quinolein, quinoline, pyridazine and naphthyridine.

The term "aryl group" refers to an aromatic group which has from 6 to 30, preferably from 6 to 18, more preferably between 6 and 10, yet even more preferably 6 or 10 carbon atoms. The aryl group can comprise 1, 2, 3 or 4 aromatic rings, which may be linked by a carbon-carbon bond or fused together and includes, for example and not restricted to, phenyl, naphthyl, diphenyl, indenyl, phenanthryl or antranyl among others. The aryl group may be substituted (mono- or poly-) or unsubstituted.

The term "aralkyl group" refers to an alkyl group substituted by an aromatic group, with from 7 to 24 carbon atoms and including, for example and not restricted to, -(CH₂)₁₋₆-phenyl, -(CH₂)₁₋₆-(1-naphthyl), -(CH₂)₁₋₆-(2-naphthyl), -(CH₂)₁₋₆-CH(phenyl)₂ and similar.

The term "heteroarylalkyl" refers to an alkyl group substituted by a heteroaryl (also known as aromatic heterocyclic) group as defined above, the alkyl group having from 1 to 6 carbon atoms and the heteroaryl group having from 2 to 24 carbon atoms and from 1 to 3 heteroatoms. Heteroarylalkyl groups include, for example and not restricted to -(CH₂)₁₋₆-imidazolyl, -(CH₂)₁₋₆-triazolyl, -(CH₂)₁₋₆-thienyl, -(CH₂)₁₋₆-furyl, -(CH₂)₁₋₆-pyrroli dinyl and similar.

As is understood in this technical field, there may be a certain degree of substitution of the aforementioned groups. In particular, there can be substitution in any of the groups identified above where it is explicitly stated. The substituted groups (radicals) referred to above are groups (or radicals) which are substituted in one or more positions available by one or more substituents. Preferably substitution is in the 1, 2 or 3 positions, more preferably in the 1 or 2 positions, yet even more preferably in the 1 position. Suitable substituents include, for example and not restricted to: C₁-C₄ alkyl; hydroxyl; C₁-C₄ alkoxyl; amino; amino-C₁-C₄alkyl; C₁-C₄ carbonyloxyl; C₁-C₄ oxycarbonyl; halogen such as fluoride, chlorine, bromine and iodine; cyano; nitro; azide; C₁-C₄ alkylsulfonyl; thiol; C₁-C₄ alkylthio; aryloxy such as phenoxyl; -NR_{b}(C=NR_{b})NR_{b}R_{c}; wherein R_{b} and R_{c} are independently selected from the group formed by H, C₁-C₄ alkyl, C₂-C₄ alkenyl, alkynyl, C₃-C₁₀ cycloalkyl, C₆-C₁₈ aryl, C₇-C₁₇ aralkyl, heterocyclyl of 3-10 members or protective group of the amino group.

As used herein, the term "comprising", which is inclusive or open-ended and does not exclude additional unrecited elements or method steps, is intended to encompass as alternative embodiments, the phrases "consisting essentially of" and "consisting of" where "consisting of" excludes any element or step not specified and "consisting essentially of" permits the inclusion of additional unrecited elements or steps that do not materially affect the essential or basic and novel characteristics of the composition or method under consideration.

### Compounds of the Invention

Employed in the invention is a compound represented by formula (I)

R₁-Wₘ-Xₙ-AA₁-AA₂-AA₃-AA₄-AA₅-AA₆-(AA₇)ₐ-(AA₈)_{b}-Yₚ-Z_{q}-R₂ (I),

its stereoisomers and/or its cosmetically or pharmaceutically acceptable salts, wherein:
AA₁ is Glu; AA₂ is Glu; AA₃ is Met; AA₄ is Gln; AA₅ is Arg; AA₆ is Arg; AA₇ is selected from the group consisting of Ala, Ser, Gly, Thr, Pro, Glu, Lys and Val; AA₈ is selected from the group consisting of Asp, Glu, Asn and Gln;
up to 2 (i.e. 0, 1 or 2) of AA₁, AA₂, AA₃, AA₄, AA₅ and AA₆ are replaced providing that:
   when AA₁ is replaced, it is replaced by an amino acid selected from the group formed by Asp, Gln and Asn;
   when AA₂ is replaced, it is replaced by an amino acid selected from the group formed by Asp and Gln;
   when AA₃ is replaced, it is replaced by an amino acid selected from the group formed by Leu and Ile;
   when AA₄ is replaced, it is replaced by an amino acid selected from the group formed by Glu, Asn and Asp;
   when AA₅ is replaced, it is replaced by Lys; and
   when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids.

The compound of formula (I) is a peptide which comprises 7, 8, 9 or 10 amino acids linked in a chain. R₁ is bound to the amino terminal end (N-terminal) of the peptide and R₂ is bound to the carboxy-terminal end (C-terminal) of the peptide.

R₁ can be selected from the group consisting of H, a polymer derived from polyethylene glycol with a molecular weight comprised between 200 and 35000 Daltons and R₅-CO-, wherein R₅ is selected from the group consisting of C₁-C₂₄ alkyl, C₂-C₂₄ alkenyl, C₂-C₂₄ alkynyl, C₃-C₂₄ cycloalkyl, C₅-C₂₄ cycloalkenyl, C₈-C₂₄ cycloalkynyl, C₆-C₃₀ aryl, C₇-C₂₄ aralkyl, 3-10 membered heterocyclyl ring, and a heteroarylalkyl containing from 2 to 24 carbon atoms and from 1 to 3 heteroatoms, wherein the alkyl group has 1 to 6 carbon atoms. In one embodiment, R₁ is selected from the group consisting of H and R₅-CO-, wherein R₅ is selected from the group consisting of C₁-C₁₈ alkyl, C₂-C₂₄ alkenyl, C₃-C₂₄ cycloalkyl or the group consisting of C₁-C₁₆ alkyl, C₂-C₁₈ alkenyl, C₃-C₇ cycloalkyl. The R₅-CO- group includes alkanoyl groups such as acetyl (CH₃-CO-, which is abbreviated herein as "Ac-"), myristoyl (CH₃-(CH₂)₁₂-CO-, which is abbreviated herein as "Myr-") and palmitoyl (CH₃-(CH₂)₁₄-CO-, which is abbreviated herein as "Palm-"). In one embodiment, R₁ is selected from the group consisting of H and acetyl, tert-butanoyl, prenyl, hexanoyl, 2-methylhexanoyl, cyclohexanecarboxyl, octanoyl, decanoyl, lauroyl, myristoyl, palmitoyl, stearoyl, oleoyl and linoleoyl. In one embodiment, R₁ is selected from the group consisting of H and R₅-CO-, wherein R₅ is selected from the group consisting of C₁-C₁₆ alkyl or C₂-C₁₈ alkenyl. In one embodiment, R₁ is selected from the group consisting of H, acetyl, myristoyl or palmitoyl.

R₂ can be selected from the group consisting of -NR₃R₄, -OR₃, -SR₃, wherein R₃ and R₄ are independently selected from the group formed by H, a polymer derived from polyethylene glycol, C₁-C₂₄ alkyl, C₂-C₂₄ alkenyl, C₂-C₂₄ alkynyl, C₃-C₂₄ cycloalkyl, C₅-C₂₄ cycloalkenyl, C₈-C₂₄ cycloalkynyl, C₆-C₃₀ aryl, C₇-C₂₄ aralkyl, 3-10 membered heterocyclyl ring, and heteroarylalkyl containing from 2 to 24 carbon atoms and from 1 to 3 heteroatoms, wherein the alkyl group has 1 to 6 carbon atoms. Optionally, R₃ and R₄ can be joined by a saturated or unsaturated carbon-carbon bond, forming a ring with the nitrogen atom. In one embodiment, R₂ is -NR₃R₄ or-OR₃. In one embodiment, R₃ and R₄ are independently selected from the group consisting of H, a polymer derived from polyethylene glycol with a molecular weight comprised between 200 and 35000 Daltons, methyl, ethyl, hexyl, dodecyl and hexadecyl. In one embodiment R₃ and R₄ are independently selected from the group consisting of H and C₁-C₁₆ alkyl. In one embodiment R₃ is H and R₄ is selected from the group formed by H and C₁-C₁₆ alkyl, including methyl, ethyl, hexyl, dodecyl and hexadecyl. In accordance with one embodiment, R₂ is selected from -NR₃R₄ and -OR₃ wherein R₃ and R₄ are independently selected from the group consisting of H and C₁-C₁₆ alkyl. In one embodiment, R₂ is selected from the group consisting of -OH, -NH₂ and -NHR₄ where R₄ is C₁-C₁₆ alkyl. R₄ can be C₆ alkyl, i.e. -C₆H₁₃, or C₁₆ alkyl, i.e. -C₁₆H₃₃.

R₁ can be selected from the group consisting of H and R₅-CO-, wherein R₅ is selected from the group consisting of C₁-C₁₈ alkyl, C₂-C₂₄ alkenyl, C₃-C₂₄ cycloalkyl; and R₂ is -NR₃R₄ or -OR₃ wherein R₃ and R₄ are independently selected from the group consisting of H and C₁-C₁₆ alkyl. In an exemplary embodiment, R₃ is H and R₄ is selected from the group formed by H and C₁-C₁₆ alkyl; for example, R₂ is selected from the group consisting of -OH, -NH₂ and -NHR₄ where R₄ is C₁-C₁₆ alkyl.

R1 can be selected from the group consisting of H and acetyl, tert-butanoyl, prenyl, hexanoyl, 2-methylhexanoyl, cyclohexanecarboxyl, octanoyl, decanoyl, lauroyl, myristoyl, palmitoyl, stearoyl, oleoyl and linoleoyl; and R₂ is -NR₃R₄ or -OR₃ wherein R₃ and R₄ are independently selected from the group consisting of H and C₁-C₁₆ alkyl. In an exemplary embodiment, R₃ is H and R₄ is selected from the group formed by H and C₁-C₁₆ alkyl; for example, R₂ is selected from the group consisting of - OH, -NH₂ and -NHR₄ where R₄ is C₁-C₁₆ alkyl.

R1 can be selected from the group consisting of H and R₅-CO-, wherein R₅ is selected from the group consisting of C₁-C₁₆ alkyl or C₂-C₁₈ alkenyl; and R₂ is -NR₃R₄ or -OR₃ wherein R₃ and R₄ are independently selected from the group consisting of H and C₁-C₁₆ alkyl. In an exemplary embodiment R₃ is H and R₄ is selected from the group formed by H and C₁-C₁₆ alkyl; for example, R₂ is selected from the group consisting of -OH, -NH₂ and -NHR₄ where R₄ is C₁-C₁₆ alkyl.

R1 can be selected from the group consisting of H, acetyl, myristoyl or palmitoyl; and R₂ is -NR₃R₄ or -OR₃ wherein R₃ and R₄ are independently selected from the group consisting of H and C₁-C₁₆ alkyl. In an exemplary embodiment R₃ is H and R₄ is selected from the group formed by H and C₁-C₁₆ alkyl; for example, R₂ is selected from the group consisting of -OH, -NH₂ and -NHR₄ where R₄ is C₁-C₁₆ alkyl.

The most preferred structures of the polymer derived from polyethylene glycol are the group (-CH₂-CH₂-O)ᵣ-H in which r is a number comprised between 4 and 795 and the group 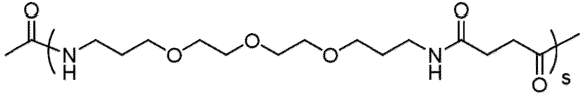 where s is a number comprised between 1 and 125.

In the compound of formula (I), 0 (none), 1 (one) or 2 (two) of AA, to AA₆ are replaced, i.e up to 2 of AA₁ to AA₆ are replaced or, optionally, 1 or 2 of AA₁ to AA₆ are replaced.

In one embodiment, the compound is of formula (I), wherein at least one of R₁ is not H; and R₂ is not OH.

In one embodiment, the compound of formula (I) is not one of the following compounds:

R₁-Glu-Glu-Met-Gln-Arg-Arg-Ala-Asp-R₂;

R₁-Glu-Leu-Glu-Glu-Met-Gln-Arg-Arg-Ala-Asp-R₂;

R₁-Glu-Leu-Glu-Glu-Met-Gln-Arg-Arg-Ala-R_{2;}

R₁-Glu-Leu-Glu-Glu-Met-Gln-Arg-Arg-R₂;

R₁-Leu-Glu-Glu-Met-Gln-Arg-Arg-Ala-Asp-Gln-R₂;

R₁-Leu-Glu-Glu-Met-Gln-Arg-Arg-Ala-Asp-R₂;

R₁-Leu-Glu-Glu-Met-Gln-Arg-Arg-Ala-R₂;

R₁-Leu-Glu-Glu-Met-Gln-Arg-Arg-R₂;

R₁- Glu-Glu-Met-Gln-Arg-Arg-Ala-Asp-Gln-Leu-R₂;

R₁- Glu-Glu-Met-Gln-Arg-Arg-Ala-Asp-Gln-R₂; and

R₁-Glu-Glu-Met-Gln-Arg-Arg-Ala-R₂.

In one embodiment , the compound is of formula (I),

R₁-Wₘ-Xₙ-AA₁-AA₂-AA₃-AA₄-AA₅-AA₆-(AA₇)ₐ-(AA₈)_{b}-Yₚ-Z_{q}-R₂ (I),

its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser and Gly; AA₈ is selected from the group consisting of Asp, Glu and Asn;
up to 2 (i.e. 0, 1 or 2) of AA₁, AA₂, AA₃, AA₄, AA₅ and AA₆ are replaced providing that:
   when AA₁ is replaced, it is replaced by an amino acid selected from the group formed by Asp and Gln;
   when AA₂ is replaced, it is replaced by an amino acid selected from the group formed by Asp and Gln;
   when AA₃ is replaced, it is replaced by an amino acid selected from the group formed by Leu and Ile;
   when AA₄ is replaced, it is replaced by an amino acid selected from the group formed by Glu, Asn and Asp;
   when AA₅ is replaced, it is replaced by Lys; and
   when AA₆ is replaced, it is replaced by Lys;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids.

In one embodiment, the compound is of formula (i), wherein 0 (none) of AA₁ to AA₆ is replaced. In one embodiment, a = 1 and b = 0 and when 0 of AA₁ to AA₆ is replaced, preferably, AA₇ is selected from the group consisting of Ala, Ser, Gly, Thr and Val. In one embodiment, a = 1 and b = 1 and when 0 of AA₁ to AA₆ is replaced, preferably, AA₇ is selected from the group consisting of Ala, Ser and Gly Thr, Pro, Glu and Lys; and AA₈ is selected from the group consisting of Asp, Glu and Asn.

In one embodiment, the compound is of formula (I),wherein 1 of AA₁ to AA₆ is replaced. In one embodiment, a = 1 and b = 0 and, preferably, when 1 of AA₁ to AA₆ is replaced AA₁ is replaced by Asn; or AA₄ is replaced by Asp. In one embodiment, a = 1 and b = 1 and, preferably, when 1 of AA₁ to AA₆ is replaced, AA₁ is replaced by Asp or Gln; or AA₃ is replaced by Leu; or AA₄ is replaced by Glu, Asp or Asn; or AA₆ is replaced by Lys or His.

In one embodiment, the compound is of formula (I), wherein 2 of AA₁ and AA₆ are replaced. In one embodiment, a = 1 and b = 0 and, preferably, when 2 of AA₁ to AA₆ are replaced, AA₁ is replaced by Asp and AA₄ is replaced by Glu. In one embodiment, a = 1 and b = 1.

In one embodiment, the compound is
of formula (I), wherein a = 1 and b = 0. In this embodiment, the compound of the invention represents a peptide which comprises 7, 8 or 9 amino acids linked in a chain and can be represented by the following formula:

R₁-Wₘ-Xₙ-AA₁-AA₂-AA₃-AA₄-AA₅-AA₆-AA₇-Yₚ-Z_{q}-R₂ (IX)

its stereoisomers and/or its cosmetically or pharmaceutically acceptable salts, wherein AA₁, AA₂, AA₃, AA₄, AA₅, AA₆, AA₇, a, b, W, X, Y, Z, m, n, p, q, R₁ and R₂ are as specified for formula (I). In this embodiment, AA₇ can be selected from the group consisting of Ala, Ser and Gly. In one embodiment, AA₇ is Ala.

In one embodiment, the compound is
of formula (I), wherein 0 of AA₁ to AA₆ is replaced and a = 1 and b = 0. In this embodiment, the compound of the invention can be represented by the following formula:

R₁-Wₘ-Xₙ-Glu-Glu-Met-Gln-Arg-Arg-AA₇-Yₚ-Z_{q}-R₂ (X)

its stereoisomers and/or its cosmetically or pharmaceutically acceptable salts, wherein: AA₇ is selected from the group consisting of Ala, Ser, Gly, Thr, Pro, Glu, Lys and Val; and wherein W, X, Y, Z, m, n, p, q, R₁ and R₂ are as specified for formula (I). In one embodiment, AA₇ is selected from the group consisting of Ala, Ser, Gly, Thr and Val.

In one embodiment, the compound is
of formula (I), wherein 1 of AA, to AA₆ is replaced and a = 1 and b = 0. In this embodiment, the compound of the invention can be represented by the following formula:

R₁-Wₘ-Xₙ- AA₁-AA₂-AA₃-AA₄-AA₅-AA₆-AA₇-Yₚ-Z_{q}-R₂ (XI)

its stereoisomers and/or its cosmetically or pharmaceutically acceptable salts, wherein:
AA₁ is Glu; AA₂ is Glu; AA₃ is Met; AA₄ is Gln; AA₅ is Arg; AA₆ is Arg; AA₇ is selected from the group consisting of Ala, Ser, Gly, Thr, Pro, Glu, Lys and Val; and 1 of AA₁, AA₂, AA₃, AA₄, AA₅ and AA₆ is replaced providing that:
   when AA₁ is replaced, it is replaced by an amino acid selected from the group formed by Asp, Gln and Asn;
   when AA₂ is replaced, it is replaced by an amino acid selected from the group formed by Asp and Gln;
   when AA₃ is replaced, it is replaced by an amino acid selected from the group formed by Leu and Ile;
   when AA₄ is replaced, it is replaced by an amino acid selected from the group formed by Glu, Asn and Asp;
   when AA₅ is replaced, it is replaced by Lys; and
   when AA₆ is replaced, it is replaced by an amino acid selected from the group formed
   by Lys and His;
and wherein W, X, Y, Z, m, n, p, q, R₁ and R₂ are as specified for formula (I). In one embodiment, AA₁ is replaced and thus the compound of the invention can be represented by the following formula:

   R₁-Wₘ-Xₙ-AA₁-Glu-Met-Gln-Arg-Arg-AA₇-Yₚ-Z_{q}-R₂ (XII)

   its stereoisomers and/or its cosmetically or pharmaceutically acceptable salts, wherein: AA₇ is selected from the group consisting of Ala, Ser, Gly, Thr, Pro, Glu, Lys and Val; AA₁ is an amino acid selected from the group formed by Asp, Gln and Asn; and wherein, W, X, Y, Z, m, n, p, q, R₁ and R₂ are as specified for formula (I). In one embodiment, AA₁ is replaced by Asn. In one embodiment, AA₂ is replaced. In one embodiment, AA₃ is replaced. In one embodiment, AA₄ is replaced. In one embodiment, AA₄ is replaced by Asp. In one embodiment, AA₅ is replaced. In one embodiment, AA₆ is replaced. Thus the invention relates to a compound of represented by the following formula,

   R₁-Wₘ-Xₙ-AA₁-Glu-Met-AA₄-Arg-Arg-AA₇-Yₚ-Z_{q}-R₂ (XIII),

   its stereoisomers and/or its cosmetically or pharmaceutically acceptable salts, wherein:
   AA₁ is Glu; AA₄ is Gin; AA₇ is selected from the group consisting of Ala, Ser, Gly, Thr, Pro, Glu, Lys and Val;
   1 of AA₁ and AA₄ is replaced providing that:
      when AA₁ is replaced, it is replaced by Asn;
      when AA₄ is replaced, it is replaced by Asp;
   wherein W, X, Y, Z, m, n, p, q, R₁ and R₂ are as specified for formula (I). In these embodiments, AA₇ can be selected from the group consisting of Ala, Ser, Gly, Thr and Val.

In one embodiment, the compound is
of formula (I) wherein 2 of AA₁ to AA₆ are replaced and a = 1 and b = 0. In this embodiment, the compound of the invention can be represented by the following formula:

R₁-Wₘ-Xₙ- AA₁-AA₂-AA₃-AA₄-AA₅-AA₆-AA₇-Yₚ-Z_{q}-R₂ (XIV)

its stereoisomers and/or its cosmetically or pharmaceutically acceptable salts, wherein:
AA₁ is Glu; AA₂ is Glu; AA₃ is Met; AA₄ is Gln; AA₅ is Arg; AA₆ is Arg; AA₇ is selected from the group consisting of Ala, Ser, Gly, Thr, Pro, Glu, Lys and Val; and 2 of AA₁, AA₂, AA₃, AA₄, AA₅ and AA₆ are replaced providing that:
   when AA₁ is replaced, it is replaced by an amino acid selected from the group formed by Asp, Gln and Asn;
   when AA₂ is replaced, it is replaced by an amino acid selected from the group formed by Asp and Gin;
   when AA₃ is replaced, it is replaced by an amino acid selected from the group formed by Leu and Ile;
   when AA₄ is replaced, it is replaced by an amino acid selected from the group formed by Glu, Asn and Asp;
   when AA₅ is replaced, it is replaced by Lys; and
   when AA₆ is replaced, it is replaced by an amino acid selected from the group formed by Lys and His;
and wherein W, X, Y, Z, m, n, p, q, R₁ and R₂ are as specified for formula (I). In one embodiment, AA₁ and AA₄ are replaced and thus the compound of the invention can be represented by the following formula:

   R₁-Wₘ-Xₙ-AA₁-Glu- Met- AA₄-Arg-Arg-AA₇-Yₚ-Z_{q}-R₂ (XV)

   its stereoisomers and/or its cosmetically or pharmaceutically acceptable salts, wherein:
   AA₇ is selected from the group consisting of Ala, Ser, Gly, Thr, Pro, Glu, Lys and Val; AA₁ is an amino acid selected from the group formed by Asp, Gln and Asn; AA₄ is an amino acid selected from the group formed by Glu, Asn and Asp; and wherein W, X, Y, Z, m, n, p, q, R₁ and R₂ are as specified for formula (I). In one embodiment, AA₁ is Asp and AA₄ is Glu. In these embodiments, AA₇ can be selected from the group consisting of Ser, Gly, Thr, Pro, Glu, Lys and Val, AA₇ can be selected from the group consisting of Ala, Ser, Gly, Thr and Val. Further, AA₇ can be Ala.

In one embodiment, the compound is
of formula (I), wherein a = 1 and b = 1. In this embodiment, the compound of the invention represents a peptide which comprises 8, 9 or 10 amino acids linked in a chain and can be represented by the following formula:

R₁-Wₘ-Xₙ-AA₁-AA₂-AA₃-AA₄-AAₛ-AA₆-AA₇-AA₈-Yₚ-Z_{q}-R₂ (XVI)

its stereoisomers and/or its cosmetically or pharmaceutically acceptable salts, wherein AA₁, AA₂, AA₃, AA₄, AA₅, AA₆, AA₇, AA₈, a, b, W, X, Y, Z, m, n, p, q, R₁ and R₂ are as specified for formula (I). In this embodiment, AA₇ can be selected from the group consisting of Ala, Ser and Gly. In one embodiment, AA₇ is Ala.

In one embodiment, the compound is
of formula (I), wherein 0 of AA₁ to AA₆ is replaced and a = 1 and b = 1. In this embodiment, the compound of the invention can be represented by the following formula:

R₁-Wₘ-Xₙ-Glu-Glu-Met-Gln-Arg-Arg-AA₇-AA₈-Yₚ-Z_{q}-R₂ (XVII)

its stereoisomers and/or its cosmetically or pharmaceutically acceptable salts, wherein:
AA₇ is selected from the group consisting of Ala, Ser, Gly, Thr, Pro, Glu, Lys and Val; AA₈ is selected from the group consisting of Asp, Glu, Asn and Gln; and wherein W, X, Y, Z, m, n, p, q, R₁ and R₂ are as specified for formula (I). In one embodiment, AA₇ is selected from the group consisting of Ala, Ser, Gly, Thr, Pro, Glu, and Lys. In one embodiment, AA₇ is selected from the group consisting of Ala, Ser, Gly, Thr and Val.

In one embodiment, the compound is
of formula (I), wherein 1 of AA₁ to AA₆ is replaced and a = 1 and b = 1. In this embodiment, the compound of the invention can be represented by the following formula:

R₁-Wₘ-Xₙ- AA₁-AA₂-AA₃-AA₄-AA₅-AA₆-AA₇-AA₈-Yₚ-Z_{q}-R₂ (XVIII)

its stereoisomers and/or its cosmetically or pharmaceutically acceptable salts, wherein:
AA₁ is Glu; AA₂ is Glu; AA₃ is Met; AA₄ is Gln; AA₅ is Arg; AA₆ is Arg; AA₇ is selected from the group consisting of Ala, Ser, Gly, Thr, Pro, Glu, Lys and Val; AA₈ is selected from the group consisting of Asp, Glu, Asn and Gln; and 1 of AA₁, AA₂, AA₃, AA₄, AA₅ and AA₆ is replaced providing that:
   when AA₁ is replaced, it is replaced by an amino acid selected from the group formed by Asp, Gln and Asn;
   when AA₂ is replaced, it is replaced by an amino acid selected from the group formed by Asp and Gln;
   when AA₃ is replaced, it is replaced by an amino acid selected from the group formed by Leu and Ile;
   when AA₄ is replaced, it is replaced by an amino acid selected from the group formed by Glu, Asn and Asp;
   when AA₅ is replaced, it is replaced by Lys; and
   when AA₆ is replaced, it is replaced by an amino acid selected from the group formed by Lys and His;
and wherein W, X, Y, Z, m, n, p, q, R₁ and R₂ are as specified for formula (I). In one embodiment, AA₁ is replaced and thus the compound of the invention can be represented by the formula:

   R₁-Wₘ-Xₙ- AA₁-Glu-Met-Gln-Arg-Arg-AA₇-AA₈-Yₚ-Z_{q}-R₂ (XIX)

   its stereoisomers and/or its cosmetically or pharmaceutically acceptable salts, wherein:
   AA₇ is selected from the group consisting of Ala, Ser, Gly, Thr, Pro, Glu, Lys and Val; AA₈ is selected from the group consisting of Asp, Glu, Asn and Gl; AA₁ is an amino acid selected from the group formed by Asp, Gln and Asn; and wherein W, X, Y, Z, m, n, p, q, R₁ and R₂ are as specified for formula (I). In one embodiment, AA₁ is replaced by Asp or Gln. In one embodiment, AA₂ is replaced. In one embodiment, AA₃ is replaced. In one embodiment, AA₃ is replaced by Leu. In one embodiment, AA₄ is replaced. In one embodiment, AA₄ is replaced by Glu, Asn or Asp. In one embodiment, AA₅ is replaced. In one embodiment, AA₆ is replaced. In one embodiment, AA₆ is replaced by Lys or His. The invention relates to a compound that can be represented by the formula:,

   R₁-Wₘ-Xₙ-AA₁-Glu-AA₃-AA₄-Arg-AA₆-AA₇-AA₈-Yₚ-Z_{q}-R₂ (XX),

   its stereoisomers and/or its cosmetically or pharmaceutically acceptable salts, wherein:
   AA₁ is Glu; AA₃ is Met; AA₄ is Gln; AA₅ is Arg; AA₇ is selected from the group consisting of Ala, Ser, Gly, Thr, Pro, Glu, Lys and Val; AA₈ is selected from the group consisting of Asp, Glu and Asn;
   1 of AA₁, AA₃, AA₄ and AA₆ is replaced providing that:
      when AA₁ is replaced, it is replaced by an amino acid selected from the group formed by Asp and Gln;
      when AA₃ is replaced, it is replaced by Leu;
      when AA₄ is replaced, it is replaced by an amino acid selected from the group formed by Glu, Asp and Asn;
      when AA₆ is replaced, it is replaced by an amino acid selected from the group formed by Lys and His;
   wherein W, X, Y, Z, m, n, p, q, R₁ and R₂ are as specified for formula (I).

Compounds of the invention include one or more compounds selected from the compounds listed in Table 2, in which the sequence identifier of each amino acid sequence is detailed, their stereoisomers, and/or their cosmetically or pharmaceutically acceptable salts.

| Table 2 | |
|---|---|
| Glu-Glu-Met-Gln-Arg-Arg | SEQ ID NO. 2* |
| Glu-Glu-Met-Gln-Arg-Arg-Ala | SEQ ID NO. 3 |
| Glu-Glu-Met-Gln-Arg-Arg-Ala-Asp | SEQ ID NO. 4 |
| Asp-Glu-Met-Gln-Arg-Arg-Ala-Asp | SEQ ID NO. 5 |
| Gln-Glu-Met-Gln-Ar-Arg-Ala-Asp | SEQ ID NO. 6 |
| Glu-Glu-Leu-Gln-Arg-Arg-Ala-Asp | SEQ ID NO. 7 |
| Glu-Glu-Met-Glu-Arg-Arg-Ala-Asp | SEQ ID NO. 8 |
| Glu-Glu-Met-Asn-Arg-Arg-Ala-Asp | SEQ ID NO. 9 |
| Glu-Glu-Met-Asp-Arg-Arg-Ala-Asp | SEQ ID NO. 10 |
| Glu-Glu-Met-Gln-Arg-Lys-Ala-Asp | SEQ ID NO. 11 |
| Glu-Glu-Met-Gln-Arg-Arg-Ser-Asp | SEQ ID NO. 12 |
| Glu-Glu-Met-Gln-Arg-Arg-Gly-Asp | SEQ ID NO. 13 |
| Glu-Glu-Met-Gln-Arg-Arg-Ala-Glu | SEQ ID NO. 14 |
| Glu-Glu-Met-Gln-Arg-Arg-Ala-Asn | SEQ ID NO. 15 |
| Glu-Glu-Met-Gln-Arg-Arg-Ala-Gln | SEQ ID NO. 16 |
| Glu-Glu-Met-Gln-Arg-His-Ala-Asp | SEQ ID NO. 17 |
| Glu-Glu-Met-Gln-Arg-Arg-Thr-Asp | SEQ ID NO. 18 |
| Glu-Glu-Met-Gln-Arg-Arg-Pro-Asp | SEQ ID NO. 19 |
| Glu-Glu-Met-Gln-Arg-Arg-Glu-Asp | SEQ ID NO. 20 |
| Glu-Glu-Met-Gln-Arg-Arg-Lys-Asp | SEQ ID NO. 21 |
| Glu-Glu-Met-Gln-Arg-Arg-Ser | SEQ ID NO. 22 |
| Glu-Glu-Met-Gln-Arg-Arg-Gly | SEQ ID NO. 23 |
| Glu-Glu-Met-Glu-Arg-Arg-Ala | SEQ ID NO. 24 |
| Asp-Glu-Met-Glu-Arg-Arg-Ala | SEQ ID NO. 25 |
| Glu-Glu-Met-Gln-Arg-Arg-Thr | SEQ ID NO. 26 |
| Glu-Glu-Met-Asp-Arg-Arg-Ala | SEQ ID NO. 27 |
| Asn-Glu-Met-Gln-Arg-Arg-Ala | SEQ ID NO. 28 |
| Glu-Glu-Met-Gln-Arg-Arg-Val | SEQ ID NO. 29 |
| Glu-Glu-Met-Gln-Arg-Lys | SEQ ID NO. 30* |
| Glu-Glu-Met-Gln-Lys-Arg | SEQ ID NO. 31* |
| Glu-Glu-Met-Glu-Arg-Arg | SEQ ID NO. 32* |
| Glu-Glu-Met-Asn-Arg-Arg | SEQ ID NO. 33* |
| Glu-Asp-Met-Gln-Arg-Arg | SEQ ID NO. 34* |
| Glu-Gln-Met-Gln-Arg-Arg | SEQ ID NO. 35* |
| Asp-Glu-Met-Gln-Arg-Arg | SEQ ID NO. 36* |
| Gln-Glu-Met-Gln-Arg-Arg | SEQ ID NO. 37* |
| Glu-Glu-Met-Gln-Arq-His | SEQ ID NO. 38* |
| Glu-Glu-Met-Gln-Lys-Lys | SEQ ID NO. 39* |
| Glu-Glu-Ile-Glu-Arg-Arg | SEQ ID NO. 40* |
| Glu-Glu-Leu-Gln-Arg-Arg | SEQ ID NO. 41* |

| | |
|---|---|
| *not of the invention | |

In particular, compounds of the invention include one or more compounds selected from the compounds listed in Table 3, in which the sequence identifier of each amino acid sequence is detailed, their stereoisomers, and/or their cosmetically or pharmaceutically acceptable salts.

| Table 3 | |
|---|---|
| Glu-Glu-Met-Gln-Arg-Arg | SEQ ID NO. 2* |
| Glu-Glu-Met-Gln-Arg-Arg-Ala-Asp | SEQ ID NO. 4 |
| Gln-Glu-Met-Gln-Arg-Arg-Ala-Asp | SEQ ID NO. 6 |
| Glu-Glu-Met-Glu-Arg-Arg-Ala-Asp | SEQ ID NO. 8 |
| Glu-Glu-Met-Gln-Arg-Arg-Ala-Glu | SEQ ID NO. 14 |
| Glu-Glu-Met-Gln-Arg-Arg-Ala-Asn | SEQ ID NO. 15 |
| Glu-Glu-Met-Gln-Arg-Lys | SEQ ID NO. 30* |
| Glu-Glu-Met-Gln-Arg-His | SEQ ID NO. 38* |

| | |
|---|---|
| *not of the invention | |

In the amino acid sequences of Tables 2 and 3, each sequence according to formula (1), R₁ and R₂ are H and OH, respectively. Compounds of the invention include sequences of Tables 2 and 3 with their N- and C- terminals modified by the other R₁ and R₂ groups, respectively, as defined herein for formula (1). For example, compounds of the invention include sequences of Table 2 and 3 in which the C-terminal amino acid residue optionally terminates (is modified) with R₁ as defined above for formula (1), where R₁ is not H. Also, compounds of the invention include sequences of Table 2 and 3 in which the N-terminal amino acid residue optionally terminates (is modified) with R₂ as defined above for formula (1), where R₂ is not OH.

Thus the compound according to formula (I) can be an amino acid sequence selected from SEQ ID NOS: 3 to 29, or its stereoisomers and/or its cosmetically or pharmaceutically acceptable salts, wherein, optionally, said sequence has its N-terminal modified by R₁ where R₁ is not H and/or its C-terminal modified by R₂ where R₂ is not OH. In one embodiment, the amino acid sequence is selected from SEQ ID NOS 4, 6, 8, 14 and 15. In one embodiment the the amino acid sequence is selected from SEQ ID NOS 3 to 29 or 5 to 29. In one embodiment the the amino acid sequence is selected from SEQ ID NOS 4, 6, 8, 14 and 15.

The compounds of this invention can exist as stereoisomers or mixtures of stereoisomers; for example, the amino acids which comprise them can have the configuration L-, D-, or be racemic independently of each other. Therefore, it is possible to obtain isomeric mixtures as well as racemic mixtures or diastereomeric mixtures, or pure diastereomers or enantiomers, depending on the number of asymmetric carbons and on which isomers or isomeric mixtures are present. The preferred structures of the compounds of the invention are pure isomers, i.e., enantiomers or diastereomers.

For example, when it is stated that AA₂ can be Glu, it is understood that AA₂ is selected from L-Glu, D-Glu or mixtures of both, racemic or non-racemic. The preparation procedures described in this document enable the person skilled in the art to obtain each of the stereoisomers of the compound of the invention by choosing the amino acid with the right configuration.

In the context of this invention, the term "amino acids" includes the amino acids encoded by the genetic code as well as non-encoded amino acids, whether they are natural or not. Examples of non-encoded amino acids are, without restriction, citrulline, ornithine, sarcosine, desmosine, norvaline, 4-aminobutyric acid, 2-aminobutyric acid, 2-aminoisobutyric acid, 6-aminohexanoyc acid, 1-naphthylalanine, 2-naphthylalanine, 2-aminobenzoic acid, 4-aminobenzoic acid, 4-chlorophenylalanine, 2,3-diaminopropionic acid, 2,4-diaminobutyric acid, cycloserine, carnitine, cystine, penicillamine, pyroglutamic acid, thienylalanine, hydroxyproline, allo-isoleucine, allo-threonine, isonipecotic acid, isoserine, phenylglycine, statin, β-alanine, norleucine, N-methyl amino acids, α-amino acids and β-amino acids, among others, as well as their derivatives. A list of non-natural amino acids can be found in the article *"*Unusual amino acids in peptide synthesis" by D.C. Roberts and F. Vellaccio, in The Peptides, Vol. 5 (1983), Chapter VI, Gross E. and MeienhoferJ., Eds., Academic Press, New York, USA or in the commercial catalogues of the companies specialized in the field.

In the context of this invention, when at least one of W, X, Y and/or Z is present, i.e. when at least one of n, m, p or q is not 0, it is understood that the nature of W, X, Y and/or Z does not hinder the activity of the compound of the invention, and, instead, contributes to it or has no effect on it. In one embodiment, W, X, Y and Z are each independently selected from the group consisting of Gly, Ala, Ile and Val.

In one embodiment of the invention each of m, n, p and q is 0, i.e. the compound of formula (I) (and/or of any one of formulae (II) to (X)) is a peptide which comprises 7 or 8 amino acids, AA₁ to AA₆, AA₁ to AA₇ or AA₁ to AA₈, linked in a chain. In one embodiment the sum of m, n, p and q is 1, i.e., i.e. the compound of formula (I) (and/or of any one of formulae (II) to (X)) is a peptide which comprises 8 or 9 amino acids linked in a chain. In one embodiment the sum of m, n, p and q is 2, i.e., i.e. the compound of formula (I) (and/or of any one of formulae (II) to (X)) is a peptide which comprises 9 or 10 amino acids linked in a chain.

Compounds of the invention include one or more compounds selected from the group of compounds listed in Table 4, their stereoisomers, and/or their cosmetically or pharmaceutically acceptable salts.

| Table 4 | |
|---|---|
| Ac-Glu-Glu-Met-Gln-Arg-Arg-NH₂ | PEP-1* |
| Ac-Glu-Glu-Met-Gln-Arg-Arg-Ala-NH₂ | PEP-2 |
| Ac-Glu-Glu-Met-Gln-Arg-Arg-Ala-Asp-NH₂ | PEP-3 |
| Ac-Glu-Glu-Met-Gln-Arg-Arg-Ala-Asp-OH | PEP-4 |
| Myr-Glu-Glu-Met-Gln-Arg-Arg-NH₂ | PEP-5* |
| Palm-Glu-Glu-Met-Gln-Arg-Arg-NH₂ | PEP-6* |
| Ac-Glu-Glu-Met-Gln-Arg-Arg-NHC₁₆H₃₃ | PEP-7* |
| Ac-Asp-Glu-Met-Gln-Arg-Arg-Ala-Asp-NH₂ | PEP-8 |
| Ac-Gln-Glu-Met-Gln-Arg-Arg-Ala-Asp-NH₂ | PEP-9 |
| Ac-Glu-Glu-Leu-Gln-Arg-Arg-Ala-Asp-NH₂ | PEP-10 |
| Ac-Glu-Glu-Met-Glu-Arg-Arg-Ala-Asp-NH₂ | PEP-11 |
| Ac-Glu-Glu-Met-Asn-Arg-Arg-Ala-Asp-NH₂ | PEP-12 |
| Ac-Glu-Glu-Met-Asp-Arg-Arg-Ala-Asp-NH₂ | PEP-13 |
| Ac-Glu-Glu-Met-Gln-Arg-Lys-Ala-Asp-NH₂ | PEP-14 |
| Ac-Glu-Glu-Met-Gln-Arg-Arg-Ser-Asp-NH₂ | PEP-15 |
| Ac-Glu-Glu-Met-Gln-Arg-Arg-Gly-Asp-NH₂ | PEP-16 |
| Ac-Glu-Glu-Met-Gln-Arg-Arg-Ala-Glu-NH₂ | PEP-17 |
| Ac-Glu-Glu-Met-Gln-Arg-Arg-Ala-Asn-NH₂ | PEP-18 |
| Ac-Glu-Glu-Met-Gln-Arg-Arg-Ala-Gln-NH₂ | PEP-19 |
| Ac-Glu-Glu-Met-Gln-Arg-His-Ala-Asp-NH₂ | PEP-20 |
| Ac-Glu-Glu-Met-Gln-Arg-Arg-Thr-Asp-NH₂ | PEP-21 |
| Ac-Glu-Glu-Met-Gln-Arg-Arg-Pro-Asp-NH₂ | PEP-22 |
| Ac-Glu-Glu-Met-Gln-Arg-Arg-Glu-Asp-NH₂ | PEP-23 |
| Ac-Glu-Glu-Met-Gln-Arg-Arg-Lys-Asp-NH₂ | PEP-24 |
| H-Glu-Glu-Met-Gln-Arg-Arg-Ala-Asn-NH₂ | PEP-25 |
| H-Glu-Glu-Met-Gln-Arg-Arg-Ala-Glu-NH₂ | PEP-26 |
| Palm-Glu-Glu-Met-Gln-Arg-Arg-Ala-Asp-NH₂ | PEP-27 |
| Palm-Glu-Glu-Met-Gln-Arg-Arg-Ala-Asn-NH₂ | PEP-28 |
| Myr-Glu-Glu-Met-Gln-Arg-Arg-Ala-Glu-NH₂ | PEP-29 |
| Ac-Glu-Glu-Met-Gln-Arg-Arg-Ala-Glu-OH | PEP-30 |
| Ac-Glu-Glu-Met-Glu-Arg-Arg-Ala-Asp-OH | PEP-31 |
| Ac-Gln-Glu-Met-Gln-Arg-Arg-Ala-Asp-OH | PEP-32 |
| Palm-Glu-Glu-Met-Gln-Arg-Arg-Ala-Asn-OH | PEP-33 |
| Ac-Glu-Glu-Met-Gln-Arg-Arg-Ala-Asp-NHC₆H₁₃ | PEP-34 |
| Ac-Glu-Glu-Met-Gln-Arg-Arg-Ala-Asp-NHC₁₆H₃₃ | PEP-35 |
| H-Glu-Glu-Met-Gln-Arg-Arg-Ala-Asp-NHC₆H₁₃ | PEP-36 |
| Ac-Glu-Glu-Met-Gln-Arg-Arg-Ser-NH₂ | PEP-37 |
| Ac-Glu-Glu-Met-Gln-Arg-Arg-Gly-NH₂ | PEP-38 |
| Ac-Glu-Glu-Met-Glu-Arg-Arg-Ala-NH₂ | PEP-39 |
| Ac-Asp-Glu-Met-Glu-Arg-Arg-Ala-NH₂ | PEP-40 |
| Ac-Glu-Glu-Met-Gln-Arg-Arg-Thr-NH₂ | PEP-41 |
| Ac-Glu-Glu-Met-Asp-Arg-Arg-Ala-NH₂ | PEP-42 |
| Ac-Asn-Glu-Met-Gln-Arg-Arg-Ala-NH₂ | PEP-43 |
| Ac-Glu-Glu-Met-Gln-Arg-Arg-Val-NH₂ | PEP-44 |
| Ac-Glu-Glu-Met-Gln-Arg-Lys-NH₂ | PEP-45* |
| Ac-Glu-Glu-Met-Gln-Lys-Arg-NH₂ | PEP-46* |
| Ac-Glu-Glu-Met-Glu-Arg-Arg-NH₂ | PEP-47* |
| Ac-Glu-Glu-Met-Asn-Arg-Arg-NH₂ | PEP-48* |
| Ac-Glu-Asp-Met-Gln-Arg-Arg-NH₂ | PEP-49* |
| Ac-Glu-Gln-Met-Gln-Arg-Arg-NH₂ | PEP-50* |
| Ac-Asp-Glu-Met-Gln-Arg-Arg-NH₂ | PEP-51* |
| Ac-Gln-Glu-Met-Gln-Arg-Arg-NH₂ | PEP-52* |
| Ac-Glu-Glu-Met-Gln-Arg-His-NH₂ | PEP-53* |
| Ac-Glu-Glu-Met-Gln-Lys-LyPEP-45 | PEP-54* |
| Ac-Glu-Glu-Ile-Glu-Arg-Arg-NH₂ | PEP-55* |
| H-Glu-Glu-Met-Gln-Arg-Arg-NH₂ | PEP-56* |
| H-Glu-Glu-Met-Gln-Arg-His-NH₂ | PEP-57* |
| Palm-Glu-Glu-Met-Gln-Arg-His-NH₂ | PEP-58* |
| Palm-Glu-Glu-Met-Gln-Arg-Lys-NH₂ | PEP-59* |
| Myr-Glu-Glu-Met-Gln-Arg-Lys-NH₂ | PEP-60* |
| Ac-Glu-Glu-Met-Gln-Arg-Arg-OH | PEP-61 * |
| Ac-Glu-Glu-Met-Gln-Arg-Lys-OH | PEP-62* |
| Ac-Glu-Glu-Leu-Gln-Arg-Arg-OH | PEP-63* |
| Ac-Glu-Glu-Met-Gln-Arg-Arg-NHC₆H₁₃ | PEP-64* |
| H-Glu-Glu-Met-Gln-Arg-Arg-NCH₆H₁₃ | PEP-65* |

| | |
|---|---|
| *Not of the invention | |

Compounds of the invention include one or more compounds selected from the group of compounds listed in Table 5, their stereoisomers, and/or their cosmetically or pharmaceutically acceptable salts.

| Table 5 | |
|---|---|
| Ac-Glu-Glu-Met-Gln-Arg-Arg-NH₂ | PEP-1* |
| Ac-Glu-Glu-Met-Gln-Arg-Arg-Ala-NH₂ | PEP-2 |
| Ac-Glu-Glu-Met-Gln-Arg-Arg-Ala-Asp-NH₂ | PEP-3 |
| Ac-Glu-Glu-Met-Gln-Arg-Arg-Ala-Asp-OH | PEP-4 |
| Myr-Glu-Glu-Met-Gln-Arg-Arg-NH₂ | PEP-5* |
| Palm-Glu-Glu-Met-Gln-Arg-Arg-NH₂ | PEP-6* |
| Ac-Glu-Glu-Met-Gln-Arg-Arg-NHC₁₆H₃₃ | PEP-7* |
| Ac-Asp-Glu-Met-Gln-Arg-Arg-Ala-Asp-NH₂ | PEP-8 |
| Ac-Gln-Glu-Met-Gln-Arg-Arg-Ala-Asp-NH₂ | PEP-9 |
| Ac-Glu-Glu-Leu-Gln-Arg-Arg-Ala-Asp-NH₂ | PEP-10 |
| Ac-Glu-Glu-Met-Glu-Arg-Arg-Ala-Asp-NH₂ | PEP-11 |
| Ac-Glu-Glu-Met-Asn-Arg-Arg-Ala-Asp-NH₂ | PEP-12 |
| Ac-Glu-Glu-Met-Asp-Arg-Arg-Ala-Asp-NH₂ | PEP-13 |
| Ac-Glu-Glu-Met-Gln-Arg-Lys-Ala-Asp-NH₂ | PEP-14 |
| Ac-Glu-Glu-Met-Gln-Arg-Arg-Ser-Asp-NH₂ | PEP-15 |
| Ac-Glu-Glu-Met-Gln-Arg-Arg-Gly-Asp-NH₂ | PEP-16 |
| Ac-Glu-Glu-Met-Gln-Arg-Arg-Ala-Glu-NH₂ | PEP-17 |
| Ac-Glu-Glu-Met-Gln-Arg-Arg-Ala-Asn-NH₂ | PEP-18 |
| Ac-Glu-Glu-Met-Gln-Arg-Arg-Ala-Gln-NH₂ | PEP-19 |
| Ac-Glu-Glu-Met-Gln-Arg-His-Ala-Asp-NH₂ | PEP-20 |
| Ac-Glu-Glu-Met-Gln-Arg-Arg-Thr-Asp-NH₂ | PEP-21 |
| Ac-Glu-Glu-Met-Gln-Arg-Arg-Pro-Asp-NH₂ | PEP-22 |
| Ac-Glu-Glu-Met-Gln-Arg-Arg-Glu-Asp-NH₂ | PEP-23 |
| Ac-Glu-Glu-Met-Gln-Arg-Arg-Lys-Asp-NH₂ | PEP-24 |
| H-Glu-Glu-Met-Gln-Arg-Arg-Ala-Glu-NH₂ | PEP-26 |
| Palm-Glu-Glu-Met-Gln-Arg-Arg-Ala-Asp-NH₂ | PEP-27 |
| Palm-Glu-Glu-Met-Gln-Arg-Arg-Ala-Asn-NH₂ | PEP-28 |
| Myr-Glu-Glu-Met-Gln-Arg-Arg-Ala-Glu-NH₂ | PEP-29 |
| Ac-Glu-Glu-Met-Glu-Arg-Arg-Ala-Asp-OH | PEP-31 |
| Ac-Gln-Glu-Met-Gln-Arg-Arg-Ala-Asp-OH | PEP-32 |
| Palm-Glu-Glu-Met-Gln-Arg-Arg-Ala-Asn-OH | PEP-33 |
| Ac-Glu-Glu-Met-Gln-Arg-Arg-Ala-Asp-NHC₆H₁₃ | PEP-34 |
| Ac-Glu-Glu-Met-Gln-Arg-Arg-Ala-Asp-NHC₁₆H₃₃ | PEP-35 |
| H-Glu-Glu-Met-Gln-Arg-Arg-Ala-Asp-NHC₆H₁₃ | PEP-36 |
| Ac-Glu-Glu-Met-Gln-Arg-Arg-Ser-NH₂ | PEP-37 |
| Ac-Glu-Glu-Met-Gln-Arg-Arg-Gly-NH₂ | PEP-38 |
| Ac-Glu-Glu-Met-Glu-Arg-Arg-Ala-NH₂ | PEP-39 |
| Ac-Asp-Glu-Met-Glu-Arg-Arg-Ala-NH₂ | PEP-40 |
| Ac-Glu-Glu-Met-Gln-Arg-Arg-Thr-NH₂ | PEP-41 |
| Ac-Glu-Glu-Met-Asp-Arg-Arg-Ala-NH₂ | PEP-42 |
| Ac-Asn-Glu-Met-Gln-Arg-Arg-Ala-NH₂ | PEP-43 |
| Ac-Glu-Glu-Met-Gln-Arg-Lys-NH₂ | PEP-45* |
| Ac-Glu-Glu-Met-Gln-Lys-Arg-NH₂ | PEP-46* |
| Ac-Glu-Glu-Met-Glu-Arg-Arg-NH₂ | PEP-47* |
| Ac-Glu-Glu-Met-Asn-Arg-Arg-NH₂ | PEP-48* |
| Ac-Glu-Glu-Leu-Gln-Arg-Arg-NH₂ | PEP-66* |
| Ac-Glu-Asp-Met-Gln-Arg-Arg-NH₂ | PEP-49* |
| Ac-Glu-Gln-Met-Gln-Arg-Arg-NH₂ | PEP-50* |
| Ac-Asp-Glu-Met-Gln-Arg-Arg-NH₂ | PEP-51* |
| Ac-Glu-Glu-Met-Gln-Lys-Lys-NH₂ | PEP-54* |
| Ac-Glu-Glu-Ile-Glu-Arg-Arg-NH₂ | PEP-55* |
| H-Glu-Glu-Met-Gln-Arg-Arg-NH₂ | PEP-56* |
| H-Glu-Glu-Met-Gln-Arq-His-NH₂ | PEP-57* |
| Palm-Glu-Glu-Met-Gln-Arg-Lys-NH₂ | PEP-59* |
| Myr-Glu-Glu-Met-Gln-Arg-Lys-NH₂ | PEP-60* |
| Ac-Glu-Glu-Met-Gln-Arg-Arg-OH | PEP-61 * |
| Ac-Glu-Glu-Met-Gln-Arg-Lys-OH | PEP-62* |
| H-Glu-Glu-Met-Gln-Arg-Arg-NHC₁₆H₃₃ | PEP-67* |

| | |
|---|---|
| *Not of the invention | |

The cosmetically or pharmaceutically acceptable salts of the compounds provided by the present invention are also found within the field of this invention. The term "cosmetically or pharmaceutically acceptable salts" means a salt recognized for its use in animals, for example, in mammals, and more specifically in human beings, and includes salts used to form base addition salts, either they are inorganic, for example and not restricted to, lithium, sodium, potassium, calcium, magnesium, manganese, copper, zinc or aluminium among others, or they are organic, for example and not restricted to, ethylamine, diethylamine, ethylenediamine, ethanolamine, diethanolamine, arginine, lysine, histidine or piperazine among others, or acid addition salts, either they are organic, for example and not restricted to, acetate, citrate, lactate, malonate, maleate, tartrate, fumarate, benzoate, aspartate, glutamate, succinate, oleate, trifluoroacetate, oxalate, pamoate or gluconate among others, or inorganic, for example and not restricted to, chloride, sulfate, borate or carbonate, among others. The nature of the salt is not critical, provided that it is cosmetically or pharmaceutically acceptable. The cosmetically or pharmaceutically acceptable salts of the compounds of the invention can be obtained by the conventional methods, well known in the prior art *[*Berge S.M. et al., "Pharmaceutical Salts", (1977), J. Pharm. Sci., 66, 1-19*].*

### Preparation procedures of the compounds of the invention

Synthesis of the compounds of the invention, their stereoisomers, mixtures thereof and/or their cosmetically or pharmaceutically acceptable salts can be carried out according to conventional methods, known in the prior art, such as solid phase peptide synthesis methods *[*Stewart J.M. and Young J.D., "Solid Phase Peptide Synthesis, 2nd edition", (1984), Pierce Chemical Company, Rockford, Illino*is;* Bodanzsky M. and Bodanzsky A., "The practice of Peptide Synthesis", (1994), Springer Verlag, Berl*in;* Lloyd-Williams P. et al., "Chemical Approaches to the Synthesis of Peptides and Proteins", (1997), CRC, Boca Raton, FL, USA*],* synthesis in solution, enzymatic synthesis *[*Kullmann W. "Proteases as catalysts for enzymic syntheses of opioid peptides", (1980), J.Biol.Chem., 255(17), 8234-8238*]* or any combination thereof. The compounds can also be obtained by fermentation of a bacterial strain, modified or unmodified by genetic engineering with the objective of producing the desired sequences, or by controlled hydrolysis of proteins with animal or plant origins, preferably plant, which results in free peptide fragments that contain the desired sequence.

For example, a method of obtaining the compounds of formula (I), their stereoisomers and mixtures thereof comprises the stages of:
- coupling of an amino acid, with the *N*-terminal end protected and the C-terminal end free, with an amino acid with the *N*-terminal end free and the C-terminal end protected or bound to a solid support;
- elimination of the protective group of the *N*-terminal end;
- repetition of the coupling sequence and elimination of the protective group of the *N*-terminal end until the desired peptide sequence is obtained;
- elimination of the protective group of the *C*-terminal end or cleavage of the solid support.

Preferably, the *C*-terminal end is bound to a solid support and the process is carried out in solid phase and, therefore, comprises the coupling of an amino acid with the *N*-terminal end protected and the *C*-terminal end free, with an amino acid with the *N*-terminal end free and the *C*-terminal end bound to a polymeric support; elimination of the protective group of the *N*-terminal end; and repetition of this sequence as many times as is necessary to thus obtain the compound of desired length, finally followed by the cleavage of the synthesized compound from the original polymeric support.

The functional groups of the side chains of the amino acids are maintained conveniently protected with temporary or permanent protective groups throughout synthesis, and can be unprotected simultaneously or orthogonally to the process of cleavage of the peptide from the polymeric support.

Alternatively, solid phase synthesis can be carried out using a convergent strategy coupling a peptide with the polymeric support or with a peptide or an amino acid previously bound to the polymeric support. Convergent synthesis strategies are widely known by persons skilled in the art and are described in Lloyd-Williams P. et al., "Convergent Solid-Phase Peptide Synthesis", (1993), Tetrahedron, 49(48), 11065-11133*.*

The process can comprise the additional stages of deprotection of the *N*-terminal and *C*-terminal ends and/or cleavage of the peptide from the polymeric support in an indiscriminate order, using standard procedures and conditions known in the prior art, after which the functional groups of these ends can be modified. The optional modification of the *N*-terminal and *C*-terminal ends can be carried out with the peptide of formula (I) anchored to the polymeric support or once the peptide has been separated from the polymeric support.

Optionally, R₁ can be introduced by the reaction of the *N*-terminal end of the compound of the invention with a R₁-X compound through a nucleophilic substitution reaction, in the presence of an adequate base and solvent, wherein the fragments that have the functional groups not involved in the N-C bond formation are suitably protected with temporary or permanent protective groups. R₁ is as defined above and X is a leaving group, for example and not restricted to, the tosyl group, the mesyl group and halogen groups among others.

Optionally and/or additionally, the R₂ radicals can be introduced by the reaction of a compound HR₂ with a complementary fragment which corresponds to the peptide of formula (I) in which R₂ is -OH in the presence of an adequate solvent and a base such as *N,N*-diisopropylethylamine (DIEA) or trimethylamine, or an additive such as 1-hydroxybenzotriazole (HOBt) or 1-hydroxyazabenzotriazole (HOAt), and a dehydrating agent such as a carbodiimide, a uronium salt, a phosphonium salt or amidinium salt, among others, or by prior formation of an acyl halide with, for example, thionyl chloride, and thereby obtaining a peptide according to the invention of formula (I), wherein the fragments that have the functional groups not involved in the N-C bond formation are suitably protected with temporary or permanent protective groups. Alternatively other R₂ radicals may be introduced by simultaneous incorporation to the peptide cleavage process from the polymeric carrier. R₂ is -OR₃, -NR₃R₄ or -SR₃, where R₃ and R₄ are as defined above.

A person skilled in the art would easily understand that the deprotection/cleavage steps of the *C*-terminal and *N*-terminal ends and their subsequent derivatization can be performed in a different order, according to the processes known in the prior art.

The term "protective group" relates to a group which blocks an organic functional group and which can be removed in controlled conditions. The protective groups, their relative reactivities and the conditions in which they remain inert are known to the person skilled in the art.

Examples of representative protective groups for the amino group are amides, such as amide acetate, amide benzoate, amide pivalate; carbamates such as benzyloxycarbonyl (Cbz or Z), 2-chlorobenzyl (CIZ), *para*-nitrobenzyloxycarbonyl (pNZ), *tert*-butyloxycarbonyl (Boc), 2,2,2-trichloroethyloxycarbonyl (Troc), 2-(trimethylsilyl)ethyl-oxycarbonyl (Teoc), 9-fluorenylmethyloxycarbonyl (Fmoc) or allyloxycarbonyl (Alloc), trityl (Trt), methoxytrityl (Mtt), 2,4-dinitrophenyl (Dnp), *N*-[1-(4,4-dimethyl-2,6-dioxocyclohex-1-ylidene)ethyl (Dde), 1-(4,4-dimethyl-2,6-dioxo-cyclohexylidene)-3-methylbutyl (ivDde), 1-(1-adamantyl)-1-methylethoxycarbonyl (Adpoc), among others, preferably Boc or Fmoc.

Examples of representative protective groups for the carboxyl group are esters, such as the *tert*-butyl ester (tBu), allyl ester (All), triphenylmethyl ester (Trt tester), cyclohexyl ester (cHx), benzyl ester (Bzl), *ortho*-nitrobenzyl ester, *para*-nitrobenzyl ester, *para*-methoxybenzyl ester, trimethylsilylethyl ester, 2-phenylisopropyl ester, fluorenylmethyl ester (Fm), 4-(*N*-[1-(4,4-dimethyl-2,6-dioxocyclohexylidene)-3-methylbutyl]amino) benzyl ester (Dmab), among others; preferred protective groups of the invention are the All, tBu, cHex, Bzl and Trt esters.

The side chains of trifunctional amino acids can be protected during the synthetic process with temporary or permanent protective groups orthogonal to the protective groups of the *N*-terminal and *C*-terminal ends.

The hydroxyl group of the tyrosine side chain can be protected with the 2-bromobenzyloxycarbonyl group (2-BrZ), tBu, All, Bzl or 2,6-dichlorobenzyl (2,6-diCIZ) among others. In a preferred embodiment, the protective group strategy used is the strategy wherein the amino groups are protected by Boc, the carboxyl groups are protected by Bzl, cHx or All esters and the tyrosine side chain is protected with 2-BrZ or Bzl. In another preferred embodiment, the protective group strategy used is the strategy wherein the amino groups are protected by Fmoc, the carboxyl groups are protected by tBu, All or Trt esters, the tyrosine side chain is protected by tBu.

The amino group of the tryptophan side chain can be protected, for example, by the formyl group (For) or Boc. In one embodiment, when the amino group is protected by Fmoc, and the tryptophan side chain can be: unprotected, i.e. the amino acid is incorporated as Fmoc-Trp-OH; protected by Boc, i.e. the amino acid is incorporated as Fmoc-Trp(Boc)-OH; or protected by For, i.e. the amino acid is incorporated as Fmoc-Trp(For)-OH. In one embodiment, the amino group is protected by Boc, and the tryptophan side chain can be protected by For, i.e. the amino acid is incorporated as Boc-Trp(For)-OH.

Examples of these and other protective groups, their introduction and removal, can be found in the literature *[*Atherton B. and Sheppard R.C., "Solid Phase Peptide Synthesis: A practical approach", (1989), IRL Oxford University Press*].* The term "protective groups" also includes the polymeric supports used in solid phase synthesis.

When synthesis takes place totally or partially in solid phase, the possible solid supports used in the process of the invention involve polystyrene support, polyethylene glycol grafted to polystyrene and similar, for example and not restricted to, *p-*methylbenzhydrylamine resins (MBHA) *[*Matsueda G.R. et al., "A p-methylbenzhydrylamine resin for improved solid-phase synthesis of peptide amides", (1981), Peptides, 2, 45-50*],* 2-chlorotrityl resins *[*Barlos K. et al., "Darstellung geschützter Peptid-Fragmente unter Einsatz substituierter Triphenylmethyl-Harze", (1989), Tetrahedron Lett., 30, 3943-3946*;* Barlos K. et al., "Veresterung von partiell geschützten Peptid-Fragmenten mit Harzen. Einsatz von 2-Chlorotritylchlorid zur Synthese von Leu1-Gastrin I", (1989), Tetrahedron Lett., 30, 3947-3951*],* TentaGel^{□} resins (Rapp Polymere GmbH), ChemMatrix^{□} resins (Matrix Innovation, Inc) and similar, which may or may not include a labile linker, such as 5-(4-aminomethyl-3,5-dimethoxyphenoxy) valeric acid (PAL) *[*Albericio F. et al., "Preparation and application of the 5-(4-(9-fluorenylmethyloxycarbonyl) aminomethyl-3,5-dimethoxy-phenoxy)valeric acid (PAL) handle for the solid-phase synthesis of C-terminal peptide amides under mild conditions", (1990), J. Org. Chem., 55, 3730-3743*],* 2-[4-aminomethyl-(2,4-dimethoxyphenyl)] phenoxyacetic acid (AM) *[*Rink H., "Solid-phase synthesis of protected peptide fragments using a trialkoxy-diphenyl-methylester resin" , (1987), Tetrahedron Lett., 28, 3787-3790*], [*Wang S.S., "p-Alkoxybenzyl Alcohol Resin and p-Alkoxybenzyloxycarbonylhydrazide Resin for Solid Phase Synthesis of Protected Peptide Fragments", (1973), J.Am.Chem.Soc., 95, 1328-1333*]* and similar, which enable simultaneous deprotection and cleavage of the compound from the polymeric support.

### Applications

The present invention is based, in part, on the finding that a compound of formula (I) is effective in maintaining and/or improving both the physical barrier function of the skin and the immunological barrier function of the skin. The physical barrier function of the skin is also referred to herein as the physical barrier of the skin, the skin's permeability barrier or simply the skin barrier. This barrier is provided by the stratum corneum and the tight junctions in the epidermis. The immunological barrier function of the skin is related in part to the microbial equilibrium of the skin and includes the skin's ability to provide an innate immune response to infections and opportunistic pathogens. The compounds of the invention have a dual facetted capability in relation to the barrier functions of the skin and thus are particularly useful in the protection and/or the treatment and/or care of the skin, hair, nails and/or mucous membranes. In particular, the protection, treatment and/or care is that of the skin. In the context of this invention, skin includes the skin of the whole body including the skin of the face (including skin around the eyes), neckline, neck, décolletage, arms, hands, legs, feet, thighs, hips, buttocks, stomach, torso and genital area (in particular the male genital area). In the context of this invention, muocous membranes include the mucous membranes of the vaginal area.

In one aspect, the invention provides the use of a compound of formula (I), its stereoisomers and/or its cosmetically or pharmaceutically acceptable salts, for the treatment and/or care of the skin, hair, nails and/or mucous membranes, as defined in the appended claims. The treatment and/or care may be of skin which has impaired barrier functions. Impaired barrier functions may be due to: external or environmental factors (e.g. temperature, humidity, exposure to UV); chemical damage (e.g. detergents, pollutants, allergens); physical damage (e.g. friction, scratches, burns). Impaired barrier functions may be due also to: intrinsic or internal factors such as hormonal changes; medical treatments (e.g. antibiotics, chemoradio therapy, dermatological peeling); illness; psychological stress or age.

The use may be cosmetic, i.e. non-therapeutic. The invention provides for the cosmetic, non-therapeutic use of a compound of formula (I), its stereoisomers and/or its cosmetically acceptable salts, or a cosmetic composition comprising a compound of formula (I), its stereoisomers and/or its cosmetically acceptable salts, for the cosmetic, non-therapeutic treatment and/or care of the skin, hair, nails and/or mucous membranes.

In one embodiment, the invention provides the use of a compound of formula (I), its stereoisomers and/or its cosmetically or pharmaceutically acceptable salts, to reduce the loss of water and electrolytes from the skin. Loss of water and electrolytes from the skin can be due to an impaired skin barrier function. Further, the invention provides the use of the compound of the invention to maintain and/or improve skin hydration, i.e. maintain and/or improve skin moisturization. In particular, it has been found that the compound of the invention is effective in the treatment of dry skin. In one embodiment, the invention provides the use of the compound of the invention to prevent or reduce desquamation of the skin. In one embodiment, the invention provides the use of the compound of the invention to prevent or reduce desquamation of the skin in a subject suffering from dry skin or having a tendency towards dry skin. The invention provides for the cosmetic, non-therapeutic use of a compound of formula (I) its stereoisomers and/or its cosmetically acceptable salts to: reduce the loss of water and electrolytes from the skin; maintain and/or improve skin moisturization; prevent or reduce desquamation of the skin; and/or prevent or reduce desquamation of the skin in a subject suffering from dry skin or having a tendency towards dry skin.

In one embodiment, the invention provides the use of a compound of formula (I), its stereoisomers and/or its cosmetically or pharmaceutically acceptable salts, to maintain and/ or improve skin microrelief and/or prevent adverse changes in skin microrelief. The compounds of the invention are useful in promoting and improving skin structure, skin strength and skin cohesion by improving and/or maintaining skin hydration and skin moisturization. This results in benefits to the skin microrelief. Adverse changes in skin microrelief can be due to impairment of the skin barrier function and such changes include an increase in skin roughness, i.e. a reduction in or loss of skin softness and/or a reduction in or loss of the skin's velvety aspect. As used herein by an increase in skin roughness is meant a reduction in or loss of skin softness and/or a reduction in or loss of the skin's velvety aspect. An increase in skin roughness gives the skin the aesthetic impression of aging. Thus, in one embodiment, the invention provides for the use of a compound of formula (I), its stereoisomers and/or its cosmetically or pharmaceutically acceptable salts, in the prevention or the treatment of the symptoms of skin aging, and in particular, the prevention or the treatment of changes in skin microrelief due to an impaired barrier function. In one embodiment, the invention provides the use of a compound of formula (I) to maintain and/or improve skin softness and/or the skin's velvety aspect. This result is that the skin has a younger aspect. The invention provides for the cosmetic, non-therapeutic use of a compound of formula (I) its stereoisomers and/or its cosmetically acceptable salts to: maintain, improve or prevent adverse changes in skin microrelief, in particular adverse changes in skin microrelief due to an impaired barrier function; and/or maintain and/or improve the softness of the skin and/or the velvety aspect of the skin.

In one embodiment, the invention provides the use of a compound of formula (I), its stereoisomers and/or its cosmetically or pharmaceutically acceptable salts, for maintaining and/or improving the physical barrier function of the skin and the immunological barrier function of the skin. As a result, the compounds of the invention are able to maintain and/or improve skin health and/or protect the skin from environmental hazards. By protect from environmental hazards is meant to alleviate or prevent any adverse effects relating to environmental hazards. Environmental hazards include chemical irritants, pollutants and pathogens. The ability of the compound to maintain and/or improve skin health and/or protect the skin from environmental hazards has implications for the maintenance and improvement of aesetheic qualities of the skin. The compounds of the invention can be used, for example, for the maintenance and/or improvement of the smooth texture of the skin, the prevention of defects of the skin (such as blemishes, bumps, blackheads and whiteheads); the maintenance and/or improvement of skin hydration including the prevention of flaking, cracking and/or roughness of the skin; the maintenance and/or improvement of luminosity of the skin due to hydration or structure of the skin; the maintenance and/or improvement of good sensation in the skin, including the prevention of itching, burning, stinging, pulling or tightness sensations; and/or the maintenance and/or improvement of even skin colour including the absence of redness of the skin. In one embodiment, the invention provides the cosmetic, non-therapeutic use of a compound of formula (I), its stereoisomers and/or its cosmetically acceptable salts, for maintaining and/or improving the physical barrier function of the skin and the immunological barrier function of the skin. In one embodiment, the invention provides the cosmetic, non-therapeutic use of a compound of formula (I), its stereoisomers and/or its cosmetically acceptable salts, for maintaining and/or improving the physical barrier function of the skin. In one embodiment, the invention provides for the cosmetic, non-therapeutic use of a compound of formula (I), its stereoisomers and/or its cosmetically acceptable salts, to maintain and/or improve skin health and protect the skin from environmental hazards.

In one embodiment, the invention provides for the use of a compound of formula (I), its stereoisomers and/or its cosmetically or pharmaceutically acceptable salts to protect the skin from chemical irritants typically contained in cosmetics (including makeup and moisturisers) and hygiene products (including cleansing products). By chemical irritant is meant a chemical or agent which causes irritation to the skin. Such chemical irritants can damage the barrier functions of the skin and they include: surfactants; oxidants; solvents; preservatives; fatty acids or alcohols; chemical sunscreens; ethoxylated compounds and other formaldehyde releasers; fragrances; and/or chemical peelings. Surfactants include alkyl sulfates (like sodium dodecyl sulfate or sodium laureth sulfate), alkyl benzene sulfonates, quaternized ammonium salts and cocamidopropyl betaine. Oxidants include sodium hypochlorite. Solvents include acetone and ethyl alcohol. Preservatives include 2-phenoxyethanol, glycols (butylene glycol, pentylene glycol or caprylyl glycol among others), methylisothizolinone or parabens. Fatty acids or alcohols include cetearyl alcohol, stearyl alcohol or stearic acid. Chemical sunscreens include octocrylene. Ethoxylated compounds and other formaldehyde releasers include bronopol, diazolidinyl urea and imidazolidinyl urea. Fragrances include herbal extracts, essential oils and perfumes. Chemical peelings include citric acid, lactic acid, ascorbic acid, glycolic acid and mandelic acid. It has been found that the compounds of the invention can restore and even improve the physical skin barrier function following damage caused by such irritants. As a result, the compounds of the invention are particularly useful in topical or transdermal cosmetic and pharmaceutical compositions comprising chemical irritants. The invention provides for the cosmetic, non-therapeutic use of a compound of formula (I) its stereoisomers and/or its cosmetically acceptable salts to protect the skin from chemical irritants. In particular, the invention provides for the cosmetic, non-therapeutic use of a compound of formula (I) its stereoisomers and/or its cosmetically acceptable salts to protect the skin from surfactants.

In one embodiment, the invention provides the use of a compound of formula (I), its stereoisomers and/or its cosmetically or pharmaceutically acceptable salts, for protecting the skin from pollutants and pathogens. By pollutants is meant external pollutants and these include: skin allergens; impurities from skin care products; chlorinated compounds in treated water; and air pollutants such as polycyclic aromatic hydrocarbons, volatile organic compounds, nitrogen oxides, particulate matter and heavy metals. The pathogens may include one or more of a bacteria, a virus and/or a fungi. The bacteria may be one or more of: Staphylococcus aureus, Propionibacterium acnes, Propionibacterium granulosum, Pseudomonas aeruginosa, Streptococcus pyogenes, Corynebacterium minutissimum, Corynebacterium tenuis, Corynebacterium xerosis, Pasteurella multocida, Capnocytophaga canimorsus, Bartonella henselae, Bartonella quintana, Bartonella bacilliformis, Klebsiella rhinoscleromatis, Vibrio vulnificus, Acinetobacter spp., Arcanobacterium haemolyticum, Haemophilus influenza, Erysipelothrix rhusiopathiae, Bacillus anthracis, Helicobacter cinaedi, Gardnerella vaginalis. The virus may be one or more of: Papillomavirus, Herpes zoster, Herpes simplex and Varicella zoster. The fungus may be one or more of Malassezia spp. (like Malassezia dermatis, Malassezia furfur, Malassezia globosa, Malassezia japonica, Malassezia obtusa, Malassezia restricta, Malassezia slooffiae, Malassezia sympodialis, Malassezia yamatoensis), Trichophyton spp. (like Trichophyton rubrum, Trichophyton tonsurans, Trichophyton mentagrophytes, Trichophyton interdigitale), Candida albicans and Microsporum spp. It is believed that the compounds of the invention can protect the skin from pollutants and/or pathogens due to their ability to maintain and improve, i.e. strengthen, the physical barrier of the skin. Further, it is believed that maintenance and/or improvement, i.e. strengthening, of the physical barrier of the skin is due to ability of the compound of the invention to tighten the cell-cell junctions, including tight cell junctions. In one embodiment, the invention provides the cosmetic, non-therapeutic use of a compound of formula (I), its stereoisomers and/or its cosmetically acceptable salts, for protecting the skin from pollutants and pathogens.

In one embodiment, the invention provides the use of a compound of formula (I), its stereoisomers and/or its cosmetically or pharmaceutically acceptable salts, to maintain and/or improve the immunological barrier of the skin.

In one embodiment, the invention provides the use of a compound of formula (I), its stereoisomers and/or its cosmetically or pharmaceutically acceptable salts, to maintain and/or improve the innate immune defense mechanism of the skin. The compound of the invention is useful in protecting the skin against infection and preventing or inhibiting opportunistic pathogens from colonising on the skin. This has implications for skin health and the aesthetic properties of skin associated with same. For example, this has implications for the maintenance and/or improvement of properites such as: good sensation in the skin, including the prevention of itching, burning, stinging, pulling or tightness sensations; and/or even skin colour including the absence of redness of the skin. In one embodiment, the invention provides the cosmetic, non-therapeutic use of a compound of formula (I), its stereoisomers and/or its cosmetically acceptable salts, for maintaining and/or improving the innate immune defense mechanism of the skin.

In one embodiment, the invention provides the use of a compound of formula (I), its stereoisomers and/or its cosmetically or pharmaceutically acceptable salts, to maintain the microbial equilibrium of the skin. By maintaining the microbial equilibrium means maintaining the relative compositions of the different microbes (i.e. the different microbial communities) present in healthy or normal skin. The set of microbial communities present in the skin is known as the skin microbiota. For example, the compound of formula (I) can be used to stimulate the presence of microorganisms on the skin which produce beneficial effects (such as commensal and/or symbiotic microrganisms) in competition with harmful microorganisms (such as pathogenic microrganisms). In one embodiment, the compounds of the invention can be used to stimulate the presence or growth of *Staphylococcus epidermidis* on the skin, thus inhibiting the colonisation of the skin with undesirable microorganisms such as *S. Aureus* and/or *P. Acnes.* This has implications for skin health and the aesthetic properties of skin associated with same. For example, the compound of the invention is useful for the maintenance and/or improvement of properites such as: good sensation in the skin, including the prevention of itching, burning, stinging, pulling or tightness sensations; and/or even skin colour including the absence of redness of the skin. In one embodiment, the invention provides the cosmetic, non-therapeutic use of a compound of formula (I), its stereoisomers and/or its cosmetically or pharmaceutically acceptable salts, for maintaining the microbial equilibrium of the skin. In one embodiment, the compounds of the invention can be used as a bactericide. A bactericide is a substance or mixture, consisting of, containing or generating one or more active substances, with the intention of destroying, deterring, rendering harmless, preventing the action of, or otherwise exerting a controlling effect on bacteria by any means other than mere physical or mechanical action. In particular, the compound of the invention can be used as a bactericide against *S*. *Aureus* and/or *P. Acnes.*

The compounds of the invention can be used for the cosmetic, non-therapeutic treatment and/or care of the skin, hair, nails and/or mucous membranes wherein the cosmetic, non-therapeutic treatment and/or care includes: the maintenance and/or improvement of the physical barrier function of the skin and the immunological barrier function of the skin; the reduction of the loss of water and electrolytes from the skin; the maintenance and/or improvement of skin hydration, i.e. the maintenance and/or improvement of skin moisturization; the maintenance and/or improvement of skin microrelief; the prevention of adverse changes in skin micro-relief due to impaired barrier function; the maintenance and/or improvement of skin softness and/or the skin's velvety aspect; the maintenance and/or improvement of good skin health; protecting the skin from environmental hazards including chemical irritants, pollutants and pathogens; the maintenance and/or improvement of the innate immune defense mechanism of the skin; and/or the maintenance of the microbial equilibrium of the skin. The compounds of the invention can be used for the cosmetic, non-therapeutic treatment and/or care of the skin, hair, nails and/or mucous membranes wherein the cosmetic, non-therapeutic treatment and/or care includes: the maintenance and/or improvement of the physical barrier function of the skin and the immunological barrier function of the skin; the reduction of the loss of water and electrolytes from the skin; the maintenance and/or improvement of skin hydration, i.e. the maintenance and/or improvement of skin moisturization; the maintenance and/or improvement of skin microrelief; the prevention of adverse changes in skin micro-relief due to impaired barrier function; the maintenance and/or improvement of skin softness and/or the skin's velvety aspect and/or the maintenance of the microbial equilibrium of the skin. The compounds of the invention can be used for the cosmetic, non-therapeutic treatment and/or care of the skin, hair, nails and/or mucous membranes wherein the cosmetic, non-therapeutic treatment and/or care includes: the maintenance and/or improvement of skin hydration, i.e. the maintenance and/or improvement of skin moisturization and/or the maintenance and/or improvement of skin softness and/or the skin's velvety aspect and/or the maintenance of the microbial equilibrium of the skin.

Disclosed is the use of the compounds of the invention in a therapeutic treatment of the skin, hair, mucous membranes and/or nails according to the appended claims. Thus the invention provides a compound of formula (I), its stereoisomers and/or its pharmaceutically acceptable salts or a pharmaceutical composition comprising the compound, its stereoisomers and/or its pharmaceutically acceptable salts for use as a medicament according to the appended claims.

In particular, the invention provides a compound of formula (I), its stereoisomers and/or its pharmaceutically acceptable salts or a pharmaceutical composition comprising the compound, its stereoisomers and/or its pharmaceutically acceptable salts for use in the prevention or treatment of a disease or disorder associated with impaired physical and/or immunological barrier functions of the skin.

Such diseases and disorders include sensitive skin, atopic dermatitis, contact dermatitis, eczema or psoriasis.

Disclosed is a compound of formula (I), its stereoisomers and/or its pharmaceutically acceptable salts for use in maintaining and/or improving the physical barrier function of the skin and the immunological barrier function of the skin.

Disclosed is a compound of formula (I), its stereoisomers and/or its pharmaceutically acceptable salts for use in maintaining and/or improving the physical barrier function of the skin.

Disclosed is a compound of formula (I), its stereoisomers and/or its pharmaceutically acceptable salts for use in maintaining and/or improving the innate immune defense mechanism of the skin.

Disclosed is a compound of formula (I), its stereoisomers and/or its pharmaceutically acceptable salts for use in maintaining the microbial equilibrium of the skin.

Disclosed is a compound of formula (I), its stereoisomers and/or its pharmaceutically acceptable salts for use in inhibiting the colonisation of the skin with S. *Aureus* and/or *P. Acne.*

The invention provides a compound of formula (I), its stereoisomers and/or its pharmaceutically acceptable salts for use in the prevention and/or treatment of atopic dermatitis.

The invention provides a compound of formula (I), its stereoisomers and/or its pharmaceutically acceptable salts for use in the prevention and/or treatment of *acne vulgaris* (also known as acne).

In another aspect, the invention provides a method of treatment and/or care of the skin, hair, nails and/or mucous membranes of a subject comprising administering an effective amount of a compound of formula (I), its stereoisomers and/or its cosmetically or pharmaceutically acceptable salts, to the subject, as defined in the appended claims.

In particular the method of treatment and/or care is a method of treatment and/or care of the skin. The administration of the compound of the invention can be topical or, for example, transdermal. The method is a cosmetic, non-therapeutic method. Also disclosed is where the method is a therapeutic method.

In one embodiment, the invention provides a cosmetic, non-therapeutic method of treatment and/or care of the skin, hair, nails and/or mucous membranes in a subject comprising administering a cosmetically effective amount of a compound of the invention, its stereoisomers and/or its cosmetically acceptable salts or a cosmetic composition comprising a cosmetically effective amount of the compound of the invention, its stereoisomers and/or its cosmetically acceptable salts, to the subject. The compound of the invention may be present in a cosmetic composition, for example a cosmetic composition as described herein. The cosmetic, non-therapeutic method can be for the treatment and/or care of the skin, hair, nails and/or mucous membranes as described above in relation to cosmetic, non-therapeutic method applications (uses) of the compounds of the invention.

Disclosed but not part of the invention is a method of treating or preventing a disease or disorder in a subject comprising administering a therapeutically effective amount of a compound of the invention, its stereoisomers and/or its pharmaceutically acceptable salts or a pharmaceutical composition comprising a therapeutically effective amount of compound, its stereoisomers and/or its pharmaceutically acceptable salts, to the subject. In particular, the invention provides for a method of treating sensitive skin, atopic dermatitis, contact dermatitis, eczema or psoriasis comprising administering a therapeutically effective amount of a compound of the invention, its stereoisomers and/or its pharmaceutically acceptable salts to the skin. The compound of the invention its stereoisomers and/or its pharmaceutically acceptable salts may be present in a pharmaceutical composition, for example the pharmaceutical compositions described herein.

For the above described methods topical or transdermal application can be carried out by iontophoresis, sonophoresis, electroporation, mechanical pressure, osmotic pressure gradient, occlusive cure, microinjections, by needle-free injections by means of pressure, by microelectric patches, face masks or any combination thereof.

For the above described methods the frequency of application or administration can vary greatly, depending on the needs of each subject, with a recommendation of an application from once a month to ten times a day, preferably from once a week to four times a day, more preferably from three times a week to twice a day, even more preferably once a day.

### Cosmetic or pharmaceutical compositions

The compounds of the disclosure can be administered for their application by any means that causes contact between the compounds and the site of action in a subject's body, preferably that of a mammal, preferably a human, and in the form of a composition which contains them.

Disclosed is a cosmetic or pharmaceutical composition comprising a compound according to formula (I), its stereoisomers and/or its cosmetically or pharmaceutically acceptable salts.

Disclosed is a cosmetic or pharmaceutical composition comprising a compound according to formula (I), its stereoisomers and/or its cosmetically or pharmaceutically acceptable salts, together with at least one cosmetically or pharmaceutically acceptable excipient or adjuvant. These compositions can be prepared by conventional means known to persons skilled in the art *["*Harry's Cosmeticology", Seventh edition, (1982), Wilkinson J.B., Moore R.J., ed. Longman House, Essex, GB*j.*

The compounds of this invention have variable solubility in water, according to the nature of their amino acid sequence or any possible modifications in the *N*-terminal and/or C-terminal ends. Therefore, the compounds of this invention can be incorporated into the compositions by aqueous solution, and those which are not soluble in water can be solubilized in cosmetically or pharmaceutically acceptable conventional solvents such as and not restricted to, ethanol, propanol, isopropanol, propylene glycol, glycerin, butylene glycol or polyethylene glycol or any combination thereof.

The compositions contain a cosmetically or pharmaceutically (therapeutically) effective amount of the compound of the invention. The cosmetically or pharmaceutically (therapeutically) effective amount of the compounds of the invention which should be administered, as well as their dosage, will depend on numerous factors, including age, state of the patient, the nature or severity of the condition, disorder or disease to be treated and/or cared for, the route and frequency of administration and of the particular nature of the compounds to be used.

The terms "cosmetically effective amount" and "pharmaceutically effective amount" are understood to mean a non-toxic but sufficient amount of the compound or compounds of the invention to provide the desired effect. The terms "pharmaceutically effective" and "therapeutically effective" are used interchangeably herein. The compounds of the invention are used in the cosmetic or pharmaceutical compositions of this invention at cosmetically or pharmaceutically effective concentrations to achieve the desired effect; for example in amounts with respect to the total weight of the composition of: from 0.00000001 wt % to 20 wt %; from 0.000001 wt % to 15 wt %; from 0.00001 wt % to 10 wt %; from 0.00005 wt % to 5 wt %; from 0.00005 wt % to 1 wt %.; from 0.00005 wt % to 0.1 wt %; from 0.00005 wt % to 0.05 wt %; from 0.00005 wt % to 0.01 wt %; from 0.00005 wt % to 0.005 wt %; from 0.0005 wt % to 0.01 wt %;or from 0.0005 wt % to 0.005 wt %. The compounds of the invention may be at least 0.00000001, 0.000001, 0.00001, 0.00005 or 0.0005 wt % of the total weight of the composition.

The compounds of formula (I), their stereoisomers, mixtures thereof and/or their cosmetic or pharmaceutically acceptable salts, can also be incorporated into cosmetic or pharmaceutical delivery systems and/or sustained release systems.

The term "delivery system" relates to a diluent, adjuvant, excipient or carrier with which the compound of the invention is administered. These cosmetic or pharmaceutical carriers can be liquids, such as water, oils or surfactants, including those of petroleum, animal, plant or synthetic origin, for example and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin and similar. A person skilled in the art knows the diluents, adjuvants or excipients which can be used in the different delivery systems in which the compound of the invention can be administered.

The term "sustained release" is used in a conventional sense relating to a delivery system of a compound which provides the gradual release of this compound during a period of time and preferably, although not necessarily, with relatively constant compound release levels over a period of time.

Examples of delivery or sustained release systems include, without restriction, liposomes, mixed liposomes, oleosomes, niosomes, ethosomes, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules and nanocapsules, as well as in microemulsions and nanoemulsions, which can be added to achieve a greater penetration of the active principle and/or improve its pharmacokinetic and pharmacodynamic properties. Preferred delivery or sustained release systems are liposomes, surfactant-phospholipid mixed micelles, microemulsions, more preferably water-in-oil microemulsions with an internal structure of reverse micelle and nanocapsules containing microemulsions.

Disclosed is a cosmetic or pharmaceutical composition comprising a compound of formula (I) and a cosmetically or pharmaceutically acceptable carrier selected from the group consisting of creams, emulsions, gels, liposomes, nanoparticles and ointments.

The sustained release systems can be prepared by methods known in the prior art, and the compositions which contain them can be administered, for example, by topical or transdermal administration, including adhesive patches, non-adhesive patches, occlusive patches and microelectric patches, or by systemic administration, for example and not restricted to, oral or parenteral route, including nasal, rectal or subcutaneous implantation or injection, or direct implantation or injection into a specific body part, and preferably should release a relatively constant quantity of the compounds of the invention. The amount of compound contained in the sustained release system will depend, for example, on where the composition is to be administered, the kinetics and duration of the release of the compound of the invention, as well as the nature of the condition, disorder and/or disease to be treated and/or cared for.

The compounds of this invention can also be adsorbed on solid organic polymers or solid mineral supports such as and not restricted to, talc, bentonite, silica, starch or maltodextrin among others.

The compositions which contain the compounds of formula (I), their stereoisomers, mixtures thereof and/or their cosmetically or pharmaceutically acceptable salts can also be incorporated into fabrics, non-woven fabrics and medical devices which are in direct contact with the skin, thus releasing the compounds of the invention whether by biodegradation of the binding system to the fabric, non-woven fabric or medical device, or by friction between them and the body, due to bodily moisture, the skin's pH or body temperature. Furthermore, the compounds of the invention can be incorporated into the fabrics and non-woven fabrics used to make garments that are in direct contact with the body.

Examples of fabrics, non-woven fabrics, garments, medical devices and means for immobilizing the compounds to them, among which are the delivery systems and/or the sustained release systems described above, can be found in literature and are known in the prior art *[*Schaab C.K. (1986) HAPPI May 1986; Nelson G., "Application of microencapsulation in textiles", (2002), Int. J. Pharm., 242(1-2), 55-62; *"*Biofunctional Textiles and the Skin" (2006) Curr. Probl. Dermatol. v.33, Hipler U.C. and Elsner P., eds. S. Karger AG, Basel, Switzerl*and;* Malcolm R.K. et al., "Controlled release of a model antibacterial drug from a novel self-lubricating silicone biomaterial". (2004), J. Cont. Release, 97(2), 313-320*].* The preferred fabrics, non-woven fabrics, garments and medical devices are bandages, gauzes, t-shirts, socks, tights, underwear, girdles, gloves, diapers, sanitary napkins, dressings, bedspreads, wipes, adhesive patches, non-adhesive patches, occlusive patches, microelectric patches and/or face masks.

The cosmetic or pharmaceutical compositions which contain the compounds of the invention, their stereoisomers, mixtures thereof and/or their cosmetically or pharmaceutically acceptable salts, can be used in different types of compositions for topical or transdermal application which optionally include cosmetically or pharmaceutically acceptable excipients necessary for formulating the desired administration form.

The compositions for topical or transdermal application can be produced in any solid, liquid or semisolid formulation, such as and not restricted to, creams, multiple emulsions such as and not restricted to, oil and/or silicone in water emulsions, water-in-oil and/or silicone emulsions, water/oil/water or water/silicone/water type emulsions and oil/water/oil or silicone/water/silicone type emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These topical or transdermal application formulations can be incorporated using techniques known by the person skilled in the art into different types of solid accessories for example and not restricted to, bandages, gauzes, t-shirts, socks, tights, underwear, girdles, gloves, diapers, sanitary napkins, dressings, bedspreads, wipes, adhesive patches, non-adhesive patches, occlusive patches, microelectric patches or face masks, or they can be incorporated into different make-up products such as make-up foundation, such as fluid foundations and compact foundations, make-up removal lotions, make-up removal milks, under-eye concealers, eye shadows, lipsticks, lip protectors, lip gloss and powders among others.

The cosmetic or pharmaceutical compositions may include agents which increase the percutaneous absorption of the compounds of the invention, for example and not restricted to, dimethylsulfoxide, dimethylacetamide, dimethylformamide, surfactants, azone (1-dodecylazacycloheptane-2-one), alcohol, urea, ethoxydiglycol, acetone, propylene glycol or polyethylene glycol, among others. Furthermore, the cosmetic or pharmaceutical compositions can be applied to local areas to be treated by means of iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof, to achieve a greater penetration of the peptide of the invention. The application area will be determined by the nature of the condition, disorder and/or disease to be treated and/or cared for.

In one embodiment, the cosmetic or pharmaceutical composition contains a chemical irritant typically contained in cosmetics (including make-up and moisturisers) and hygiene products (including cleansing products). By chemical irritant is meant a chemical which causes irritation to the skin. Thus the cosmetic or pharmaceutical composition can contain one or more chemicals selected from surfactants; oxidants; solvents; preservatives; fatty acids or alcohols; chemical sunscreens; ethoxylated compounds and other formaldehyde releasers; fragrances; and/or chemical peelings. In one embodiment the cosmetic or pharmaceutical composition contains one or more surfactants.

Furthermore, the cosmetic compositions containing the compounds of formula (I), their stereoisomers, mixtures thereof and/or their cosmetically or pharmaceutically acceptable salts can be used in different types of formulations for oral administration, preferably in the form of oral cosmetics or drugs, such as and not restricted to, capsules, including gelatin capsules, soft capsules, hard capsules, tablets, including sugar coated tablets, tablets, pills, powders, granules, chewing gum, solutions, suspensions, emulsions, syrups, elixirs, polysaccharide films, jellies or gelatins, and any other form known by the person skilled in the art. In a particular embodiment, the compounds of the invention can be incorporated into any form of functional food or fortified food, such as and not restricted to, dietary bars or compact or non-compact powders. These powders can be dissolved in water, soda, dairy products, soy derivatives or can be incorporated into dietary bars. The compounds of this invention can be formulated with common excipients and adjuvants for oral compositions or food supplements, for example and not restricted to, fat components, aqueous components, humectants, preservatives, texturizing agents, flavors, aromas, antioxidants and colorants common in the food industry.

Cosmetic or pharmaceutical compositions containing the compounds of formula (I), their stereoisomers, mixtures thereof and/or their cosmetically or pharmaceutically acceptable salts can also be administered, as well as by topical or transdermal route, by any other appropriate route, such as oral or parenteral route, for which they will include the pharmaceutically acceptable excipients necessary for the formulation of the desired administration form. In the context of this invention, the term "parenteral" includes nasal, auricular, ophthalmic, rectal, urethral, vaginal, subcutaneous, intradermal route, intravascular injections, such as intravenous, intramuscular, intraocular, intravitreous, intracorneal, intraspinal, intramedullary, intracranial, intracervical, intracerebral, intrameningeal, intraarticular, intrahepatic, intrathoracic, intratracheal, intrathecal and intraperitoneal, and any another similar injection or infusion technique. A person skilled in the art knows the different means by which the cosmetic or pharmaceutical compositions which contain the compounds of the invention can be administered.

Among the cosmetically or pharmaceutically acceptable adjuvants contained in the cosmetic or pharmaceutical compositions described herein are additional ingredients commonly used in cosmetic or pharmaceutical compositions, for example and not restricted to other agents improving or restoring the skin barrier function, nocturnin modulating agents, agents that boost mitochondrial metabolism, agents that enhance adiponectin release, agents that boost intercellular communication, agents that increase connexins in skin cells, agents that promote self-renewal of the skin, agents that prevent hypertrophic scarring of the skin, DNA protecting agents, DNA repair agents, stem cell protecting agents, agents reactivating the pool of epidermal stem cells, agents inhibiting neuronal exocytosis, anticholinergic agents, agents inhibiting muscular contraction, antiaging agents, anti-wrinkle agents, antiperspirant agents, anti-inflammatory and/or analgesic agents, anti-itching agents, calming agents, anesthetic agents, inhibitors of acetylcholine-receptor aggregation, inhibitors of acetylcholinesterase, skin relaxant agents, melanin synthesis stimulating or inhibiting agents, whitening or depigmenting agents, propigmenting agents, self-tanning agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, detoxifying agents, antihistamine agents, antiviral agents, antiparasitic agents, emulsifiers, emollients, organic solvents, liquid propellants, skin conditioners, humectants, substances which retain moisture, alpha hydroxy acids, beta hydroxy acids, moisturizers, hydrolytic epidermal enzymes, vitamins, amino acids, proteins, pigments or colorants, dyes, biopolymers, gelling polymers, thickeners, surfactants, softening agents, emulsifiers, binding agents, preservatives, agents able to reduce or treat the bags under the eyes, exfoliating agents, keratolytic agents, desquamating agents, antimicrobial agents, antifungal agents, fungistatic agents, bactericidal agents, bacteriostatic agents, agents stimulating the synthesis of dermal or epidermal macromolecules and/or capable of inhibiting or preventing their degradation, collagen synthesis-stimulation agents, elastin synthesis-stimulation agents, decorin synthesis-stimulation agents, laminin synthesis-stimulation agents, defensin synthesis-stimulating agents, chaperone synthesis-stimulating agents, cAMP synthesis-stimulating agents, AQP-3 modulating agents, aquaporin synthesis-stimulating agents, proteins of the aquaporin family, hyaluronic acid synthesis-stimulating agents, glycosaminoglycan synthesis-stimulating agents, fibronectin synthesis-stimulating agents, sirtuin synthesis-stimulating agents, sirtuin-activating agents, heat shock proteins, heat shock protein synthesis-stimulating agents, agents stimulating the synthesis of lipids and components of the stratum corneum, ceramides, fatty acids, agents that inhibit collagen degradation, agents that inhibit matrix metalloproteinase, agents that inhibit elastin degradation, agents that inhibit serine proteases such as kallikreins, elastase or cathepsin, agents stimulating fibroblast proliferation, agents stimulating keratinocyte proliferation, agents stimulating adipocyte proliferation, agents stimulating melanocyte proliferation, agents stimulating keratinocyte differentiation, agents stimulating or delaying adipocyte differentiation, antihyperkeratosis agents, comedolytic agents, anti-psoriatic agents, stabilizers, agents for the treatment and/or care of sensitive skin, firming agents, anti-stretch mark agents, binding agents, agents regulating sebum production, lipolytic agents or agents stimulating lipolysis, adipogenic agents, agents modulating PGC-1α expression, agents modulating the activity of PPARγ, agents which increase or reduce the triglyceride content of adipocytes, anti-cellulite agents, agents which inhibit PAR-2 activity, agents stimulating healing, coadjuvant healing agents, agents stimulating reepithelialization, coadjuvant reepithelialization agents, cytokine growth factors, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, agents delaying hair loss, preservatives, perfumes, cosmetic and/or absorbent and/or body odor-masking deodorants, chelating agents, plant extracts, essential oils, marine extracts, agents obtained from a biotechnological process, mineral salts, cell extracts, sunscreens and organic or mineral photoprotective agents active against ultraviolet A and/or B rays and/or infrared A rays, or mixtures thereof, provided they are physically and chemically compatible with the rest of components of the composition and in particular with the compounds of the invention. Furthermore, the nature of these additional ingredients should not unacceptably alter the benefits of the compounds of this invention. The nature of these additional ingredients can be synthetic or natural, such as plant extracts, or come from a biotechnological process or from a combination of a synthetic procedure and biotechnological process. Additional examples can be found in CTFA International Cosmetic Ingredient Dictionary & Handbook, 12th Edition (2008*).* In the context of this invention, biotechnological process is understood to be any process that produces the active ingredient, or part of it, in an organism, or in part of it.

Disclosed is a cosmetic or pharmaceutical composition comprising a compound of formula (I) and a pharmaceutically or cosmetically effective amount of one or more adjuvants selected from the group consisting of: humectants/emollients/moisturizers; wound healing/reepithelization agents; anti-wrinkle/aging actives; whitening/depigmenting agents; sensitive skin treatment agents; anti-itching agents; anti-inflammation agents; and/or actives for the treatment of psoriasis, dermatitis or eczema.

The cosmetic or pharmaceutical composition can comprise a cosmetically or pharmaceutically effective quantity of at least one compound, oil or wax selected from the group of the cosmetic or pharmaceutical adjuvants formed by humectants, substances which retain moisture, moisturizers and emollients, such as and not restricted to, polyols and polyethers such as glycerin, ethylhexylglycerin, caprylyl glycol, pentylene glycol, butylene glycol, propylene glycol and its derivatives, triethylene glycol, polyethylene glycol, Glycereth-26, Sorbeth-30; panthenol; pyroglutamic acid and its salts or derivatives; amino acids, such as serine, proline, alanine, glutamate or arginine; ectoin and its derivatives; N-(2-hydroxyethyl)acetamide; pyrrolidone carboxylic acid (PCA); *N*-lauroyl-pyrrolidone carboxylic acid; *N*-lauroyl-L-lysine; *N*-alpha-benzoyl-L-arginine; urea; creatine; alpha- and beta-hydroxy acids such as lactic acid, glycolic acid, malic acid, citric acid, tartaric acid or salicylic acid, and its salts; polyglyceryl acrilate; sugars and polysaccharides, such as glucose, isomerate saccharide, sorbitol, pentaerythritol, inositol, xylitol, sorbitol, trehalose and its derivatives, sodium glucuronate, carrageenans *(Chondrus crispus)* or chitosan; glycosaminoglycans such as hyaluronic acid and its derivatives; aloe vera in any of its forms; honey; soluble collagen; lecithin and phosphatidylcholine; ceramides; cholesterol and its esters; tocopherol and its esters, such as tocopheryl acetate or tocopheryl linoleate; long chain alcohols such as cetearyl alcohol, stearic alcohol, cetyl alcohol, oleyl alcohol, isocetyl alcohol or octadecan-2-ol; long chain alcohol esters such as lauryl lactate, myristyl acetate or C₁₂-C₁₅ alkyl benzoates; fatty acids such as stearic acid, isostearic acid or palmytic acid; polyunsaturated fatty acids (PUFAs); sorbitans such as sorbitan distearate; glycerides such as glyceryl monoricinoleate, glyceryl monostearate, glyceryl stearate citrate or caprylic and capric acid triglyceride; saccharose esters such as saccharose palmitate or saccharose oleate; butylene glycol esters, such as dicaprylate and dicaprate; fatty acid esters such as isopropyl isostearate, isobutyl palmitate, isocetyl stearate, isopropyl laurate, hexyl laurate, decyl oleate, cetyl palmitate, di-n-butyl sebacate, isopropyl myristate, isopropyl palmitate, isopropyl stearate, butyl stearate, butyl myristate, isopropyl linoleate, 2-ethylhexyl palmitate, 2-ethylhexyl cocoate, decyl oleate, myristyl myristate; squalene; mink oil; lanolin and its derivatives; acetylated lanolin alcohols; silicone derivatives such as cyclomethicone, dimethicone or dimethylpolysiloxane; Antarcticine^{®} [INCI: Pseudoalteromonas Ferment Extract], Xpertmoist^{®} [INCI: Glycerin, Pseudoalteromonas Ferment Extract, Xanthan Gum, Proline, Alanine, Serine, Ethylhexylglycerin, Caprylyl Glycol], Bodyfensine^{®} [INCI: Acetyl Dipeptide-3 Aminohexanoate], Hyadisine^{®} [INCI: Pseudoalteromonas Ferment Extract] or Diffuporine^{®} [INCI: Acetyl Hexapeptide-37] marketed by Lipotec/Lubrizol; Amiporine^{®} ER [INCI: Glycerin, Punica Granatum Fruit Extract], HPS3^{®} [INCI: Padina Pavonica Thallus Extract] or Phytoamine Biocomplexe [INCI: Symphytum Officinale Extract, Plantago Ovata Seed Extract, Hydrolyzed Wheat Protein, Glutamine, Proline, Leucine, Serine] marketed by Alban Muller; Dermaclarine^{™} [INCI: Hydrolyzed Egg Protein, Protease] marketed by Aqua Bio Technology; Bio-Plex NMF [INCI: Sodium PCA, Lactic Acid, Sodium Lactate, Urea, Collagen Amino Acids], DermaFlux^{®} [INCI: Urea, Yeast Amino Acids, Trehalose, Inositol, Taurine, Betaine] or ReGeniStem^{™} Red Rice [INCI: Ozonized Oryza Sativa (Rice) Callus Culture Extract] marketed by Arch/Lonza; Hydralphatine^{™} Asia [INCI: Hydrogenated Starch Hydrolysate, Panthenol, Bambusa Vulgaris Shoot Extract, Nelumbo Nucifera Flower Extract, Nymphaea Alba Root Extract], Hydriame^{®} [INCI: Glycosaminoglycans, Sclerotium Gum], Hydraporine^{™} [INCI: Betaine, Hydrogenated Lecithin, Honey, Pectin] or Exo-H^{™} [INCI: Alteromonas Ferment Extract] marketed by Lucas Meyer Cosmetics/Unipex; PatcH₂O^{™} [INCI: Trehalose, Urea, Serine, Glyceryl Polyacrylate, Algin, Sodium Hyaluronate, Pullulan] marketed by BASF; Cellike [INCI: Caprylic/Capric Triglyceride, Hydrogenated Phospatidylcholine, Butyrospermum Parkii (Shea) Butter, Phytosterols, Glyceryl Caprylate, Ceramide NP] marketed by BioSpectrum; Aquasense^{®} [INCI: Piptadenia Colubrina Peel Extract] marketed by Chemyunion; DayMoist^{™} CLR [INCI: Hydrolyzed Corn Starch, Beta Vulgaris (Beet) Root Extract] marketed by CLR; Hydranov [INCI: Sodium Carrageenan, Maris Sal] or Hydrasalinol [INCI: Salicornia Herbacea Extract, Caprylic/Capric Triglyceride] marketed by Codif; Marine Filling Spheres^{™} [INCI: Pentaerythrityl Tetraisostearate, Silica Dimethyl Silylate, Sodium Chondroitin Sulfate, Atelocollagen] marketed by Coletica/Engelhard/BASF; HyaCare^{®} [INCI: Sodium Hyaluronate] or Skinmimics^{®} [INCI: Ceteareth-25, Cetyl Alcohol, Behenic Acid, Cholesterol, Ceramide NP, Ceramide NS, Ceramide EOS, Ceramide EOP, Ceramide AP, Caprooyl Phytosphingosine, Caprooyl Sphingosine] marketed by Evonik; DS-Sphyngomielin M [INCI: Sphingolipids] marketed by Doosan; Syn-Up^{™} [INCI: Benzylsulfonyl D-Seryl Homophenylalanine Amidinobenzamide Acetate] marketed by DSM; Aqualicia^{®} [INCI: Hydrolyzed Acacia Macrostachya Seed Extract] or Soline^{®} [INCI: Helianthus Annuus (Sunflower) Seed Oil Unsaponifiables] marketed by Laboratoires Expanscience; Arct'Alg^{®} [INCI: Chondrus Crispus Extract] or Glistin^{®} [INCI: Glutamylamidoethyl Indole] marketed by Exsymol; Bonicel^{™} [INCI: Bacillus Ferment] marketed by Ganeden Biotech; Gatuline^{®} Renew [INCI: Cryptomeria Japonica Bud Extract] marketed by Gattefossé; Biotilys^{®} [INCI: Lactobacillus Ferment Lysate] marketed by Greentech; Aqua Shuttle [INCI: Sorbitol, Laminaria Digitata Extract, Diatomaceous Earth] marketed by Infinitec; Aquarize^{™} IS [INCI: Hydrolyzed Rice Extract] or Aqua-Osmoline^{™} [INCI: Ceratonia Siliqua (Carob) Seed Extract] marketed by Vincience/ISP/Ashland; Aqu'activ^{™} [INCI: Behenyl Alcohol, Glyceryl Oleate, Cocamide MIPA], Hibiscin^{®} HP [INCI: Hibiscus Esculentus Seed Extract], Hyalurosmooth^{®} [INCI: Cassia Angustifolia Seed Polysaccharide], Indinyl^{®} CA [INCI: Cassia Angustifolia Seed Polysaccharide], Irwinol^{®} [INCI: Octyldodecanol, Irvingia Gabonensis Kernel Butter, Hydrogenated Coco-Glycerides], Lipodermol^{®} [INCI: Octyldodecanol, Arachidyl Propionate, Tocopheryl Acetate, Retinyl Palmitate, Ethyl Linoleate, Ethyl Linolenate] or Seanamin^{®} SU [INCI: Sorbitol, Algae Extract, Chrondrus Crispus (Carrageenan), Fucus Vesiculosus Extract, Algin] marketed by L. Serobiologiques/Cognis/BASF; Lipocare HA/EC [INCI: Sodium Hyaluronate, Echinacin] marketed by Lipochemicals; Hydro-Gain^{™} [INCI: Canola Oil, Hydrogenated Lecithin, Opuntia Ficus-Indica Seed Oil, Betula Alba Bark Extract] marketed by Lipoid Kosmetik; RonaCare^{®} RenouMer [INCI: Algae Extract] marketed by Merck; AquaCacteen [INCI: Opuntia Ficus-Indica Stem Extract], Snow Algae Powder [INCI: Coenochloris Signiensis Extract] or Trimoist KMF [INCI: Sodium Stearoyl Lactylate, Cetyl Alcohol, Olus Oil/Vegetable Oil, Tocopheryl Acetate, Glycine Soja (Soybean) Sterols, Sodium Carboxymethyl Betaglucan, Sodium Lactate, Carnosine, Lactic Acid] marketed by Mibelle; Alpaflor^{®} Nectapure [INCI: Thymus Vulgaris (Thyme) Flower/Leaf Extract, Buddleja Davidii Extract], Hyasol BT [INCI: Sodium Hyaluronate], Pentavitin^{®} [INCI: Saccharide Isomerate] or Phytaluronate^{®} [INCI: Ceratonia Siliqua (Carob) Gum] marketed by Pentapharm/DSM; Aquaxtrem^{™} [INCI: Rheum Rhaponticum Root Extract] or Hydromanil [INCI: Hydrolyzed Caesalpinia Spinosa Gum, Caesalpinia Spinosa Gum] marketed by Provital; Aquarich^{®} [INCI: Avena Strigosa Seed Extract], CellActive^{®}-Hydro [INCI: Pyrus Malus (Apple) Fruit Extract, Pectin, Chlorella Vulgaris/Lupinus Albus Protein Ferment], CellActive^{®}-Men [INCI: Taurine, Chlorella Vulgaris/Lupinus Albus Protein Ferment, Acanthopanax Senticosus (Eleuthero) Root Extract], Hydractin^{®}-LMF [INCI: Polypodium Vulgare Rhizome Extract, Cetraria Islandica (Iceland Moos) Thallus Extract, Sphagnum Magellanicum Extract], Myramaze^{®} [INCI: Myrothamnus Flabellifolia Extract, Ascorbic Acid] or Reforcyl^{®} [INCI: Glutamine, Decyl Glucoside, Phenethyl Alcohol, Cistus Incanus Flower/Leaf/Stem Extract, Gynostemma Pentaphyllum Leaf/Stem Extract] marketed by Rahn; Aqualance^{™} [INCI: Erythritol, Homarine HCI], Hydraprotectol^{™} [INCI: Glyceryl Polymethacrylate, Aleuritic Acid, Yeast Extract (Faex), Glycoprotein], Moist 24^{™} [INCI: Imperata Cylindrica Root Extract], Optim Hyal^{™} [INCI: Hydrolyzed Yeast Extract, Cetyl Hydroxyethylcellulose, Polyglucuronic Acid], Osmocide^{®} 4 [INCI: Glycerin, Acrylates/C10-30 Alkyl Acrylate Crosspolymer], Renovage^{™} [INCI: Caprylic/Capric Triglyceride, Teprenone], Revidrate^{™} [INCI: Ethylhexyl Palmitate, Sorbitan Oleate, Sorbitan Laureate, Myristyl Malate Phosphonic Acid], Subliskin^{™} [INCI: Sinorhizobium Meliloti Ferment Filtrate, Cetyl Hydroxyethylcellulose] or Venuceane^{™} [INCI: Thermus Thermophillus Ferment] marketed by Sederma/Croda; Aquaxyl^{™} [INCI: Xylitylglucoside Anhydroxylitol, Xylitol] or Sepicalm^{™} S [INCI: Sodium Cocoyl Amino Acids, Sarcosine, Potassium Aspartate, Magnesium Aspartate] marketed by Seppic; Cohesium^{®} [INCI: Ophiopogon Japonicus Root Extract], Nerenyl^{®} [INCI: Saccharide Hydrolysate] or Pro-Lipiskin^{®} [INCI: Pichia Anomala Extract] marketed by Silab; Hydreïs [INCI: Hydrolyzed Beta-Glucan], Hydrintense [INCI: Porphyridium Cruentum Extract] or RenovHyal [INCI: Sodium Hyaluronate] marketed by Soliance; SymLift^{™} [INCI: Trehalose, Beta-Glucan, Hordeum Vulgare Seed Extract, Sodium Hyaluronate] marketed by Symrise; petrolatum; mineral oil; mineral and synthetic waxes; beeswax (cera alba); paraffin; or waxes and oils of plant origin such as candelilla wax *(Euphorbia cerifera*), carnauba wax *(Copernicia cerifera*), shea butter *(Butirospermum parkii*), cocoa butter *(Theobroma cacao),* castor oil *(Ricinus communis),* sunflower oil *(Helianthus annuus),* olive oil *(Olea europaea),* coconut oil *(Cocos nucifera),* palm oil *(Elaeis guineensis),* wheat germ oil *(Triticum vulgare),* sweet almond oil *(Prunus amygdalus dulces),* musk rose seed oil *(Rosa moschata),* wild soybean oil *(Glycine soja),* grape seed oil *(Vitis vinifera),* calendula oil *(Calendula officinalis),* jojoba oil *(Simmonsis chinensis),* mango oil *(Mangifera indica),* avocado oil *(Persea gratissima),* among others, and/or mixtures thereof.

The cosmetic or pharmaceutical composition can comprise a cosmetically or pharmaceutically effective quantity of at least one agent stimulating wound healing, coadjuvant wound healing agent, agent stimulating reepithelization and/or coadjuvant reepithelialization agent selected, for example, and not restricted to, from the group formed by extracts of *Aristoloquia clematis, Centella asiatica, Rosa moschata, Echinacea angustifolia, Symphytum officinale, Equisetum arvense, Hypericum perforatum, Mimosa tenuiflora, Persea gratísima, Prunus africanum, Tormentilla erectea, Aloe vera,* Polyplant^{®} Epithelizing [INCI: Calendula Officinalis, Hypericum Perforatum, Chamomilla Recutita, Rosmarinus Officinalis] marketed by Provital; Cytokinol^{®} LS 9028 [INCI: Hydrolyzed Casein, Hydrolyzed Yeast Protein, Lysine HCl] marketed by Laboratories Serobiologiques/Cognis; Deliner^{®} [INCI: Zea May (Corn) Kernel Extract] marketed by Coletica/Engelhard/BASF; Antarcticine^{®} [INCI: Pseudoalteromonas Ferment Extract], Decorinyl^{®} [INCI: Tripeptide-10 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Bodyfensine^{®} [INCI: Acetyl Dipeptide-3 Aminohexanoate], Delisens^{™} [INCI: Acetyl Hexapeptide-49] or Diffuporine^{®} [INCI: Acetyl Hexapeptide-37] marketed by Lipotec/Lubrizol; Alpaflor^{®} Imperatoria AO [INCI: Peucedanum Ostruthium Leaf Extract] or OXY 229-BT [INCI: Saccharomyces Lysate] marketed by Pentapharm/DSM; CellActive^{®}-Men [INCI: Chlorella Vulgaris/Lupinus Albus Protein Ferment, Taurine, Acanthopanax Senticosus (Eleuthero) Root Extract] marketed by Rahn; Drieline^{™} [INCI: Hydrogenated Starch Hydrolysate, Yeast Extract] marketed by Lucas Meyer Cosmetics/Unipex; NAB^{®} Rhodiola Extract [INCI: Rhodiola Rosea Root Extract] marketed by Arch/Lonza; Neoskin^{™} [INCI: Mimosa Tenuiflora Bark Extract] marketed by Gattefossé; or SymPeptide^{™} 222 [INCI: Myristoyl Pentapeptide-8], SymPeptide^{™} 225 [INCI: Myristoyl Pentapeptide-11] or SymPeptide^{™} 230 [INCI: Myristoyl Hexapeptide-4] marketed by Symrise, allantoin, cadherins, integrins, selectins, hyaluronic acid receptors, immunoglobulins, fibroblast growth factors, connective tissue growth factors, platelet-derived growth factors, vascular endothelial growth factors, epidermal growth factors, insulin-like growth factors, keratinocyte growth factor, colony-stimulating factor, transforming growth factor beta, tumor necrosis factor-alpha, interferons, interleukins, matrix metalloproteinases, receptor protein tyrosine phosphatases among others, and/or mixtures thereof.

The cosmetic or pharmaceutical composition can comprise a cosmetically or pharmaceutically effective quantity of at least one anti-wrinkle and/or antiaging agent selected, for example and not restricted to, from the group formed by the extracts or hydrolyzed extracts of *Vitis vinifera, Rosa canina, Curcuma longa, Theobroma cacao, Ginkgo biloba, Leontopodium alpinum* or *Dunaliella salina* among others, Matrixyl^{®} [INCI: Palmitoyl Pentapeptide-4], Matrixyl^{®} 3000^{®} [INCI: Palmitoyl Tetrapeptide-7, Palmitoyl Oligopeptide], Matrixyl^{®} Synthe'6^{™} [INCI: Hydroxypropyl Cyclodextrin, Palmitoyl Tripeptide-38], Essenskin^{™} [INCI: Calcium Hydroxymethionine], Renovage [INCI: Teprenone], Resistem^{™} [INCI: Globularia Cordifolia Ferment], Dermaxyl^{®} [INCI: Palmitoyl Oligopeptide], Beautifeye^{™} [INCI: Albizia Julibrissin Bark Extract, Darutoside], Meiritage^{™} [INCI: Astragalus Membranaceus Root Extract, Atractylodes Macrocephala Root Extract, Bupleurum Falcatum Root Extract], Senestem^{™} [INCI: Plantago Lanceolata Leaf Extract], Venuceane^{™} [INCI: ThermusThermophillus Ferment] or Majestem^{™} [INCI: Leontopodium Alpinum Callus Culture Extract] marketed by Sederma/Croda; Vialox^{®} [INCI: Pentapeptide-3], Syn^{®} Ake^{®} [INCI: Dipeptide Diaminobutyroyl Benzylamide Diacetate], Syn^{®}-Coll [INCI: Palmitoyl Tripeptide-5], Phytaluronate [INCI: Locust Bean (Ceratonia Siliqua) Gum], Preregen^{®} [INCI: Glycine Soja (Soybean) Protein, Oxido Reductases], Syn^{®}-TC [INCI: Tetradecyl Aminobutyroylvalylaminobutyric Urea Trifluoroacetate, Palmitoyl Tripeptide-5, Palmitoyl Dipeptide-5 Diaminobutyroyl Hydroxythreonine], Syn-Star^{™} [INCI: Dipeptide Diaminobutyroyl Benzylamide Diacetate] or Regu^{®}-Scence [INCI: Asparagus Officinalis Stem Extract, Gluconolactone, Calcium Gluconate] marketed by Pentapharm/DSM; Myoxinol^{™} [INCI: Hydrolyzed Hibiscus Esculentus Extract], Syniorage^{™} [INCI: Acetyl Tetrapeptide-11], Dermican^{™} [INCI: Acetyl Tetrapeptide-9], DN AGE^{™} LS [INCI: Cassia Alata Leaf Extract], EquiStat [INCI: Pyrus Malus Fruit Extract, Glycine Soja Seed Extract], Juvenesce [INCI: Ethoxydiglycol, Caprylic Triglyceride, Retinol, UrsolicAcid, Phytonadione, Ilomastat], Shadownyl^{™} [INCI: Algae Extract], Epigenist^{™} [INCI: Voandzeia Subterranea Seed Extract, Maltodextrin], LOX-AGE^{™} [INCI: Cichorium Intybus (Chicory) Leaf Extract] or Perlaura^{™} [INCI: Polygonum Bistorta Root Extract] marketed by BASF; Algisum C^{®} [INCI: Methylsilanol Mannuronate], Hydroxyprolisilane CN^{®} [INCI: Methylsilanol Hydroxyproline Aspartate] or Exage [INCI: Imidazolylethyl Diaminopropanamide] marketed by Exsymol; Argireline^{®} [INCI: Acetyl Hexapeptide-8], SNAP-7^{™} [INCI: Acetyl Heptapeptide-4], SNAP-8^{™} [INCI: Acetyl Octapeptide-3], Leuphasyl^{®} [INCI: Pentapeptide-18], Inyline^{®} [INCI: Acetyl Hexapeptide-30], Aldenine^{®} [INCI: Hydrolyzed Wheat Protein, Hydrolyzed Soy Protein, Tripeptide-1], Preventhelia^{®} [INCI: Diaminopropionoyl Tripeptide-33], Decorinyl^{®} [INCI: Tripeptide-10 Citrulline], Decorinol^{®} [INCI: Tripeptide-9 Citrulline], Trylagen^{®} [INCI: Pseudoalteromonas Ferment Extract, Hydrolyzed Wheat Protein, Hydrolyzed Soy Protein, Tripeptide-10 Citrulline, Tripeptide-1], Eyeseryl^{®} [INCI: Acetyl Tetrapeptide-5], Peptide AC29 [INCI: Acetyl Tripeptide-30 Citrulline], Relistase^{®} [INCI: Acetylarginyltriptophyl Diphenylglycine], Thermostressine^{®} [INCI: Acetyl Tetrapeptide-22], Lipochroman^{®} [INCI: Dimethylmethoxy Chromanol], Chromabright^{®} [INCI: Dimethylmethoxy Chromanyl Palmitate], Antarcticine^{®} [INCI: Pseudoalteromonas Ferment Extract], dGlyage^{®} [INCI: Lysine HCl, Lecithin, Tripeptide-9 Citrulline], Vilastene^{™} [INCI: Lysine HCl, Lecithin, Tripeptide-10 Citrulline], Hyadisine^{®} [INCI: Pseudoalteromonas Ferment Extract], Hyanify^{™} [INCI: Saccharide Isomerate], Diffuporine^{®} [INCI: Acetyl Hexapeptide-37], Silusyne^{®} [INCI: Soybean (Glycine Soja) Oil, Sorbitan Sesquioleate, Isohexadecane, Sodium Hyaluronate, Lauryldimonium Hydroxypropyl Hydrolized Soy Protein, Acetyl Hexapeptide-39], Adifyline^{®} [INCI: Acetyl Hexapeptide-38], Uplevity^{™} [INCI: Acetyl Tetrapeptide-2], Juveleven^{™} [INCI: Acetyl Hexapeptide-51 Amide], Argirelox^{™} [INCI: Acetyl Hexapeptide-8, Pentapeptide-18], Seacode^{™} [INCI: Pseudoalteromonas Ferment Extract], Eyedeline^{™} [INCI: Plankton Extract] Cellynkage^{™} [INCI: Saccharide Isomerate] or Reproage^{™} [INCI: Acetyl Hexapeptide-8] marketed by Lipotec/Lubrizol; Kollaren^{®} [INCI: Tripeptide-1, Dextran] marketed by Institut Europeen de Biologie Cellulaire; Collaxyl^{®} IS [INCI: Hexapeptide-9], Laminixyl IS^{™} [INCI: Heptapeptide], Orsirtine^{™} GL [INCI: Oryza Sativa (Rice) Extract], D'Orientine^{™} IS [INCI: Phoenix Dactylifera (Date) Seed Extract], Phytoquintescine^{™} [INCI: Einkorn (Triticum Monococcum) Extract], Quintescine^{™} IS [INCI: Dipeptide-4], Peptide Q10^{™} [INCI: Pentapeptide-34Trifluoroacetate] or Telosense^{™} [INCI: Hydrolyzed Yeast Protein, Hydrolyzed Soy Protein] marketed by Vincience/ISP/Ashland; BONT-L-Peptide [INCI: Palmitoyl Hexapeptide-19] marketed by InfinitecActivos; Deepaline^{™} PVB [INCI: Palmitoyl hydrolyzed Wheat Protein], Sepilift^{®} DPHP [INCI: Dipalmitoyl Hydroxyproline] or Ephemer^{™} [INCI: Caprylic/Capric Triglyceride, Undaria Pinnatifida Extract] marketed by Seppic; Gatuline^{®} Expression [INCI: Acmella Oleracea Extract], Gatuline^{®} In-Tense [INCI: Spilanthes Acmella Flower Extract], Gatuline^{®} Age Defense 2 [INCI: Juglans Regia (Walnut) Seed Extract] or Gatuline^{®} Renew [INCI: Cryptomeria Japonica Bud Extract] marketed by Gattefossé; Thalassine^{™} [INCI: Algae Extract] marketed by Biotechmarine; ChroNOline^{™} [INCI: Caprooyl Tetrapeptide-3] or Thymulen-4 [INCI: Acetyl Tetrapeptide-2] marketed by Atrium/Unipex Innovations; Ameliox [INCI: Carnosine, Tocopherol, Silybum Marianum Fruit Extract], PhytoCellTec^{™} Malus Domestica [INCI: Malus Domestica Fruit Cell Culture], PhytoCellTec^{™} Symphytum [INCI: Isomalt, Symphytum Officinale Root Cell Culture, Lecithin], PhytoCellTec^{™} nunatak^{®} [INCI: Saponaria Pumila Callus Culture Extract, Isomalt, Lecithin], AnaGain^{™} [INCI: Pisum Sativum (Pea) Sprout Extract], Dermcom [INCI: Acacia Senegal Gum, Crocus Chrysanthus Bulb Extract], Snow Algae Powder [INCI: Chlamydocapsa Extract, Maltodextrin, Lecithin], RoyalEpigen P5 [INCI: Pentapeptide-48, Hydrogenated Lecithin, Butyrospermum Parkii (Shea) Butter] or Vin-upLift [INCI: Wine, Caesalpinia Spinosa Gum] marketed by Mibelle Biochemistry; Bioxilift [INCI: Pimpinella Anisum Extract], SMS Anti-Wrinkle^{®} [INCI: Annona Squamosa Seed Extract] Vitagenyl^{®} [INCI: Prunus Persica (Peach) Leaf Extract], Epigenomyl^{®} [INCI: Calendula Officinalis Flower Extract], Eternaline^{®} [INCI: Helichrysum Stoechas Extract] or Filmexel^{®} [INCI: Kappaphycus Alvarezii Extract, Caesalpinia Spinosa Fruit Extract] marketed by Silab; Citrustem^{™} [INCI: Xanthan Gum, Gluconolactone, Calcium Gluconate], Melavoid^{™} [INCI: Boerhavia Diffusa Root Extract], Darkout^{™} [INCI: Hypoxis Rooperi Rhizome Extract, Caesalpinia Spinosa Gum], Linefill^{™} [INCI: Caprylic/Capric Glycerides, Dimethyl Isosorbide, Sesamum Indicum (Sesame) Seed Extract] or Lingostem^{™} [INCI: Vaccinium Vitis-Idaea Fruit Extract] marketed by Provital; Novhyal^{®} Biotech G [INCI: Disodium Acetyl Glucosamine Phosphate], Rubixyl^{®} [INCI: Hexapeptide-47] or Tightenyl^{™} [INCI: Disodium Acetyl Glucosamine Phosphate, Sodium Glucuronate] marketed by Induchem/Givaudan; Sphingokine^{®} NP [INCI: Caprooyl Phytosphingosine] marketed by Evonik; Effipulp^{®} [INCI: Hydrolyzed Avocado Protein, Maltodextrin] or Neurovity^{®} [INCI: Vitex Negundo Extract] marketed by Laboratoires Expanscience; Progeline^{™} [INCI: Dextran, Trifluoroacetyl Tripeptide-2], Adipofill'in^{™} [INCI: Ornithine, Phospholipids, Glycolipids], Elix-IR^{™} [INCI: Polygonum Aviculare Extract] or SWT-7^{™} [INCI: Swertia Chirata Extract] marketed by Lucas Meyer Cosmetics/Unipex; Amiperfect [INCI: Gaultheria Procumbens (Wintergreen) Leaf Extract] or Repulpami ER [INCI: Adansonia Digitata Pulp Extract, Hibiscus Sabdariffa Flower Extract] marketed by Alban Muller; Liftonin^{®}-Xpress [INCI: Hydroxypropyl Methylcellulose, Pullulan, Porphyridium Cruentum Extract] or Littonin^{®}-Xpert [INCI: Bulbine Frutescens Leaf Juice] marketed by Rahn; Actiporine 8G [Jania Rubens Extract], EPS Seafill [INCI: Plankton Extract] or Neuroguard [INCI: Hydrolyzed Algin] marketed by Codif; Celloxyl^{®} [INCI: Uapaca Bojeri Leaf Extract] or Resistress^{®} [INCI: Sophora Japonica Flower Extract] marketed by Solabia; ProSynergen^{™} DF [INCI: Lactobacillus/Ulkenia Amoeboidea Ferment Extract Filtrate] marketed by Lonza; SymVital^{®} AgeRepair [INCI: Zingiber Officinale (Ginger) Root Extract] marketed by Symrise; Cernilys^{®} [INCI: Cedrus Atlantica Bark Extract] or Reverskin^{®} [INCI: Phytosterols] marketed by Greentech; Clotholine^{®} [INCI: Coumaroyl Methoxytryptamine, PEG-8 Caprylic/Capric Glycerides] marketed by Sollice Biotech; Fision^{™} WrinkleFix [INCI: Hydrolyzed Rice Protein, Hydrolyzed Pea Protein, Glycine, Proline, Hydrolyzed Sodium Hyaluronate] marketed by TRI-K Industries; antagonists of the Ca²⁺ channel such as and not restricted to, alverine, manganese or magnesium salts, certain secondary or tertiary amines, retinol and its derivatives, idebenone and its derivatives, Coenzyme Q10 and its derivatives, boswellic acid and its derivatives, GHK and its derivatives and/or salts, carnosine and its derivatives, DNA repair enzymes such as and not restricted to, photolyase or T4 endonuclease V, or chloride channel agonists among others, and/or mixtures thereof.

The cosmetic or pharmaceutical composition can comprise a cosmetically or pharmaceutically effective quantity of at least one whitening or depigmenting agent or melanin synthesis inhibiting agent, selected, for example and not restricted to, from the extracts of *Achillea millefolium, Aloe vera, Aradirachta indica, Asmuna japonica, Autocarpus incisus, Bidens pilosa, Broussonetia papyrifera, Chlorella vulgaris, Cimicifuga racemosa, Emblica officinalis, Glycyrrhiza glabra, Glycyrrhiza uralensis, Ilex purpurea, Ligusticum lucidum, Ligusticum wallichii, Mitracarpus scaber, Morinda citrifolia, Morus alba, Morus bombycis, Naringi crenulata, Prunus domesticus, Pseudostellariae radix, Rumex crispus, Rumex occidentalis, Sapindus mukurossi, Saxifragia sarmentosa, Scutellaria galericulate, Sedum sarmentosum bunge, Stellaria medica, Triticum Vulgare, Arctostaphylos Uva ursi* or *Whitania somnifera* among others and/or Lipochroman^{®} [INCI: Dimethylmethoxy Chromanol], Chromabright^{®} [INCI: Dimethylmethoxy Chromanyl Palmitate] or Brighlette^{™} [INCI: Plankton Extract] marketed by Lipotec/Lubrizol; Whitami [INCI: Papain, Titanium Dioxide, Angelica Acutiloba Root Extract, Saposhnikovia Divaricata Root Extract, Thioctic Acid, Kaolin, Ascorbyl Glucoside, Pinus Pinaster Bark Oligomeric Proanthocyanidins] marketed by Alban Muller; NAB^{®} Asafetida Extract [INCI: Ethoxydiglycol, Ferula Foetida Extract] marketed by Arch/Lonza; Licorice Roots Extract [INCI: Licorice (Glycyrrhiza Glabra) Extract] marketed by Campo Research; Belides^{™} [INCI: Bellis Perennis (Daisy) Flower Extract] marketed by CLR; Algowhite [INCI: Ascophyllum Nodosum Extract] marketed by Codif; Biowhite^{™} [INCI: Saxifraga Sarmentosa Extract, Vitis Vinifera (Grape) Fruit Extract, Morus Bombycis Root Extract, Scutellaria Baicalensis Root Extract], Melarrest^{®} A [INCI: Lactic Acid, Kojic Acid, Ascorbic Acid], Melarrest^{®} L [INCI: Phospholipids, Glycyrrhiza Glabra (Liquorice) Extract, Kojic Acid, Ammonium Glycyrrhizate], Vitagen [INCI: Aminopropyl Ascorbyl Phosphate] or Collalift [INCI: Hydrolyzed Malt Extract] marketed by Coletica/Engelhard/BASF; DC Skin Bright^{™} [INCI: PEG-12 Glyceryl Distearate, Methyl Dihydroxybenzoate, Ethoxydiglycol, Polyethylene] marketed by DC Ingredients; DS-Whitekle [INCI: Acetylphytosphingosine] marketed by Doosan; TEGO Cosmo C 250 [INCI: 1-Methylhydantoine-2-imide] or TEGO Pep 4-Even [INCI: Tetrapeptide-30] marketed by Evonik Goldschmidt; Albatin^{®} [INCI: Aminoethylphosphinic Acid] marketed by Exsymol; Synerlight^{™} [INCI: Actinidia Chinensis (Kiwi) Fruit Water, Sophora Angustifolia Root Extract] or Gatuline^{®} Spot-Light [INCI: Actinidia Chinensis (Kiwi) Fruit Water, Sophora Flavescens Root Extract] marketed by Gattefossé; Clerilys^{™} [INCI: Cucumis Santivus, Morus Alba Extract, Hibiscus Sabdariffa Extract, Wine Extract] marketed by Greentech; Melanostatine^{®}-5 [INCI: Dextran, Nonapeptide-1] marketed by lEB/Unipex; Actiwhite^{™} [INCI: Sucrose Dilaurate, Polysorbate 20, Pisum Sativum Extract], Active^{®} Powder Whiteness [INCI: Lauryl Methacrylate/Glycol Dimethacrylate Copolymer, Dicaprylyl Ether, Titanium Dioxide, Algae, Waltheria Indica Leaf Extract, Ferulic Acid, Polyglyceryl-2-Dipolyhydroxystearate], Dermawhite^{®} NF LS 9410 [INCI: Mannitol, Waltheria Indica Leaf Extract, Dextrin, Ferulic Acid], Dermawhite^{®} WF [INCI: Tartaric Acid, Saxifraga Sarmentosa Extract, Carica Papaya (Papaya) Fruit Extract, Pisidium Guajava Fruit Extract] or Radianskin^{™} [INCI: Hydroxyphenoxy Propionic Acid] marketed by L. Serobiologiques/Cognis/BASF; Lipobrite^{®} HCA-4 [INCI: PEG-4, Hydroxycinnamic Acid] marketed by Lipochemicals; Whitessence^{™} [INCI: Artocarpus Heterophyllus Seed Extract] marketed by Lucas Meyer Cosmetics/Unipex; Emblica^{™} [INCI: Phyllanthus Emblica Fruit Extract] or RonaCare^{®} Pristine Bright^{™} [INCI: Methoxyphenyl t-Butylphenyl Propanediol] marketed by Merck; SulforaWhite [INCI: Lepidium Sativum Sprout Extract], Delentigo^{™} [INCI: Lepidium Sativum Sprout Extract, Lecithin, Soy Isoflavones] marketed by Mibelle; Alpha-Arbutin [INCI: Alpha-arbutin], Gigawhite [INCI: Malva Sylvestris (Mallow) Extract, Mentha Piperita Leaf Extract, Primula Veris Extract, Alchemilla Vulgaris Extract, Veronica Officinalis Extract, Melissa Officinalis Leaf Extract, Achillea Millefolium Extract], Melawhite^{®} [INCI: Leukocyte Extract, AHA], Melfade^{®}-J [INCI: Arctostaphylos Uva-Ursi Leaf Extract, Magnesium Ascorbyl Phosphate] or Regu-Fade [INCI: Resveratrol] marketed by Pentapharm/DSM; CellActive^{®} White [INCI: Niacinamide, Zinc PCA, Chlorella Vulgaris/Lupinus Albus Protein Ferment, Nasturtium Officinale Extract] or Illumiscin^{®} [INCI: Olea Europaea Leaf Extract, Ascorbyl Glucoside, Zinc PCA] marketed by Rahn; Arlatone^{™} Dioic DCA [INCI: Octadecenedioic Acid, BHT], Etioline^{™} [INCI: Arctostaphylos Uva Ursi Leaf Extract, Mitracarpus Scaber Extract], Lumiskin^{™} [INCI: Caprylic/Capric Triglycerid, Diacetyl Boldine], Melaclear^{™} 2 [INCI: Dithiaoctanediol, Gluconic Acid, Sutilains, Beta-carotene], Lumisphere^{™} [INCI: Cetyl Hydroxyethylcellulose, Polymethylmethacrylate, Trilaurin, Diacetyl Boldine], O.D.A.white^{™} [INCI: Octadecenedioic Acid] Wonderlight^{™} [INCI: Humulus Lupulus (Hops) Strobile], Mediatone^{™} [INCI: Octadecenedioic Acid] or Senestem^{™} [INCI: Plantago Lanceolata Leaf Extract] marketed by Sederma/Croda; Sepiwhite^{™} MSH [INCI: Undecylenoyl Phenylalanine], Sepicalm^{™} VG [INCI: Sodium Palmitoyl Proline, Nymphaea Alba Flower Extract] or Sea Shine [INCI: Algae Extract, Undaria Pinnatifida Extract] marketed by Seppic; Clariskin II [INCI: Triticum Vulgare Extract], Dermalight^{®} [INCI: Tropaeolum Majus Extract] or Whitonyl^{®} [INCI: Palmaria Palmata Extract] marketed by Silab; DermaPep^{®} A350 [INCI: Myristoyl Tripeptide-31] or DermaPep^{®} W411 [INCI: Palmitoyl Hexapeptide-36, Methyl Undecenoyl Leucinat] marketed by Miwon Commercial; Neurolight 61G [INCI: Pancratium Maritimum Extract] marketed by Codif; Azeloglicina^{®} [INCI: Potassium Azelaoyl Diglycinate] marketed by Sinerga; Whitesphere Premium [INCI: Sucrose Palmitate, Glyceryl Linoleate, Prunus Amygdalus Dulcis, Almond Oil, Glycyrrhiza Glabra (Liquorice) Root Extract, Magnesium Ascorbyl Phosphate, Undaria Pinnatifida Extract] or Axolight [INCI: Triticum Aestivum Extract] marketed by Soliance; SymWhite^{®} [INCI: Phenylethyl Resorcinol] or Extrapone^{™} Nutgrass GW [INCI: Cyperus Rotundus Root Extract] marketed by Symrise; Synovea^{®} HR [INCI: Hexylresorcinol] marketed by Sytheon; β-White [INCI: Hydrogenated Lecithin, Sodium Oleate, Oligopeptide-68] marketed by Unipex; Achromaxyl^{™} [INCI: Brassica Napus Extract] marketed by Vincience/ISP; Active.Lite^{®} [INCI: Saccharomyces/Grape Ferment Extract] marketed by Active Concepts; Brightenyl^{™} [INCI: Diglucosyl Gallic Acid] marketed by Induchem/Givaudan; CinderellaCare [INCI: Thymus Serpillum Extract] marketed by Ichimaru Pharcos; Darkout^{™} [INCI: Hypoxis Rooperi Rhizome Extract, Caesalpinia Spinosa Gum] or Melavoid^{™} [INCI: Boerhavia Diffusa Root Extract] marketed by Provital; Kimarine^{®} [INCI: Undaria Pinnatifida Extract] marketed by Gelyma; Omegalight^{®} [INCI: Safflower Seed Oil Piperonyl Esters] or Solawhite^{®} [INCI: Calcium Alginate, Titanium Dioxide, Boron Nitride, Biosaccharide Gum-4] marketed by Solabia; Beracare BBA [INCI: Pentaclethra Macroloba Seed Oil] marketed by Beraca; Dermostatyl^{™} IS [INCI: Hexapeptide-2] marketed by Ashland; Et-VC^{™} [INCI: 3-O-Ethyl Ascorbic Acid] or GenoWhite^{™} [INCI: Acetyl Glycyl β-Alanine] marketed by Corum; Inside Light Poet's narcissus [INCI: Narcissus Poeticus Callus Extract] marketed by Naolys; Phytessence^{™} White Peony [INCI: Paeonia Lactiflora Root Extract] marketed by Croda; ReGeniStem^{™} Brightening [INCI: Glycyrrhiza Glabra (Licorice) Root Callus Culture Extract, Prunus Armeniaca (Apricot) Kernel Oil] marketed by Lonza; Skin Moon^{®} [INCI: Citrus Sinensis (Orange) Fruit Water, Oryza Sativa (Rice) Extract, Capparis Spinosa Fruit Extract, Olea Europaea (Olive) Leaf Extract] marketed by Bionap; Sweetone^{®} [INCI: Saccharide Hydrolysate] marketed by Laboratoires Expanscience; arbutin and its isomers, kojic acid and its derivatives, vitamin C and its derivatives, for example and not restricted to, 6-O-palmitoyl ascorbic acid, dipalmitoyl ascorbic acid, magnesium salt from ascorbic-2-phosphate acid (MAP), sodium from ascorbic-2-phosphate acid (NAP), ascorbyl glucoside or ascorbyl tetraisopalmitate (VCIP) among others, retinol and its derivatives, including tretinoin and isotretinoin, idebenone, hydroxybenzoic acid and its derivatives, flavonoides, soy extract, extract of lemon, extract of orange, extract of ginkgo, extract of cucumber, extract of geranium, extract of bearberry, extract of carob, extract of cinnamon, extract of marjoram, extract of rosemary, extract of clove, soluble extract of liquorice, extract of blackberry leaf, niacinamide, liquiritin, resorcinol and its derivatives, hydroquinone, α-tocopherol, γ-tocopherol, azelaic acid, resveratrol, mercury salts, linoleic salts, α-lipoic acid, dihydrolipoic acid, alfa hydroxy acids, beta hydroxy acids, ellagic acid, ferulic acid, cinnamic acid, oleanolic acid, aloesin and its derivatives and/or inhibitors of serine protease activity, for example and not restricted to, inhibitors of tryptase, trypsin or PAR-2 activity, among others, and/or mixtures thereof.

The cosmetic or pharmaceutical composition can comprise a cosmetically or pharmaceutically effective quantity of at least one natural extract or essential oil for the treatment of sensitive skin, selected from, for example and not restricted to, extracts of *Abelmoschus esculentus,* amaranth seed oil, oil from *Alkanna tinctoria* wood, *Aloe vera, Althaea officinalis, Altingia excelsa, Andropogon virginicus, Aralia nudicaulis, Aralia racemosa, Argemone mexicana, Arnica montana, Artemisia vulgaris, Asarum maximum, Barleria prionitis, Calendula officinalis, Camelia sinensis, Caesalpinia digyna, Campsis grandiflora, Capsicum spp., Carissa congesta, Carthamus oxyacantha, Cassia tora, Centipeda cunninghamii, Chamomilla recutita, Chrysanthemum indicum, Cimicifuga racemosa, Cinnamomum camphora, Clematis vitalba, Cuscuta reflexa, Crinum asiaticum, Diospyros peregrina, Enicostema axillare, Hamamelis virginiana, Harpagophytum procumbens, Hypericum perforatum, Jatropha multifida, Lavandula officinalis, Lavandula latifolia, Lilium candidum, Liquidambar orientalis, Lithospermum officinale, Madhuca longifolia, Malva sylvestris, Martynia annua, Melaleuca alternifolia, Medicago sativa, Michelia champaca, Mikania glomerata, Mimosa pudica, Origanum majorana, Origanum vulgare, Oryza sativa, Phaseolus vulgaris, Phyllanthus urinaria, Phyllanthus virgatus, Pistacia vera, Polygonum hydropiper, Prunus laurocerasus, Rosmarinus officialis, Quercus ilex, Rauvolfia caffra, Ricinus communis, Rubus idaeus, Sagittaria sagittifolia, Salix alba, Sandoricum koetjape, Sapindus mukorossi, Schleichera oleosa, Sesbania grandiflora, Silybum marianum, extracts of Spondias dulcis, Tanacetum parthenium, Thymus vulgaris, Tilia sp., Toona ciliata, Tragia involucrata, Trichosanthes quinquangulata, Uncaria guianensis or Vaccaria pyramidata, Vaccinum myrtillus, Ventilago madraspatana, Veratrum album or Xanthium* strumarium among others; coenzyme Q10 or alkyl glycerol ethers, Abyssine^{®} [INCI: Alteromonas Ferment Extract], Aldavine^{™} [INCI: Ascophyllum Nodosum Extract, Asparagopsis Armata Extract, Sorbitol], Neutrazen^{™} [INCI: Dextran, Palmitoyl Tripeptide-8] or Melitane^{®} [INCI: Dextran, Acetyl Hexapeptide-1] marketed by Atrium/Unipex; AquaCacteen^{®} [INCI: Opuntia Ficus Indica Stem Extract], CM-Glucan [INCI: Sodium Carboxymethyl Betaglucan] or MAXnolia [INCI: Magnolia Officinalis Bark Extract, Vitis Vinifera/Vitis Vinifera (Grape) Seed Extract, Tocopherol] marketed by Mibelle; Bacocalmine^{™} [INCI: Bacopa Monniera Extract], Calmosensine^{™} [INCI: Acetyl Dipeptide-1 Cetyl Ester] or Pacifeel^{™} [INCI: Mirabilis Jalapa Extract] marketed by Sederma/Croda; Defensil^{®} [INCI: Octyl Dodecanol, Echium Plantagineum Seed Oil, Cardiospermum Halicacabum Extract, Helianthus Annuus Seed Oil Unsaponifiables] marketed by Rahn; DS-Phytosphingosine [INCI: Phytosphingosine], DS-TAPS [INCI: Tetraacetylphytosphingosine] or DS-Whitekle [INCI: Acetylphytosphingosine] marketed by Doosan; Elhibin^{®} [INCI: Glycine Soja Protein, Disodium cocoamphodiacetate] marketed by Pentapharm/DSM; Gatuline^{®} Derma-Sensitive [INCI: Octyldodecyl Myristate, Capparis Spinosa Fruit Extract] marketed by Gattefossé; Glutrapeptide^{®} [INCI: Pyroglutamylamidoethyl Indole] or Glistin^{®} [INCI: Glutamylamidoethyl Indole] marketed by Exsymol; Inhipase^{™} [INCI: Pueraria Lobata Root Extract] or Symbiocell^{™} [INCI: Cestrum Latifolium Leaf Extract] marketed by BASF; Meliprene^{®} [INCI: Dextran, Acetyl Heptapeptide-1] marketed by Institut Européen de Biologie Cellulaire/Unipex Group; NAB^{®} Arnica Extract [INCI: Arnica Montana Flower Extract, Algae Extract] marketed by Arch/Lonza; Ocaline [INCI: Cucurbita Pepo (Pumpkin) Seed Extract], Mariliance [INCI: Rhodosorus Marinus Extract] or Soothex^{®} [INCI: Boswellia Serrata Gum] marketed by Soliance/Givaudan; Phytosphingosine [INCI: Phytosphingosine] marketed by Evonik Goldschmidt; Protectol [INCI: Betula Alba Bark Extract, Scrophularia Nodosa Extract] marketed by Greentech; Skinasensyl^{™} [INCI: Coco-Glucoside, Acetyl Tetrapeptide-15] or Eperuline^{™} [INCI: Eperua Falcata Bark Extract] marketed by L. Serobiologiques/Cognis/BASF; SymSitive^{®} 1609 [INCI: 4-t-Butylcyclohexanol], SymCare^{®} O [INCI: 4-t-Butylcyclohexanol, Bisabolol, Cetylhydroxyproline Palmitamide, Propamidobenzoic Acid, Brassica Campestris (Rapeseed) Sterols, Zingiber Officinale (Ginger) Root Extract] or SymCare^{®} W [INCI: 4-t-Butylcyclohexanol, PEG-40 Hydrogenated Castor Oil, Trideceth-9, Hydroxyphenyl Propamidobenzoic Acid] marketed by Symrise; Stimu-Tex^{®} AS [INCI: Spent Grain Wax, Butyrospermum Parkii (Shea Butter) Extract, Argania Spinosa Kernel Oil] marketed by Pentapharm/DSM; Timecode^{™} [INCI: Palmitoyl Glycine] or Sepicalm S [INCI: Sodium Cocoyl Amino Acids, Sarcosine, Potassium aspartate, Magnesium Aspartate] marketed by Seppic; Unisooth ST-32 [INCI: Tamarindus Indica Seed Extract, Stevioside] marketed by Induchem; Vital ET^{®} [INCI: Disodium Lauriminodipropionate Tocopheryl Phosphates] marketed by ISP; Delisens^{™} [INCI: Acetyl Hexapeptide-49] or Telangyn^{™} [INCI: Acetyl Tetrapeptide-40] marketed by Lipotec/Lubrizol; Ambora extract [INCI: Tambourissa Trichophylla Leaf Extract] or Embelia Extract [INCI: Embelia Concinna Leaf Extract] marketed by Bayer; Caresoft^{™} [INCI: Curculigo Orchioides Root Extract] marketed by Provital; PhytoDefense CLR^{™} [INCI: Glycine Soja (Soybean) Oil, Dicaprylyl Ether, Magnolia Grandiflora Bark Extract, Lauryl Alcohol] marketed by CLR Chemisches Laboratorium; or Sereniks-207 [INCI: Tsuga Canadensis Leaf Extract] marketed by Biopharmacopae, among others, and/or mixtures thereof.

The cosmetic or pharmaceutical composition can comprise a cosmetically or pharmaceutically effective quantity of at least one anti-itching agent selected from, for example and not restricted to, extracts of *Abelmoschus esculentus, Actaea alba, Aglaia odorata, Alkanna tinctoria, Althaea officinalis, Altingia excelsa, Andropogon virginicus, Aralia nudicaulis, Aralia racemosa, Argemone mexicana, Barleria prionitis, Camelia sinensis, Caesalpinia digyna, Campsis grandiflora, Carissa congesta, Carthamus oxyacantha, Cassia tora, Chrysanthemum indicum, Cimicifuga racemosa, Cinnamomum camphora, Clematis vitalba, Cuscuta reflexa, Diospyros peregrina, Enicostema axillare, Hammamelis virginiana, Jatropha multifida, Lavandula officinalis, Lavandula latifolia, Liquidambar orientalis, Lithospermum officinale, Madhuca longifolia, Martynia annua, Medicago sativa, Michelia champaca, Mikania glomerata, Mimosa pudica, Oryza sativa, Phaseolus vulgaris, Phyllanthus urinaria, Phyllanthus virgatus, Pistacia vera, Polygonum hydropiper, Quercus ilex, Rauvolfia caffra, Ricinus communis, Rubus idaeus, Sagittaria sagittifolia, Sandoricum koetjape, Sapindus mukorossi, Schleichera oleosa, Sesbania grandiflora, Spondias dulcis, Tilia sp., Toona ciliata, Tragia involucrata, Trichosanthes quinquangulata, Vaccaria pyramidata, Ventilago madraspatana, Veratrum album* or *Xanthium strumarium* among others or as well as one synthetic compound or product of biotechnological origin which is an anti-itching agent, for example and not restricted to, mepyramine (pyrilamine), antazoline, diphenhydramine, carbinoxamine, doxylamine, clemastine, dimenhydrinate, pheniramine, chlorphenamine (chlorpheniramine), dexchlorpheniramine, brompheniramine, triprolidine, cyclizine, chlorcyclizine, hydroxyzine, meclizine, cetirizine, levocetirizine, promethazine, thenaldine, alimemazine (trimeprazine), cyproheptadine, azatidine, ketotifen, acrivastine, astemizole, cetirizine, loratadine, desloratadine, mizolastine, terfenadine, fexofenadine, fexofenadine, azelastine, levocabastine, olopatadine, corticosteroids such as cortisone, hydrocortisone dexamethasone, prednisone; Neutrazen^{™} [INCI: Dextran, Palmitoyl Tripeptide-8] marketed by Atrium /Unipex Innovations; Meliprene^{®} [INCI: Dextran, Acetyl Heptapeptide-1] marketed by Institut Européen de Biologie Cellulaire/Unipex Innovations; Delisens^{™} [INCI: Acetyl Hexapeptide-49] marketed by Lipotec/Lubrizol; Skinasensyl^{™} [INCI: Acetyl Tetrapeptide-15] marketed by Laboratoires Serobiologiques/Cognis/BASF; SymSitive^{®} 1609 [INCI: 4-t-Butylcyclohexanol] marketed by Symrise; Symbiocell^{™} [INCI: Extract from Cestrum Latifolium] marketed by BASF; Gatuline^{®} Derma-Sensitive [INCI: Octyldodecyl Myristate, Capparis Spinosa Fruit Extract] marketed by Gattefossé; MAXnolia [INCI: Magnolia Officinalis Bark Extract, Vitis Vinifera/Vitis Vinifera (Grape) Seed Extract, Tocopherol] marketed by Mibelle Biochemistry; Calmosensine^{™} [INCI: Acetyl Dipeptide-1 Cetyl Ester] or Pacifeel^{™} [INCI: Mirabilis Jalapa Extract] marketed by Sederma/Croda; or PhytoDefense CLR^{™} [INCI: Glycine Soja (Soybean) Oil, Dicaprylyl Ether, Magnolia Grandiflora Bark Extract, Lauryl Alcohol] marketed by CLR Chemisches Laboratorium among others, or mixtures thereof.

The cosmetic or pharmaceutical composition can comprise a cosmetically or pharmaceutically effective quantity of at least one anti-inflammatory agent and/or analgesic is selected, for example and not restricted to, from the group formed by extract of madecassoside, extract of echinacea, amaranth seed oil, sandal wood oil, extract of peach tree leaf, extract of *Aloe vera, Arnica montana, Artemisia vulgaris, Asarum maximum, Calendula officinalis, Capsicum, Centipeda cunninghamii, Chamomilla recutita, Crinum asiaticum, Hamamelis virginiana, Harpagophytum procumbens,* Hypericum *perforatum, Lilium candidum, Malva sylvestris, Melaleuca alternifolia, Origanum majorana, Origanum vulgare, Prunus laurocerasus, Rosmarinus officialis, Salix alba, Silybum marianum, Tanacetum parthenium, Thymus vulgaris, Uncaria guianensis* or *Vaccinum myrtillus,* omega-3 and omega-6 fatty acids, Neutrazen^{™} [INCI: Dextran, Palmitoyl Tripeptide-8] marketed by Atrium Innovations/Unipex Group; Delisens^{™} [INCI: Acetyl Hexapeptide-49] marketed by Lipotec/Lubrizol; Meliprene^{®} [INCI: Dextran, Acetyl Heptapeptide-1] marketed by Institut Européen de Biologie Cellulaire/Unipex Group; Skinasensyl^{™} [INCI: Acetyl Tetrapeptide-15] or Anasensyl^{™} [INCI: Mannitol, Ammonium Glycyrrhizate, Caffeine, Hippocastanum (Horse Chestnut) Extract] marketed by BASF; Calmosensine^{™} [INCI: Acetyl Dipeptide-1] marketed by Sederma/Croda; coenzyme Q10 or alkyl glyceryl ethers among others, or mixtures thereof.

The cosmetic or pharmaceutical composition can comprise a cosmetically or pharmaceutically effective quantity of at least anti-psoriasis, anti-dermatitis and/or anti-eczema agent, for example and not restricted to, corticosteroids, such as chlobetasol, betametasone, dexamethasone, halobetasol, diflorasone, fluocinonide, halcinonide, amcinonide, desoxymethasone, triamcinolone acetonide, mometasone, fluticasone, fluocinolone acetonide, flurandrenolide, desonide, prednicarbate, hidrocortisone, calcipotriene, vitamin D, anthralin, calcitriol, salicylic acid; coal tar, derivatives and sub-products of the coal industry; tar, derivatives and sub-products of petroleum distillation, tazarotene, antibiotics, azathioprine, colchicine, 5-fluorouracil, fumaric acid esters, hydroxyurea, mycophenolate mofetil, propylthiouracil, sulfasalazine, 6-thioguanine, calcineurin inhibitors, for example and not restricted to, tacrolimus and pimecrolimus; or topically applied retinoids, for example and not excluding, tretinoin among others, and/or mixtures thereof.

The compositions may be for use in any of the applications or uses discussed above under the heading "Applications".

The invention is illustrated by the following non-limiting examples.

### EXAMPLES

### General Methodology

All reagents and solvents are of synthesis quality and are used without additional treatment.

### Abbreviations

The abbreviations used for amino acids follow the 1983 IUPAC-IUB Joint Commission on Biochemical Nomenclature recommendations outlined in Eur. J. Biochem. (1984) 138:9-37.

(R), resin; 2-CITrt-(R), 2-chlorotrityl resin; Ac, acetyl; AcOH, acetic acid; Ala, alanine; AM, 2-[4-aminomethyl-(2,4-dimethoxyphenyl)] phenoxyacetic acid; Arg, arginine; Asn, asparagine; Asp, aspartic acid; Boc, tert-butyloxycarbonyl; DCM, dichloromethane; DIEA, *N,N'*-diisopropylethylamine; DIPCDI, *N,N'-*diisopropylcarbodiimide; DMF, N,N-dimethylformamide; ESI-MS, electrospray ionization mass spectrometry; Fmoc, 9-flluorenylmethyloxycarbonyl; Gln, glutamine; Glu, glutamic acid; Gly, Glycine; His, histidine; HOBt, 1-hydroxybenzotriazole; HPLC, high performance liquid chromatography; Ile, isoleucine; KOH, potassium hydroxide; Leu, leucine; Lys, lysine; MBHA, p-methylbenzhydrylamine; MeCN, acetonitrile; MeOH, methanol; Met, Methionine; Myr, myristoyl; Palm, palmitoyl; Pbf, 2,2,4,6,7-pentamethyldihydrobenzofuran-5-sulfonyl; Pro, proline; Ser, serine; tBu, tert-butyl; TFA, trifluoroacetic acid; Thr threonine; Trt, trithyl; Val, valine.

### Chemical Synthesis

All synthetic processes are carried out in polypropylene syringes fitted with porous polyethylene discs. All the reagents and solvents are synthesis quality and are used without any additional treatment. The solvents and soluble reagents are removed by suction. The Fmoc group is removed with piperidine-DMF (2:8, v/v) (1 × 1 min, 1 × 5 min, 5 ml/g resin) *[*Lloyd- Williams P. et al. (1997) "Chemical Approaches to the Synthesis of Peptides and Proteins" CRC, Boca Raton (FL, USA*)j.* Washes between stages of deprotection, coupling and, again, deprotection, are carried out with DMF (3x1 min) each time using 10 ml solvent/g resin. Coupling reactions are performed with 3 ml solvent/g resin. The control of the couplings is performed by carrying out the ninhydrin test *[*Kaiser E. etal., Anal. Biochem. (1970), 34: 595-598*]* or chloranil test *[*Christensen T., Acta Chem. Scand., (1979), 338, 763-766]. All synthetic reactions and washes are carried out at 25°C.

Some amino acids are used with their functional groups in side chains protected. Protecting groups used are:
- tBu, tert-butyl for protection of aminoacids Asp, Glu, Thr and Ser;
- Boc, tert-butyloxycarbonyl for aminoacid Lys;
- Pbf, 2,2,4,6,7-pentamethyldihydrobenzofuran-5-sulfonyl for aminoacid Arg; and
- Trt, trithyl for aminoacids Asn, Gln and His.

HPLC chromatographic analysis is carried out with Shimadzu equipment (Kyoto, Japan) using a reversed-phase column thermostatized at 30°C (50 x 4.6 mm, Kromasil C18, 3.5 µm, Akzo Nobel, Sweden). The elution is carried out using a gradient of acetonitrile (+0.07% TFA) in water (+0.1% TFA) at a flow rate of 1.6 mL/min and detection is carried out at 220 nm. The electrospray ionization mass spectrometry is carried out in a WATERS Alliance ZQ 2000 detector using a mixture of MeCN:H₂O 4:1 (+0.1% TFA) as the mobile phase and a flow rate of 0.3 ml/min.

### EXAMPLE 1

### Obtaining H-AA₈-O-2-ClTrt-(R), wherein AA₈ is L-Asp, L-Asn or L-Glu.

Weights have been normalized. 3.2 mmol (1 equiv) of Fmoc-L-Asp(tBu)-OH, Fmoc-L-Asn(Trt)-OH or Fmoc-L-Glu(tBu)-OH dissolved in 20 ml of DCM, to which is added 0.83 equiv of DIEA, is coupled onto dry 2-chlorotrityl resin (3.2 mmol) with a functionalization 1.6 mmol/g. The mixture is stirred for 5 min, after which 1.63 equiv of DIEA are added. The mixture is left to react for 40 min. The remaining chloride groups are blocked by treatment with 2 ml of MeOH.

The resin is then washed and the *N*-terminal Fmoc group is deprotected as described in the general methods.

### Obtaining H-AA₆-O-2-ClTrt-(R) wherein AA₆ is L-Arg or L-Lys.

Weights have been normalized. 1.6 mmol (1 equiv) of Fmoc-Arg(Pbf)-OH or Fmoc-L-Lys(Boc)-OH dissolved in 10 ml of DCM, to which is added 0.83 equiv of DIEA, is coupled onto dry 2-chlorotrityl resin (1.6 mmol) with a functionalization 1.6 mmol/g. The mixture is stirred for 5 min, after which 1.63 equiv of DIEA are added. The mixture is left to react for 40 min. The remaining chloride groups are blocked by treatment with 1 ml of MeOH.

The resin is then washed and the N-terminal Fmoc group is deprotected as described in the general methods.

### EXAMPLE 2

### Obtaining Fmoc-Wₘ-Xₙ-AA₁-AA₂-AA₃AA₄-AA₅-AA₆-AA₇-AA₈-Yₚ-Z_{q}-O-2-ClTrt-(R), wherein AA₁ is L-Glu; AA₂ is L-Glu; AA₃ is L-Met; AA₄ is L-Gln; AAs is L-Arg; AA₆ is L-Arg; AA₇ is Ala; AA₈ is L-Asp; and n, m, p and q are each 0.

Weights have been normalized. 143mg of H-L-Asp(tBu)-O-2-Cl-Trt resin with a functionalization of 0.98 mmol/g (0.14 mmol) is washed as described in the general methods. Following the protocols described, 5 equiv of Fmoc-Ala-OH (Fmoc-AA₇-OH) is coupled onto the peptidyl resins in the presence of 5.5 equiv of DIPCDI and 5 equiv of HOBt using DMF as a solvent for 60 minutes.

The resin is then washed as described in the general methods and the deprotection treatment of the Fmoc group is repeated to couple the next amino acid. Following the previously described protocols, 5 equiv of Fmoc-L-Arg(Pbf)-OH (Fmoc-AA₆-OH) is coupled onto the peptidyl resins in the presence of 5.5 equiv of DIPCDI and 5 equiv of HOBt using DMF as a solvent for 60 minutes. The resin is then washed as described in the general methods and the deprotection treatment of the Fmoc group is repeated to couple the next amino acid. Following the previously described protocols, steps of coupling, washing and deprotecting are repeated to sequentially couple 5 equiv of Fmoc-L-Arg(Pbf)-OH (Fmoc-AA₅-OH); 5 equiv of Fmoc-L-Gln(Trt)-OH (Fmoc-AA₄-OH); 5 equiv of Fmoc-L-Met-OH (Fmoc-AA₃-OH); 5 equiv of Fmoc-L-Glu(tBu)-OH (Fmoc-AA₂-OH); and 5 equiv of Fmoc-L-Glu(tBu)-OH (Fmoc-AA₁-OH); each of them in presence of 5 equiv of HOBt and 5.5 equiv of DIPCDI during the coupling step.

After the synthesis, the peptidyl resin is washed with DCM (3 × 1 min).

All the peptides shown in Table 5a might be synthesized following the protocol described in this example

**Table 5a**

| Peptides |
|---|
| Fmoc-Glu-Glu-Met-Gln-Arg-Arg-Ala-Asp-O-2-Cl-Trt-(R) |
| Fmoc-Glu-Glu-Met-Gln-Arg-Arg-Ala-Glu-O-2-Cl-Trt-(R) |
| Fmoc-Glu-Glu-Met-Glu-Arg-Arg-Ala-Asp-O-2-Cl-Trt-(R) |
| Fmoc-Gln-Glu-Met-Gln-Arg-Arg-Ala-Asp-O-2-Cl-Trt-(R) |
| Fmoc-Glu-Glu-Met-Gln-Arg-Arg-Ala-Asn-O-2-Cl-Trt-(R) |
| Fmoc-Glu-Glu-Met-Gln-Arg-Arg-O-2-Cl-Trt-(R) |
| Fmoc-Glu-Glu-Met-Gln-Arg-Lys-O-2-Cl-Trt-(R) |
| Fmoc-Glu-Glu-Leu-Gln-Arg-Arg-O-2-Cl-Trt-(R) |

### EXAMPLE 3

### Obtaining Fmoc-Wₘ-Xₙ-AA₁-AA₂-AA₃-AA₄-AA₅-AA₆-AA₇-AA₈-Yₚ-Z_{q}-AM-MBHA-(R), wherein AA₁ is L-Glu; AA₂ is L-Glu; AA₃ is L-Met; AA₄ is L-Gln; AAs is L-Arg; AAs is L-Arg; AA₇is Ala; AA₈ is L-Asp; and m, n, p and q are each 0.

Weights have been normalized. 230 mg (0.12 mmol) of Fmoc-AM-MBHA resin with a functionalization of 0.51 mmol/g is treated with piperidine:DMF according to the described general protocol in order to remove the Fmoc group. 5 equiv of Fmoc-L-Asp(tBu)-OH (Fmoc-AA₈-OH) is incorporated onto the deprotected resin in the presence of 5.5 equiv of DIPCDI and 5 equiv of HOBt using DMF as a solvent for 1 hour.

The resin is then washed as described in the general methods and the deprotection treatment of the Fmoc group is repeated to couple the next amino acid Following the previously described protocols 5 equiv of Fmoc-Ala-OH (Fmoc-AA₇-OH); and subsequently 5 equiv of Fmoc-L-Arg(Pbf)-OH (Fmoc-AA₆-OH); 5 equiv of Fmoc-L-Arg(Pbf)-OH (Fmoc-AA₅-OH); 5 equiv of Fmoc-L-Gln(Trt)-OH (Fmoc-AA₄-OH); 5 equiv of Fmoc-L-Met-OH (Fmoc-AA₃-OH); 5 equiv of Fmoc-L-Glu(tBu)-OH (Fmoc-AA₂-OH); and finally 5 equiv of Fmoc-L-Glu(tBu)-OH (Fmoc-AA₁-OH) are sequentially coupled in the presence of 5 equiv of HOBt and 5.5 equiv of DIPCDI in each coupling step.

After the synthesis, the peptidyl resin is washed with DCM (3 × 1 min).

All the peptides showed in Table 5b might be synthesized following the protocol described in this example.

| Table 5b |
|---|
| Peptides |
| Fmoc-Asp-Glu-Met-Gln-Arg-Arg-Ala-Asp-AM-MBHA-(R) |
| Fmoc-Gln-Glu-Met-Gln-Arg-Arg-Ala-Asp-AM-MBHA-(R) |
| Fmoc-Glu-Glu-Leu-Gln-Arg-Arg-Ala-Asp-AM-M BHA-(R) |
| Fmoc-Glu-Glu-Met-Glu-Arg-Arg-Ala-Asp-AM-MBHA-(R) |
| Fmoc-Glu-Glu-Met-Asn-Arg-Arg-Ala-Asp-AM-MBHA-(R) |
| Fmoc-Glu-Glu-Met-Asp-Arg-Arg-Ala-Asp-AM-M BHA-(R) |
| Fmoc-Glu-Glu-Met-Gln-Arg-Lys-Ala-Asp-AM-MBHA-(R) |
| Fmoc-Glu-Glu-Met-Gln-Arg-Arg-Ser-Asp-AM-M BHA-(R) |
| Fmoc-Glu-Glu-Met-Gln-Arg-Arg-Gly-Asp-AM-MBHA-(R) |
| Fmoc-Glu-Glu-Met-Gln-Arg-Arg-Ala-Glu-AM-MBHA-(R) |
| Fmoc-Glu-Glu-Met-Gln-Arg-Arg-Ala-Asn-AM-M BHA-(R) |
| Fmoc-Glu-Glu-Met-Gln-Arg-Arg-Ala-Gln-AM-MBHA-(R) |
| Fmoc-Glu-Glu-Met-Gln-Arg-His-Ala-Asp-AM-MBHA-(R) |
| Fmoc-Glu-Glu-Met-Gln-Arg-Arg-Thr-Asp-AM-MBHA-(R) |
| Fmoc-Glu-Glu-Met-Gln-Arg-Arg-Pro-Asp-AM-M BHA-(R) |
| Fmoc-Glu-Glu-Met-Gln-Arg-Arg-Glu-Asp-AM-M BHA-(R) |
| Fmoc-Glu-Glu-Met-Gln-Arg-Arg-Lys-Asp-AM-M BHA-(R) |
| Fmoc-Glu-Glu-Met-Gln-Arg-Arg-Ala-Asp-AM-MBHA-(R) |
| Fmoc-Glu-Glu-Met-Gln-Arg-Arg-Ala-AM-MBHA-(R) |
| Fmoc-Glu-Glu-Met-Gln-Arg-Arg-Ser-AM-MBHA-(R) |
| Fmoc-Glu-Glu-Met-Gln-Arg-Arg-Gly-AM-MBHA-(R) |
| Fmoc-Glu-Glu-Met-Glu-Arg-Arg-Ala-AM-MBHA-(R) |
| Fmoc-Asp-Glu-Met-Glu-Arg-Arg-Ala-AM-M BHA-(R) |
| Fmoc-Glu-Glu-Met-Gln-Arg-Arg-Thr-AM-MBHA-(R) |
| Fmoc-Glu-Glu-Met-Asp-Arg-Arg-Ala-AM-MBHA-(R) |
| Fmoc-Asn-Glu-Met-Gln-Arg-Arg-Ala-AM-M BHA-(R) |
| Fmoc-Glu-Glu-Met-Gln-Arg-Arg-Val-AM-MBHA-(R) |
| Fmoc-Glu-Glu-Met-Gln-Arg-Arg-AM-MBHA-(R) |
| Fmoc-Glu-Glu-Met-Gln-Arg-Lys-AM-MBHA-(R) |
| Fmoc-Glu-Glu-Met-Gln-Lys-Arg-AM-MBHA-(R) |
| Fmoc-Glu-Glu-Met-Glu-Arg-Arg-AM-MBHA-(R) |
| Fmoc-Glu-Glu-Met-Asn-Arg-Arg-AM-MBHA-(R) |
| Fmoc-Glu-Glu-Leu-Gln-Arg-Arg-AM-MBHA-(R) |
| Fmoc-Glu-Asp-Met-Gln-Arg-Arg-AM-MBHA-(R) |
| Fmoc-Glu-Gln-Met-Gln-Arg-Arg-AM-MBHA-(R) |
| Fmoc-Asp-Glu-Met-Gln-Arg-Arg-AM-MBHA-(R) |
| Fmoc-Gln-Glu-Met-Gln-Arg-Arg-AM-MBHA-(R) |
| Fmoc-Glu-Glu-Met-Gln-Arg-His-AM-MBHA-(R) |
| Fmoc-Glu-Glu-Met-Gln-Lys-Lys-AM-MBHA-(R) |
| Fmoc-Glu-Glu-Ile-Glu-Arg-Arg-AM-MBHA-(R) |

### EXAMPLE 4

### General process for removal of Fmoc N-terminal protective group.

The N-terminal Fmoc group of the peptidyl resins obtained in examples 1, 2 and 3 is deprotected as described in the general methods (20% piperidine in DMF, 1 × 1 min + 1 × 5 min). The peptidyl resins are washed with DMF (5 × 1 min), DCM (3 × 1 min), diethyl ether (3 × 1 min) and dried under vacuum.

### EXAMPLE 5

### Process for introducing the R₁ palmitoyl group onto the peptidyl resins obtained in Example 4.

180 mg (0.7 mmol; 5 equiv) or 154mg (0.6 mmol, 5equiv) of palmitic acid pre-dissolved in DMF (1 ml) is added onto 0.14 mmol or 0.12 mmol respectively of each of the peptidyl resins obtained in Example 4, in the presence of 107 mg (0.7 mmol; 5 equiv) or 92 mg (0.6mmol, 5equiv) of HOBt and 127 µl of DIPCDI (0.77 mmol; 5.5 equiv) or 102 µl (0.66 mmol, 5.5equiv) of DIPCDI. The mixture is allowed to react for 3 hours, after which the resin is washed with DMF (3 × 1 min), DCM (3 × 1 min), diethyl ether (3 × 1 min) and is dried under vacuum.

### EXAMPLE 6

### Process for introducing the R₁ myristoyl group onto the peptidyl resins obtained in Example 4.

137 mg of myristic acid (0.6 mmol; 5 equiv) pre-dissolved in DMF (1 ml) is added onto 0.12 mmol of each of the peptidyl resins obtained in Example 5, in the presence of 92 mg of HOBt (0.6 mmol; 5 equiv) and 102 µL of DIPCDI (0.66 mmol; 5.5 equiv). The mixture is allowed to react for 3 hours, after which the resin is washed with DMF (3 × 1 min), DCM (3 × 1 min), diethyl ether (3 × 1 min) and is dried under vacuum.

### EXAMPLE 7

### Process for introducing the R₁ acetyl group onto the peptidyl resins obtained in Example 4.

0.12 mmol or 0.14 mmol of the peptidyl resins obtained in Example 4 is treated with 25 equiv of acetic anhydride in the presence of 25 equiv of DIEA using 2 ml of DMF as a solvent. The mixture is allowed to react for 30 min, after which the resin is washed with DMF (3 × 1 min), DCM (3 × 1 min), diethyl ether (3 × 1 min) and is dried under vacuum.

### EXAMPLE 8

### Cleavage process from the polymeric support of the peptidyl resins obtained in Examples 4, 5, 6 and 7.

Each of the dried peptidyl resins obtained in Examples 4, 5, 6 and 7 are treated with 3 ml of TFA:H₂O (95:5, v/v) for 2 hours at room temperature under stirring. The filtrate is collected onto 20 ml of cold diethyl ether, then it is filtered through a polypropylene syringe fitted with porous polyethylene discs and washed 5 times with 10 ml diethyl ether. The final precipitate is dried under vacuum.

Peptides of general formula R₁-Wₘ-Xₙ-AA₁-AA₂-AA₃₋AA₄-AAs-AA₆-AA₇-AA₈-Yₚ-Z_{q}-OH or R₁-Wₘ-Xₙ-AA₁-AA₂-AA₃-AA₄-AA₅-AAₑ-AA₇-AA₈-Yₚ-Z_{q}-NH₂, wherein R₁ is H, acetyl, palmitoyl or myristoyl are obtained following this method.

HPLC analyses of the obtained peptides in gradients of MeCN (+0.07% TFA) in H₂O (+0.1% TFA) show a purity exceeding 80% in all cases. The identity of the peptides obtained is confirmed by ESI-MS.

### EXAMPLE 9

### Cleavage process from the polymeric support and functionalization with R₂ substituted amine: Obtaining H-Wₘ-Xₙ-AA₁-AA₂-AA₃-AA₄-AA₅-AA₆-AA₇-AA₈-Yₚ-Z_{q}-NH-(CH₂)₅-CH₃, wherein AA₁ is L-Glu; AA₂ is L-Glu; AA₃ is L-Met; AA₄ is L-Gln; AA₅ is L-Arg; AA₆ is L-Arg; AA₇ is a bond or L-Ala; AA₈ is a bond or L-Asp; and m, n, p and q are each 0.

The peptides H-Wₘ-Xₙ-AA₁-AA₂-AA₃-AA₄-AA₅-AA₆-AA₇-AA₈-Yₚ-Z_{q}-OH with fully protected side chains are obtained by treating 290 mg of each of the peptidyl resins H-Wₘ-Xₙ-AA₁-AA₂-AA₃-AA₄-AA₅-AA₆-AA₇-AA₈-Yₚ-Z_{q}-O-2-CITrt-(R) obtained in Example 4, which are previously desiccated under vacuum in the presence of KOH, with 2.03 mL AcOH for 2 hours. Liquid phase is separated through filtration. The filtrate is collected and the resin is then washed with 1 mL AcOH (1 × 1 min). All the liquid phases are combined and lyophilized.

0.05 mmol or 0.08 mmol of each of the obtained crude peptides is weighed in a flask and 3 equiv of hexylamine.HOBt and 4-5 ml of anhydrous DMF is added. 2 equiv of DIPCDI is added, and left to react under magnetic stirring at 47°C. The reaction is monitored by HPLC until disappearance of the initial products, and are complete after 2-4 hours. Solvents are evaporated to dryness and co-evaporated twice with DCM. The obtained residue is dissolved in 6 to 10 ml of a mixture of TFA:H₂O (95:5, v/v) and left to react for 2 hours at room temperature. 42 or 70 ml of cold diethyl ether are added, the solvents are evaporated under reduced pressure and two additional co-evaporations with diethyl ether are carried out. The residue is dissolved in a mixture of 50% MeCN in H₂O (v/v) and lyophilized.

HPLC analyses of the obtained peptides in gradients of MeCN (+0.07% TFA) in H2O (+0.1% TFA) show a purity exceeding 80% in all cases. The identity of the peptides obtained is confirmed by ESI-MS.

### EXAMPLE 10

### Cleavage process from the polymeric support and functionalization with R₂ substituted amine: Obtaining H-Wₘ-Xₙ-AA₁-AA₂-AA₃-AA₄-AA₅-AA₆-AA₇-AA₈-Yₚ-Z_{q}-NH-(CH₂)₁₅-CH₃, wherein AA₁ is L-Glu; AA₂ is L-Glu; AA₃ is L-Met; AA₄ is L-Gln; AA₅ is L-Arg; AA₆ is L-Arg; AA₇ is a bond or L-Ala; AA₈ is a bond or L-Asp; and m, n, p and q are each 0.

The peptides H-Wₘ-Xₙ-AA₁-AA₂-AA₃-AA₄-AA₅-AA₆-AA₇-AA₈-OH with fully protected side chains are obtained by treating 290 mg of each of the peptidyl resins H-Wₘ-Xₙ-AA₁-AA₂-AA₃-AA₄-AA₅-AA₆- AA₇-AA₈-Yₚ-Z_{q}-O-2-ClTrt-(R) of Example 4, which are previously desiccated under vacuum in the presence of KOH, with 2.03 mL AcOH for 2 hours. Liquid phase is separated through filtration. The filtrate is collected and the resin is then washed with 1 mL AcOH (1 × 1 min). All the liquid phases are combined and lyophilized.

0.04 mmol or 0.07 mmol of each of the obtained crude peptides is weighed in a flask and 3 equiv of hexadecylamine.HOBt and 3-5 ml of anhydrous DMF is added. 2 equiv of DIPCDI is added, and left to react under magnetic stirring at 47°C. The reaction is monitored by HPLC until disappearance of the initial products, and are complete after 2-4 hours. Solvents are evaporated to dryness and co-evaporated twice with DCM. The obtained residue is dissolved in 5 to 8 ml of a mixture of TFA:H₂O (95:5, v/v) and left to react for 2 hours at room temperature. 35 or 56 ml of cold diethyl ether is added, the solvents are evaporated under reduced pressure and two additional co-evaporations with diethyl ether are carried out. The residue is dissolved in a mixture of 50% MeCN in H₂O (v/v) and lyophilized.

HPLC analyses of the obtained peptides in gradients of MeCN (+0.07% TFA) in H2O (+0.1% TFA) show a purity exceeding 80% in all cases. The identity of the peptides obtained is confirmed by ESI-MS.

### EXAMPLE 11

### Preparation of a microemulsion comprising peptide Ac-Glu-Glu-Met-Gln-Arg-Arg-Ala-NH₂ (PEP-2).

In an appropriate container the peptide Ac-Glu-Glu-Met-Gln-Arg-Arg-Ala-NH₂ (PEP-2) is dissolved in water [INCI: WATER (AQUA)] (phase A1), and then a mixture of the ingredients of phase A2 (2-phenoxyethanol [INCI: PHENOXYETHANOL], Structure^{®} XL [INCI: HYDROXYPROPYL STARCH PHOSPHATE], Zemea^{™} [INCI: PROPANEDIOL], Amigel^{®} [INCI: SCLEROTIUM GUM] and sodium hyaluronate [INCI: SODIUM HYALURONATE]; see table 5c), which had been pre-mixed in a separate recipient, is introduced. The resulting mixture is heated at 70°C while stirring gently and then Cola^{®}Fax CPE-K [INCI: POTASSIUM CETYL PHOSPHATE] is added (phase A3).

In another recipient, the components of phase B: Schercemol^{™} DIS Ester [INCI: DIISOPROPYL SEBACATE] and Montanov^{™} 68 [INCI: CETEARYL ALCOHOL; CETEARYL GLUCOSIDE] are introduced, heating them at 80°C and stirring the mixture. Phase B is slowly introduced over phase A while intense stirring.

Keeping the temperature at 70-80°C, the sample is homogenized with a titanium probe for 30 seconds.

**Table 5c**

| Phase | INGREDIENT | % weight |
|---|---|---|
| A1 | WATER (AQUA) | q.s.100 |
| A1 | Ac-Glu-Glu-Met-Gln-Arg-Arg-Ala-NH₂ (PEP-2) | 0.0010 |
| A2 | PHENOXYETHANOL | 2.7382 |
| A2 | HYDROXYPROPYL STARCH PHOSPHATE | 0.6570 |
| A2 | PROPANEDIOL | 5.4764 |
| A2 | SCLEROTIUM GUM | 0.3285 |
| A2 | SODIUM HYALURONATE | 0.0109 |
| A3 | POTASSIUM CETYL PHOSPHATE | 0.5476 |
| B | DIISOPROPYL SEBACATE | 10.9500 |
| B | [CETEARYL ALCOHOL; CETEARYL GLUCOSIDE] | 4.3811 |

### EXAMPLE 12

### Preparation of a lipid nanoparticle composition comprising the microemulsion of example 11.

The microemulsion prepared in Example 11 is introduced into an appropriate recipient (phase A).

Separately, phase B (see Table 5d) is prepared by dissolving N-Hance^{®} CG-17 Cationic Guar [INCI: GUAR HYDROXYPROPYLTRIMONIUM CHLORIDE; WATER (AQUA)] in water [INCI: WATER (AQUA)]. Phase B is added to phase A under intense stirring.

Components of phase C (Structure^{®} XL [INCI: HYDROXYPROPYL STARCH PHOSPHATE] and Amigel^{®} [INCI: SCLEROTIUM GUM]) and phase D (Heliogel^{™}; [INCI: SODIUM ACRYLATES COPOLYMER; HYDROGENATED POLYISOBUTENE; LECITHIN; POLYGLYCERYL-10 STEARATE; SUNFLOWER (HELIANTHUS ANNUUS) SEED OIL; TOCOPHEROL]) are added slowly and one by one under intense stirring.

| Table 5d | | |
|---|---|---|
| Phase | INGREDIENT | % weight |
| A | Microemulsion of example 11 | q.s. 100% |
| B | [GUAR HYDROXYPROPYLTRIMONIUM CHLORIDE; WATER (AQUA)] | 0.20 |
| B | WATER (AQUA) | 6.0 |
| C | HYDROXYPROPYL STARCH PHOSPHATE | 1.50 |
| C | SCLEROTIUM GUM | 0.75 |
| D | [SODIUM ACRYLATES COPOLYMER; HYDROGENATED POLYISOBUTENE; LECITHIN; POLYGLYCERYL-10 STEARATE; SUNFLOWER (HELIANTHUS ANNUUS) SEED OIL; TOCOPHEROL] | 0.25 |

### EXAMPLE 13

### Preparation of liposomes comprising peptide Ac-Glu-Glu-Met-Gln-Arg-Arg-Ala-NH₂ (PEP-2).

In an appropriate container, phase A is prepared by dissolving peptide Ac-Glu-Glu-Met-Gln-Arg-Arg-Ala-NH₂ (PEP-2) in water [INCI: WATER (AQUA)]. Zemea^{™} [INCI: PROPANEDIOL] and 2-phenoxyethanol [INCI: PHENOXYETHANOL] (Phase B) are added to phase A.

When all the previous components are dissolved, lecithin [INCI: LECITHIN] (phase C) is added little by little and under intense stirring, until complete dispersion. The finally obtained composition is shown in Table 5e.

| Table 5e | | |
|---|---|---|
| Phase | INGREDIENT | % weight |
| A | WATER (AQUA) | q.s. 100% |
| A | Ac-Glu-Glu-Met-Gln-Arg-Arg-Ala-NH₂ (PEP-2) | 0.0010 |
| B | PROPANEDIOL | 8.5000 |
| B | PHENOXYETHANOL | 0.9050 |
| C | LECITHIN | 0.5000 |

The sample is homogenized with a titanium probe for 30 seconds.

### EXAMPLE 14

### Preparation of liposomes of example 3 bound to cationic polymers.

The liposomes obtained in example 3 are added to SENSOMER^{®} CT-50 Polymer [INCI: WATER (AQUA); STARCH HYDROXYPROPYLTRIMONIUM CHLORIDE; UREA, SODIUM LACTATE; SODIUM CHLORIDE; SODIUM BENZOATE] at a liposomes:cationic polymer ratio of 95:5 (w/w) under slow stirring.

### EXAMPLE 15

### Preparation of a cosmetic composition (cream) containing Ac-Glu-Glu-Met-Gln-Arg-Arg-Ala-NH₂ (PEP-2)

In a suitable vessel, the ingredients of phase A (water, [INCI: WATER (AQUA)], Zemea^{™} [INCI: PROPANEDIOL], and Dermosoft^{®} GMCY [INCI: GLYCERYL CAPRYLATE]) are dissolved under rotor stirring.

Next, phase A1 (Carbopol^{®} Ultrez 21 Polymer [INCI: ACRYLATES/C10-30 ALKYL ACRYLATE CROSSPOLYMER]) is added and let to wet and disperse in the mixture. Phase A2 (xanthan gum [INCI: XANTHAN GUM]) is added and let to disperse too. Finally, phase A3 (Cola^{®}Fax CPE-K [INCI: POTASSIUM CETYL PHOSPHATE]) is subsequently added. The mixture is then heated at 70-75°C.

In a separate vessel, phase B ingredients (Schercemol^{™} DIA Ester [INCI: DIISOPROPYL ADIPATE], Glucate^{™} SS Emulsifier [INCI: METHYL GLUCOSE SESQUISTEARATE; STEARIC ACID], and Glucamate^{™} SSE-20 Emulsifier [INCI: PEG-20 METHYL GLUCOSE SESQUISTEARATE]) are weighted and the mixture is heated at 70-75°C.

When both mixtures reach the corresponding temperature, the emulsion is made by adding slowly phase B on the combined phases A under stirring with turbine.

When the mixture is cooling to 40°C, phase C (pre-mixture of water [INCI: WATER (AQUA)], octane-1,2-diol [INCI: CAPRYLYL GLYCOL], and Ac-Glu-Glu-Met-Gln-Arg-Arg-Ala-NH₂ (PEP-2) is added. Finally, phase D (water [INCI: WATER (AQUA)] and sodium hydroxide [INCI: SODIUM HYDROXIDE]) is incorporated into the previous mixture for pH adjustment to 6.0-6.5.

The whole list of ingredients is shown in Table 6.

| Table 6 | | |
|---|---|---|
| INGREDIENT (INCI name) | % weight | Phase |
| WATER (AQUA) | 74.1000 | A |
| PROPANEDIOL | 10.0000 | A |
| GLYCERYL CAPRYLATE | 1.0000 | A |
| ACRYLATES/C10-30 ALKYL ACRYLATE CROSSPOLYMER | 0.3000 | A1 |
| XANTHAN GUM | 0.3000 | A2 |
| POTASSIUM CETYL PHOSPHATE | 1.0000 | A3 |
| DIISOPROPYL ADIPATE | 9.0000 | B |
| [METHYL GLUCOSE SESQUISTEARATE; STEARIC ACID] | 2.1000 | B |
| PEG-20 METHYL GLUCOSE SESQUISTEARATE | 1.2000 | B |
| WATER (AQUA) | 0.9945 | C |
| CAPRYLYL GLYCOL | 0.0050 | C |
| Ac-Glu-Glu-Met-Gln-Arg-Arg-Ala-NH₂ (PEP-2) | 0.0005 | C |
| [WATER (AQUA); SODIUM HYDROXIDE] | q.s. | D |

### EXAMPLE 16

### Preparation of a cosmetic composition (cream) containing Ac-Glu-Glu-Met-Gln-Arg-Arg-Ala-NH₂ (PEP-2)

In a suitable vessel, the ingredients of phase A (water [INCI: WATER (AQUA)], 1,2-dihydroxypentane [INCI: PENTYLENE GLYCOL], and Microcare^{®} BNA [INCI: BENZYL ALCOHOL]) are dissolved under rotor stirring.

Next, phase A1 (Massocare^{®} TCK1 [INCI: CARBOMER]) is added and let it wet and disperse in the mixture. Phase A2 (Cola^{®}Fax CPE-K [INCI: POTASSIUM CETYL PHOSPHATE]) is subsequently added and let to disperse too. The mixture is then heated at 70-75°C.

In a separate vessel, phase B ingredients (Lincol Bas [INCI: C12-15 ALKYL BENZOATE], Schercemol^{™} DIA Ester [INCI: DIISOPROPYL ADIPATE], Phytocream 2000^{®} [INCI: GLYCERYL STEARATE; CETEARYL ALCOHOL; POTASSIUM PALMITOYL HYDROLYZED WHEAT PROTEIN], 2-phenoxyethanol [INCI: PHENOXYETHANOL], Astro-Sil 2C 350 [INCI: DIMETHICONE], and vitamin E acetate [INCI: TOCOPHERYL ACETATE]) are weighted and the mixture is heated at 70-75°C.

When both mixtures reach the corresponding temperature, the emulsion is made by slowly adding phase B on the combined phases A under stirring with turbine.

When the mixture is cooling at 40°C, phase C (Novemer^{™} EC-2 Polymer [INCI: WATER (AQUA); SODIUM ACRYLATES/BEHENETH-25 METACRYLATE CROSSPOLYMER; HYDROGENATED POLYDECENE; LAURYL GLUCOSIDE] followed by phase D [a pre-mixture of water [INCI: WATER (AQUA)], octane-1,2-diol [INCI: CAPRYLYL GLYCOL], and Ac-Glu-Glu-Met-Gln-Arg-Arg-Ala-NH₂ (PEP-2) are sequentially added to it.

Last, phase E (perfume [INCI: FRAGRANCE]) is added and phase F (water [INCI: WATER (AQUA)] and sodium hydroxide [INCI: SODIUM HYDROXIDE]) is then incorporated into the previous mixture for pH adjustment to 6.0-6.5

The whole list of ingredients can be found in Table 7.

| Table 7 | | |
|---|---|---|
| INGREDIENT (INCI name) | % weight | Phase |
| WATER | 83.1200 | A |
| PENTYLENE GLYCOL | 5.0000 | A |
| BENZYL ALCOHOL | 0.4000 | A |
| CARBOMER | 0.2800 | A1 |
| POTASSIUM CETYL PHOSPHATE | 0.5000 | A2 |
| C12-15 ALKYL BENZOATE | 2.5000 | B |
| DIISOPROPYL ADIPATE | 2.0000 | B |
| [GLYCERYL STEARATE; CETEARYL ALCOHOL; POTASSIUM PALMITOYL HYDROLYZED WHEAT PROTEIN] | 2.0000 | B |
| PHENOXYETHANOL | 0.6000 | B |
| DIMETHICONE | 0.5000 | B |
| TOCOPHERYL ACETATE | 0.5000 | B |
| [WATER (AQUA); SODIUM ACRYLATES/BEHENETH-25 METACRYLATE CROSSPOLYMER; HYDROGENATED POLYDECENE; LAURYL GLUCOSIDE] | 1.5000 | C |
| WATER (AQUA) | 0.9945 | D |
| CAPRYLYL GLYCOL | 0.0050 | D |
| Ac-Glu-Glu-Met-Gln-Arq-Arq-Ala-NH₂ (PEP-2) | 0.0005 | D |
| FRAGRANCE | 0.1000 | E |
| [WATER (AQUA); SODIUM HYDROXIDE] | q.s. | F |

### EXAMPLE 17

### Preparation of a placebo cosmetic composition (cream)

In a suitable vessel, the ingredients of phase A (water [INCI: WATER (AQUA)], 1,2-dihydroxypentane [INCI: PENTYLENE GLYCOL], and Microcare^{®} BNA [INCI: BENZYL ALCOHOL]) are dissolved under rotor stirring.

Next, phase A1 (Massocare^{®} TCK1 [INCI: CARBOMER]) is added and let to wet and disperse in the resulting mixture. Phase A2 (Cola^{®}Fax CPE-K [INCI: POTASSIUM CETYL PHOSPHATE]) is subsequently added and let to disperse too. The mixture is then heated at 70-75°C.

In a separate vessel, phase B ingredients (Lincol Bas [INCI: C12-15 ALKYL BENZOATE], Schercemol^{™} DIA Ester [INCI: DIISOPROPYL ADIPATE], Phytocream 2000^{®} [INCI: GLYCERYL STEARATE; CETEARYL ALCOHOL; POTASSIUM PALMITOYL HYDROLYZED WHEAT PROTEIN], 2-phenoxyethanol [INCI: PHENOXYETHANOL], Astro-Sil 2C 350 [INCI: DIMETHICONE], and vitamin E acetate [INCI: TOCOPHERYL ACETATE]) are weighted and the mixture is heated at 70-75°C.

When both mixtures reach the corresponding temperature, the emulsion is made by slowly adding phase B on the mixture of phases A under stirring with turbine.

When the mixture is cooling at 40°C, phase C (Novemer^{™} EC-2 Polymer [INCI: WATER (AQUA); SODIUM ACRYLATES/BEHENETH-25 METACRYLATE CROSSPOLYMER; HYDROGENATED POLYDECENE; LAURYL GLUCOSIDE] is added to it.

Last, phase D (perfume [INCI: FRAGRANCE]) is added and phase E (water [INCI: WATER (AQUA)] and sodium hydroxide [INCI: SODIUM HYDROXIDE] is then incorporated into the previous mixture for pH adjustment to 6.0-6.5.

The whole list of ingredients is shown in Table 8.

| Table 8 | | |
|---|---|---|
| INGREDIENT (INCI name) | % weight | Phase |
| WATER (AQUA) | 84.12 | A |
| PENTYLENE GLYCOL | 5.00 | A |
| BENZYL ALCOHOL | 0.40 | A |
| CARBOMER | 0.28 | A1 |
| POTASSIUM CETYL PHOSPHATE | 0.50 | A2 |
| C12-15 ALKYL BENZOATE | 2.50 | B |
| DIISOPROPYL ADIPATE | 2.00 | B |
| [GLYCERYL STEARATE; CETEARYL ALCOHOL; POTASSIUM PALMITOYL HYDROLYZED WHEAT PROTEIN] | 2.00 | B |
| PHENOXYETHANOL | 0.60 | B |
| DIMETHICONE | 0.50 | B |
| TOCOPHERYL ACETATE | 0.50 | B |
| [WATER (AQUA); SODIUM ACRYLATES/BEHENETH-25 METACRYLATE CROSSPOLYMER; HYDROGENATED POLYDECENE; LAURYL GLUCOSIDE] | 1.50 | C |
| FRAGRANCE | 0.10 | D |
| WATER (AQUA) & SODIUM HYDROXIDE | q.s. | E |

### EXAMPLE 18

### Study of the profile of the gene expression of primary human keratinocyte cells adult (HEKa).

The number of times that sets of genes significantly increase/decrease is studied, within the gene profile of primary human keratinocyte cells adult (HEKa), by treatment with 50 µg/ml of peptide Ac-Glu-Glu-Met-Gln-Arg-Arg-NH₂ (PEP-1), 100 µg/ml of peptide Ac-Glu-Glu-Met-Gln-Arg-Arg-Ala-NH₂ (PEP-2) and 50 µg/ml of peptide Ac-Glu-Glu-Met-Gln-Arg-Arg-Ala-Asp-NH₂ (PEP-3) compared to the basal levels in untreated cells grown in culture medium (basal conditions).

HEKa cells (Life Technologies) are trypsinized and then seeded at a density of 7.5×10⁴ to 10×10⁴ cells/flask on 25 cm² flasks and incubated in culture medium Epilife with Defined Growth Supplement (EDGS, Life Technologies). Cells are grown at 37°C, 5% CO2 during one week until confluence. Then, treatment with 100 µg/ml of peptide Ac-Glu-Glu-Met-Gln-Arg-Arg-NH₂ (PEP-1), 50 µg/ml of peptide Ac-Glu-Glu-Met-Gln-Arg-Arg-Ala-NH₂ (PEP-2) or 50 µg/ml of peptide Ac-Glu-Glu-Met-Gln-Arg-Arg-Ala-Asp-NH₂ (PEP-3) is carried out for 16-24 hours at 37°C, 5% CO2. Non-treated cells incubated in culture medium alone are used as basal conditions. Each condition is tested at least in 4 flasks.

After the last incubation, cells are lysed directly in the wells, and RNA is extracted and purified from each replica and each condition by means of the RNeasyPlus Mini kit (Qiagen) following the manufacturer's protocol. Briefly, the lysed cells are homogenized and the RNases are inactivated. The genomic DNA is removed from the samples by using gDNA Eliminator spin columns (Qiagen). Then, the samples are passed through special RNA binding columns (Qiagen) and after several microcentrifugation washes to eliminate contaminants and impurities, the purified RNA is eluted with 50 µl of ultrapure water.

The purity, integrity and concentration of the RNA obtained are evaluated by means of spectrophotometry (Nanodrop) and with a bioanalyzer (Agilent Bioanalyzer). Four control samples and four treated samples are selected according to the results of purity and integrity. Later, the labelling is carried out and the samples are hybridized in a human gene expression microarray (ASurePrint G3, Agilent).

The normalized values obtained with the treatment are compared with the normalized values obtained with the basal conditions to identify genes with differential expression. Next, a parametric analysis of the data is carried out by means of the Bioconductor software. The values obtained are then evaluated by means of GSEA (Gene Set Analysis Enrichment) to group together the genes with differential expression. Gene Set Analysis Enrichment GSEA was performed taking normalized data obtained with Bioconductor from treatments compared two by two and functionally analyzed with Gene Ontology (GO) and the pathway databases Reactome, KEGG and Biocarta.

Most significant pathways regarding their False Discovery Rate (FDR) are obtained. The results of FRD obtained for selected up regulated pathways respect to basal conditions are shown in table 9.

**Table 9: Selected significantly upregulated pathways by application of treatment based in peptides of the invention**

| | FDR | | |
|---|---|---|---|
| | PEP-1* | PEP-2 | PEP-3 |
| **Immune System** | | | |
| REACTOME_INTERFERON_ALPHA_BETA_SIGNALING | 0.00 | 0.54 | 0.71 |
| REACTOME_CYTOKINE_SIGNALING_IN_IMMUNE_ SYSTEM | 0.19 | 0.25 | 0.45 |
| REACTOME_NOD1_2_SIGNALING_PATHWAY | 0.24 | 0.41 | 0.68 |
| KEGG_TOLL_LIKE_RECEPTOR_SIGNALING_PATHWAY | 0.23 | 0.71 | 0.33 |
| DEFENSE_RESPONSE | 0.11 | 0.35 | 0.09 |

| **Cell Junctions** | | | |
|---|---|---|---|
| REACTOME_TIGHT_JUNCTION_INTERACTIONS | 0.23 | 0.31 | 0.10 |
| INTERCELLULAR_JUNCTION | 0.23 | 0.14 | 0.29 |

| **Lipids** | | | |
|---|---|---|---|
| LIPID_METABOLIC_PROCESS | 0.23 | 0.31 | 0.21 |
| CELLULAR_LIPID_METABOLlC_PROCESS | 0.37 | 0.90 | 0.11 |

| | | | |
|---|---|---|---|
| *not of the invention | | | |

The results shown in Table 9 demonstrate that the peptides of the invention are able to upregulate molecular pathways related to barrier function in primary human keratinocyte cells at the tested concentrations.

### EXAMPLE 19

### Treatment effect on the lipidomic profiles of in vitro epidermal model samples.

The stratum corneum (SC) is the outermost layer of skin that functions as a barrier and protects against environmental influences and transepidermal water loss. Its unique morphology consists of keratin-enriched corneocytes embedded in a distinctive mixture of lipids containing mainly ceramides (>50% by weight), free fatty acids, and cholesterol (between 15 and 20 wt.%) as well as minor fractions of triacylglycerols and phospholipids *[*Shin J, et al. A lipidomic platform establishment for structural identification of skin ceramides with non-hydroxyacyl chains. Anal Bioanal Chem (2014) 406:1917-1932*].*

Ceramides are sphingolipids consisting of sphingoid bases, which are linked to fatty acids by an amide bond. Ceramides are found in all tissues of the human body, but greatest diversity is found in the skin or, more precisely, in the uppermost layer of the epidermis, the stratum corneum. The general aim of the study is to evaluate potential metabolic differences between control and treated sample. Endogenous lipidomic profiles of *in vitro* epidermal model samples are studied using ultra performance liquid chromatography- mass spectrometry (UPLC^{®}-MS). The effect of treatment on these skin-like samples is evaluated and compared to control.

The SkinEthic human tissue models (EpiSkin) are removed from their agarose-nutrient solution in the multiwell plate immediately after arrival and are placed in a 6-well plate in which each well has previously been filled with SkinEthic Growth Medium (EpiSkin). After overnight incubation at 37°C, 5% CO₂, Reconstructed Human Epidermis (RHE) are treated with 50 µg/ml of peptides Ac-Glu-Glu-Met-Gln-Arg-Arg-NH₂ (PEP-1), Ac-Glu-Glu-Met-Gln-Arg-Arg-Ala-NH₂ (PEP-2) or Ac-Glu-Glu-Met-Gln-Arg-Arg-Ala-Asp-NH₂ (PEP-3) or medium (basal conditions) for 24 hours at 37°C and 5% CO₂. After the 24 hours treatment, RHE are frozen and stored at -20°C. Each treatment is applied to five RHE per condition.

Epidermal membrane model samples are thawed and removed from the inserts using a scalpel and transferred to homogenization tubes. Samples are mixed with sodium chloride (50 mM) and chloroform/methanol (2/1) (v:v) and homogenized. The extraction solvent is spiked with metabolites not detected in unspiked human serum extracts. After homogenizing the samples, extracts are incubated for 1 hour at -20 °C and centrifuged at 18,000 × g for 15 minutes. The organic phase is then collected and dried under vacuum. Dried extracts are finally reconstituted in acetonitrile/isopropanol (1/1) (v:v), centrifuged at 18,000 × g for 5 minutes and transferred to vials for UPLC^{®}-MS analysis.

Chromatographic separation and mass spectrometric detection conditions employed are summarized in Table 10.

| Table 10 | |
|---|---|
| Column type | UPLC BEH C18, 2.1 x 100 mm, 1.7 µm |
| Flow rate | 0.40 ml/min |
| Solvent A | H2O + ACN* + 10mM ammonium formate |
| Solvent B | ACN* + Isopropanol + 10mM ammonium formate |
| (%B), time | 40%, 0 minutes |
| (%B), time | 100%, 10 minutes |
| (%B), time | 40%, 15 minutes |
| (%B), time | 40%, 17 minutes |
| Column temperature | 60 °C |
| Injection volume | 3 µl |
| Source temperature | 120 °C |
| Nebulisation N2 flow | 1000 l / hour |
| Nebulisation N2 temperature | 500 °C |
| Cone N2 flow | 30 l / hour |
| Capillary voltage | 3.2 kV |
| Cone voltage | 30 V |

| | |
|---|---|
| * ACN = Acetonitrile. | |

All data are processed using the TargetLynx application manager for MassLynx 4.1 software (Waters Corp., Milford, USA). A set of predefined retention time, mass-to-charge ratio pairs, *Rt-m*/*z,* corresponding to metabolites included in the analysis are fed into the program. Associated extracted ion chromatograms (mass tolerance window = 0.05 Da) are then peak-detected and noise-reduced in both the LC and MS domains such that only true metabolite related features are processed by the software. A list of chromatographic peak areas is then generated for each sample injection and value respect to basal conditions is calculated.

Average of five values obtained for each peptide in relation to those obtained for basal conditions (without peptide in the medium) are shown in Table 11.

| Table 11 | | | | |
|---|---|---|---|---|
| Symbol | Name | PEP-1* (fold chance) | PEP-2 (fold change) | PEP-3 (fold change) |
| Cer_NdS47 | N-acyl dihydrosphingosines | 1.14 | 1.10 | 1.20 |
| Cer_NdS48 | N-acyl dihydrosphingosines | 1.21 | 1.23 | 1.34 |
| Cer_NdS49 | N-acyl dihydrosphingosines | 1.38 | 1.46 | 1.43 |
| Cer_NdS50 | N-acyl dihydrosphingosines | 1.42 | 1.50 | 1.68 |
| Cer_NP48 | N-acyl phytosphingosines | 1.21 | 1.24 | 1.19 |
| Cer_NP49 | N-acyl phytosphingosines | 1.17 | 1.23 | 1.14 |
| Cer_NP50 | N-acyl phytosphingosines | 1.18 | 1.23 | 1.30 |
| Cer_NP51 | N-acyl phytosphingosines | 1.44 | 1.19 | 1.24 |
| Cer_NS50 | N-acyl sphingosines | 1.20 | 1.18 | 1.14 |
| Cer_NS51 | N-acyl sphingosines | 1.14 | 1.13 | 1.10 |
| Cer_NS52 | N-acyl sphingosines | 1.35 | 1.37 | 1.30 |
| Cer_AP48 | N-(alpha-hydroxyacyl) phytosphingosines | 1.21 | 1.37 | 1.15 |

| | | | | |
|---|---|---|---|---|
| *not of the invention | | | | |

The results show that peptides of the invention are able to strengthen the barrier function of the stratum corneum by increasing the amount of different groups of ceramides (Cer_NdS, Cer_NS, Cer_NP and Cer_AP) with respect to basal conditions in Reconstructed Human Epidermis at the tested concentration.

### EXAMPLE 20

### Study of tight junctions (TJs) expression profile in human epidermal keratinocytes (HEKa) using Real Time PCR Arrays.

TJs are structures in the stratum granulosum of the epidermis that serve as a physical barrier to prevent solutes and water passing through the paracellular space, by forming cell-cell junctions that are composed of claudins, occludins, and scaffolding proteins. The incorporation and association of occludin and claudins in tight junctional strands require local clustering of these scaffolding proteins. An important group of tight junctional scaffolding molecules are the zonula occludens proteins ZO-1, ZO-2, and ZO-3.

ZO proteins also known as tight junction protein (TJP), can interact directly with occludin and claudins via their PDZ domains, whereas their C-terminus can associate with actin, thus providing a direct link with the cytoskeleton. Recently, it was shown that either ZO-1 (also identified as TJP1) or ZO-2, but not ZO-3, is crucial for clustering of claudins, strand formation, and barrier function *[*Niessen C M, "Tight Junctions/Adherens Junctions: Basic Structure and Function", Journal of Investigative Dermatology (2007) 127, 2525-2532].

The aim of this study is to investigate the ability of the peptides of the invention of enhancing the skin barrier function by increasing TJs gene expression in primary human epidermal keratinocytes isolated from adult skin (HEKa).

HEKa cells (Life Technologies) are seeded at 6.0 × 10⁵ cells/well in 6-well plates and incubated in culture medium Epilife with defined Growth Supplement (EDGS, Life Technologies) at 37°C in a water-saturated atmosphere of 95% air and 5% CO₂. After 24 hours of incubation under these conditions, medium is removed and cells are incubated with 50 µg/ml of a peptide of the invention (see Table 12) in fresh culture medium, except for two wells where HEKa cells are incubated with medium only (basal conditions), at 37°C and 5% CO₂.

After 24 hours of incubation, the cells are lysed directly in the wells following the protocol described on the RNeasy Mini kit (Qiagen) according to the manufacturer's protocol. The lysed cells are homogenized and the Rnases are inactivated. The genomic DNA is removed from the samples by using gDNA Eliminator spin columns (Qiagen). Then the samples are passed through special RNA binding columns (Qiagen) and, after several microcentrifugation washes to eliminate contaminants and impurities, the purified RNA is eluted with 50 µl of ultrapure water. Quantification and analysis of purity of RNA samples are performed after RNA elution with a biophotometer (Eppendorf). 1 µg of high quality RNA is retrotranscribed with iScript advanced (BioRad) in a final volume of 20 µl. Complete reaction mix is incubated in a thermal cycler (Eppendorf) at 42°C for 30 minutes, and the reactions are then stopped at 85°C for 5 minutes. Complementary DNA is amplified by qPCR in a real-time PCR thermocycler (BioRad) using SYBRgreen supermix (BioRad) and a customized plate for use with SYBR^{®} Green (BioRad). SYBR Green binds to double-stranded DNA molecules and emits fluorescence which is quantified and is proportional to the amount of the product in the PCR reaction. Cycling conditions in BioRad CFX96 instrument are 95°C for 3 minutes, followed by 40 cycles of denaturing at 95°C for 5 seconds, annealing and elongation at 60°C for 30 seconds. GAPDH (Glyceraldehyde 3-phosphate dehydrogenase), TBP (TATA box binding protein) and HRPT1 (hypoxanthine phosphoribosyltransferase 1) are used as endogenous controls. Fold change relative to the expression of the sample genes and reference genes is calculated using normalized expression (ΔΔ(Ct)) method with default threshold values using CFX Manager Software (BioRad).

Fold induction of tight junctions genes Claudin 1 (CLDN1) and Tight Junction Protein 1 (TJP1) respect to basal conditions are shown in Table 12.

| Table 12 | | |
|---|---|---|
| **Peptides** | **CLDN1** | **TJP1** |
| Ac-Glu-Glu-Met-Gln-Arg-Arg-NH₂ * | 2.44 | 2.97 |
| Ac-Glu-Glu-Met-Gln-Arg-Arg-Ala-NH₂ | 2.38 | 2.56 |
| Ac-Glu-Glu-Met-Gln-Arg-Arg-Ala-Asp-NH₂ | 1.40 | 1.83 |
| Ac-Glu-Glu-Met-Gln-Arg-Arg-Ala-Asp-OH | 2.66 | 3.16 |
| Myr-Glu-Glu-Met-Gln-Arg-Arg-NH₂ * | 2.84 | 3.14 |
| Palm-Glu-Glu-Met-Gln-Arg-Arg-NH₂ * | 2.43 | 2.73 |
| Ac-Glu-Glu-Met-Gln-Arg-Arg-NHC₁₆H₃₃ * | 1.86 | 2.78 |
| Ac-Asp-Glu-Met-Gln-Arg-Arg-Ala-Asp-NH₂ | 1.76 | 1.99 |
| Ac-Gln-Glu-Met-Gln-Arg-Arg-Ala-Asp-NH₂ | 2.48 | 2.78 |
| Ac-Glu-Glu-Leu-Gln-Arg-Arg-Ala-Asp-NH₂ | 2.24 | 2.57 |
| Ac-Glu-Glu-Met-Glu-Arg-Arg-Ala-Asp-NH₂ | 2.52 | 2.58 |
| Ac-Glu-Glu-Met-Asn-Arg-Arg-Ala-Asp-NH₂ | 2.63 | 2.75 |
| Ac-Glu-Glu-Met-Asp-Arg-Arg-Ala-Asp-NH₂ | 2.63 | 2.70 |
| Ac-Glu-Glu-Met-Gln-Arg-Lys-Ala-Asp-NH₂ | 2.59 | 2.77 |
| Ac-Glu-Glu-Met-Gln-Arg-Arg-Ser-Asp-NH₂ | 2.94 | 2.85 |
| Ac-Glu-Glu-Met-Gln-Arg-Arg-Gly-Asp-NH₂ | 2.07 | 2.61 |
| Ac-Glu-Glu-Met-Gln-Arg-Arg-Ala-Glu-NH₂ | 2.08 | 2.35 |
| Ac-Glu-Glu-Met-Gln-Arg-Arg-Ala-Asn-NH₂ | 2.66 | 2.87 |
| Ac-Glu-Glu-Met-Gln-Arg-Arg-Ala-Gln-NH₂ | 2.03 | 2.56 |
| Ac-Glu-Glu-Met-Gln-Arg-His-Ala-Asp-NH₂ | 2.53 | 2.46 |
| Ac-Glu-Glu-Met-Gln-Arg-Arg-Thr-Asp-NH₂ | 2.30 | 2.66 |
| Ac-Glu-Glu-Met-Gln-Arg-Arg-Pro-Asp-NH₂ | 2.48 | 2.63 |
| Ac-Glu-Glu-Met-Gln-Arg-Arg-Glu-Asp-NH₂ | 2.58 | 2.59 |
| Ac-Glu-Glu-Met-Gln-Arg-Arg-Lys-Asp-NH₂ | 2.80 | 2.57 |
| H-Glu-Glu-Met-Gln-Arg-Arg-Ala-Asn-NH₂ | 2.32 | 2.44 |
| H-Glu-Glu-Met-Gln-Arg-Arg-Ala-Glu-NH₂ | 2.70 | 2.98 |
| Palm-Glu-Glu-Met-Gln-Arg-Arg-Ala-Asp-NH₂ | 4.72 | 2.43 |
| Palm-Glu-Glu-Met-Gln-Arg-Arg-Ala-Asn-NH₂ | 2.17 | 2.55 |
| Myr-Glu-Glu-Met-Gln-Arg-Arg-Ala-Glu-NH₂ | 2.77 | 2.88 |
| Ac-Glu-Glu-Met-Gln-Arg-Arg-Ala-Glu-OH | 2.92 | 2.92 |
| Ac-Glu-Glu-Met-Glu-Arg-Arg-Ala-Asp-OH | 2.20 | 2.26 |
| Ac-Gln-Glu-Met-Gln-Arg-Arg-Ala-Asp-OH | 2.67 | 2.65 |
| Palm-Glu-Glu-Met-Gln-Arg-Arg-Ala-Asn-OH | 4.29 | 2.77 |
| Ac-Glu-Glu-Met-Gln-Arg-Arg-Ala-Asp-NHC₆H₁₃ | 2.39 | 2.71 |
| Ac-Glu-Glu-Met-Gln-Arg-Arg-Ala-Asp-NHC₁₆H₃₃ | 4.98 | 2.58 |
| H-Glu-Glu-Met-Gln-Arg-Arg-Ala-Asp-NHC₆H₁₃ | 2.97 | 2.88 |
| Ac-Glu-Glu-Met-Gln-Arg-Arg-Ser-NH₂ | 3.11 | 2.96 |
| Ac-Glu-Glu-Met-Gln-Arg-Arg-Gly-NH₂ | 2.78 | 2.56 |
| Ac-Glu-Glu-Met-Glu-Arg-Arg-Ala-NH₂ | 2.67 | 2.80 |
| Ac-Asp-Glu-Met-Glu-Arg-Arg-Ala-NH₂ | 4.25 | 3.15 |
| Ac-Glu-Glu-Met-Gln-Arg-Arg-Thr-NH₂ | 4.00 | 2.88 |
| Ac-Glu-Glu-Met-Asp-Arg-Arg-Ala-NH₂ | 2.89 | 2.93 |
| Ac-Asn-Glu-Met-Gln-Arg-Arg-Ala-NH₂ | 2.85 | 2.71 |
| Ac-Glu-Glu-Met-Gln-Arg-Arg-Val-NH₂ | 1.75 | 1.28 |
| Ac-Glu-Glu-Met-Gln-Arg-Lys-NH₂ * | 2.42 | 2.58 |
| Ac-Glu-Glu-Met-Gln-Lys-Arg-NH₂ * | 1.99 | 1.99 |
| Ac-Glu-Glu-Met-Glu-Arg-Arg-NH₂ * | 2.04 | 1.78 |
| Ac-Glu-Glu-Met-Asn-Arg-Arg-NH₂ * | 1.85 | 1.69 |
| Ac-Glu-Asp-Met-Gln-Arg-Arg-NH₂ * | 1.22 | 1.21 |
| Ac-Glu-Gln-Met-Gln-Arg-Arg-NH₂ * | 2.03 | 2.26 |
| Ac-Asp-Glu-Met-Gln-Arg-Arg-NH₂ * | 2.02 | 2.14 |
| Ac-Gln-Glu-Met-Gln-Arg-Arg-NH₂ * | 2.01 | 2.56 |
| Ac-Glu-Glu-Met-Gln-Arg-His-NH₂ * | 2.46 | 3.07 |
| Ac-Glu-Glu-Met-Gln-Lys-Lys-NH₂ * | 2.09 | 2.06 |
| Ac-Glu-Glu-Ile-Glu-Arg-Arg-NH₂ * | 3.17 | 3.16 |
| H-Glu-Glu-Met-Gln-Arg-Arg-NH₂ * | 2.01 | 2.05 |
| H-Glu-Glu-Met-Gln-Arg-His-NH₂ * | 2.09 | 1.67 |
| Palm-Glu-Glu-Met-Gln-Arg-His-NH₂ * | 3.03 | 2.53 |
| Palm-Glu-Glu-Met-Gln-Arg-Lys-NH₂ * | 2.62 | 2.49 |
| Myr-Glu-Glu-Met-Gln-Arg-Lys-NH₂ * | 1.42 | 1.22 |
| Ac-Glu-Glu-Met-Gln-Arg-Arg-OH * | 1.67 | 1.71 |
| Ac-Glu-Glu-Met-Gln-Arg-Lys-OH * | 1.44 | 1.41 |
| Ac-Glu-Glu-Leu-Gln-Arg-Arg-OH * | 1.71 | 1.60 |
| Ac-Glu-Glu-Met-Gln-Arg-Arg-NHC₆H₁₃ * | 1.87 | 1.53 |
| H-Glu-Glu-Met-Gln-Arg-Arg-NCH₆H₁₃ * | 2.73 | 2.50 |

| | | |
|---|---|---|
| *not of the invention | | |

The results show that the peptides of the invention are able to increase the expression level of tight junctions genes, respect to basal conditions in primary human epidermal keratinocytes and at the tested concentration.

### EXAMPLE 21

### Assessing intercellular permeability barrier of tight junctions (TJs) in reconstructed human epidermal tissue (RHE) using dye penetration assay.

The functionality of the tight junction (TJ) barrier can be tested using tracer molecule of a certain size that cannot pass a properly functioning TJ barrier. Once incubated into the dermis, biotin reagents will diffuse through the intercellular space but will not pass a functional TJ barrier in the granular layer. Thus, diffusion of biotin above the stratum granulosum indicates a disrupted TJ barrier. *[*Schmitz A, Lazic' E, Koumaki D, Kuonen F, Verykiou S, and Rübsam M, "Assessing the In Vivo Epidermal Barrier in Mice: Dye Penetration Assays", Journal of Investigative Dermatology, (2014), 13*].*

TJ permeability assay using surface biotinylation technique was performed in incubated reconstructed human epidermis (RHE) with EZ-LIN Sulfo-NHS-LC-LC-Biotin from the dermal side. The aim of this study is to investigate the ability of peptides of the invention to enhance the barrier function by inhibiting diffusion of the dye through stratum granulosum in presence of sodium dodecyl sulfate (SDS) which causes functional deterioration of TJs.

SkinEthic human tissue models (EpiSkin) are removed from their agarose-nutrient solution in a multiwell plate immediately after arrival and are placed in a 6-well plate in which each well has previously been filled with SkinEthic Growth Medium (EpiSkin). After overnight incubation at 37°C, 5% CO₂, tissue models are treated with 50 µg/ml of peptide Ac-Glu-Glu-Met-Gln-Arg-Arg-Ala-NH₂ (PEP-2) and a 0.02 mg/ml sodium dodecyl sulfate (SDS, Sigma) solution in growth medium, (1 ml/well). Growth medium without peptide (1 ml/well) is used as basal condition, and a 0.02 mg/ml SDS solution in growth medium (1 ml/well) is used as a positive control. All samples are incubated at 37°C and 5% CO₂ for 24hours. All treatments are applied from dermal side.

After the 24-hours treatment, tissue models are incubated with 2 mg/ml EZ-LIN Sulfo-NHS-LC-LC-Biotin (ThermoFischer) in phosphate-buffered saline (PBS, Sigma) from the dermal side. After a 30 minutes incubation, the RHE samples are removed and embedded in tissue freezing medium (Leica). Frozen tissue sections of about 10 µm thickness are cut using a cryomicrotome (Leica, CM1950), collected on coated slides and finally stored at -20°C.

Defrosted slides are fixed in 96% ethanol for 30 minutes at 4°C in a humidified chamber and then placed in frozen solution of acetone for 1 minute at room temperature and then are rinsed with PBS. The slides are soaked in 1% (w/v) bovine serum albumin (BSA, Sigma) for 15 minutes and then are incubated with 5 µg/ml streptavidin Alexa Fluor 488 conjugated (ThermoFischer) in a humidified chamber at room temperature for 1hour. After rinsing with PBS, the slides are mounted in prolong Gold antifade reagent with 4',6-diamino-2-fenilindol (Dapi, Invitrogen).

Microscopical observations are performed with a Zeiss fluorescence microscopy and photographs of each condition are taken with the associated camera. Fluorescence images of biotin are quantified as the product of its area and the intensity mean value of channel 'EGFP' Green (excitation: 488 nm and emission: 509 nm) and normalized by basal conditions. At least 12 representative images from each condition are collected and analyzed with the ZEN software (Zeiss).

Value obtained respect to basal conditions as the mean of 3 independent immunohistochemistry experiments is represented in Table 13.

| Table 13 | | |
|---|---|---|
| Treatment | Peptide concentration | Fold change respect to basal condition |
| Basal conditions | - | 1 |
| SDS 0.02 mg/ml (Positive control) | - | 1.90 |

The results show that peptide Ac-Glu-Glu-Met-Gln-Arg-Arg-Ala-NH₂ in SDS is able to strengthen the tight junction (TJ) barrier at the tested concentration. This can be concluded from the observed inhibition in the penetration of biotin to stratum corneum in reconstructed human epidermis when compared with the basal conditions.

### EXAMPLE 22

### Assessing stratum corneum integrity in reconstructed human epidermis (RHE) using toluidine blue diffusion assay.

The skin barrier function is mainly conferred by the top layer of the epidermis, the stratum corneum (SC). The SC barrier function mainly depends on the SC structure at the tissue level, its composition, and the organization of intercellular lipidic cement at the molecular levels. The SC barrier can be assessed by applying a dye from the outside and measuring its diffusion through the SC. One of the most commonly used dyes is toluidine blue, which has been used in investigation of skin barrier dysfunction *[*Schmitz A, Lazic' E, Koumaki D, Kuonen F, Verykiou S, and Rübsam M, "Assessing the In Vivo Epidermal Barrier in Mice: Dye Penetration Assays", Journal of Investigative Dermatology, (2014), 13*].* The aim of this study is to investigate the ability of peptides of the invention to enhance the SC barrier function in reconstructed human epidermis (RHE) by decreasing diffusion of the dye through stratum corneum in presence of sodium dodecyl sulfate (SDS), which is known to cause functional deterioration of the SC.

The SkinEthic human tissue models (EpiSkin) are removed from their agarose-nutrient solution in the multiwell plate immediately after arrival and are placed in a 6-well plate in which each well has previously been filled with SkinEthic Growth Medium (EpiSkin). After overnight incubation at 37°C, 5% CO₂, the top layer of tissue model is treated with 100 µl of a 50 µg/ml solution of peptide Ac-Glu-Glu-Met-Gln-Arg-Arg-Ala-NH₂ (PEP-2) in a 0.02 mg/ml sodium dodecyl sulfate (SDS, Sigma) solution in growth medium and dermal side of tissue model is treated with 1 ml of a 50 µg/ml solution of the same peptide in growth medium. The top layer and dermal side of a tissue model is treated with growth medium as basal condition. In another tissue model, its top layer is treated with a 0.02 mg/ml SDS solution in growth medium while its dermal side is treated with growth medium alone, both in absence of peptide, for its use as a positive control. All incubations are performed at 37°C and 5% CO₂ for 24h.

After the 24-hours treatment, the top layer of tissue models are further treated with 200 µl of methanol for 2 min at room temperature and, after that, they are rinsed twice with 200 µl of phosphate-buffered saline (PBS, Sigma). Then, the top layer of tissue models are incubated with 200 µl of 0.1 % toluidine blue (Sigma) solution in PBS (Sigma) for 10 min at room temperature. At the end of the incubation, tissue models are washed four times with 200 µl of PBS and, after that, the RHE samples are removed and embedded in tissue freezing medium (Leica). Frozen tissue sections of about 10 µm thickness are cut using a cryomicrotome (Leica, CM1950), collected on coated slides and finally stored at -20°C. Frozen tissue sections are thawed at room temperature for 5 min and then sections are observed using a Zeiss optical microscopy and images are captured using Zen software. At least 12 representative images from each condition are collected and scored according to the ranking described in Table 14.

| Table 14 | |
|---|---|
| Toluidine Blue staining | |
| 4 | Strong |
| 3 | Clear |
| 2 | Moderate |
| 1 | Slight |

The scores obtained for each condition are added and then divided by the total number of images. The obtained result is shown in Table 15.

**Table 15**

| | Peptide concentration | Total score |
|---|---|---|
| Basal conditions | - | 2 |
| SDS 0.02 mg/ml (Positive control) | - | 4 |
| Ac-Glu-Glu-Met-Gln-Arg-Arg-Ala-NH₂ + 0.02 mq/ml sodium dodecyl sulfate (SDS) | 50 µg/ml | 2 |

The results show that the peptide Ac-Glu-Glu-Met-Gln-Arg-Arg-Ala-NH₂ (PEP-2), at the tested concentration in SDS, is able to strengthen stratum corneum barrier as shown by the decrease in the penetration of toluidine blue through stratum corneum, compared with the basal conditions in reconstructed human epidermis.

### EXAMPLE 23

### Study of innate and adaptive immune responses expression profile in human epidermal keratinocytes (HEKa) using Real Time PCR Arrays.

The epidermis has developed physical and immunological barriers that prevent infiltration of deleterious chemicals and pathogens. Keratinocytes, comprising the epidermis of the skin, normally use the defensive barrier to effectively prevent pathogen entry or long-term survival on the skin while permitting survival of the diverse microbial ecosystem of the skin microbiome. The mechanisms responsible for selective detection and response to the microbial ecosystem of the skin involve elements such as the toll-like receptors (TLRs) and its adaptor MyD88 to enable recognition of microbial invasion. In addition, chemokines (CCL) and also cytokines are released by TLR-triggered keratinocytes as defense against different pathogens. CCL5 is one of the aforementioned chemokines CCL.

The aim of this study is to investigate the ability of the peptides of the invention to enhance those defense mechanisms by measuring the modulation of the expression of genes involved in innate and adaptive immune responses in primary human epidermal keratinocytes isolated from adult skin (HEKa) using RT² PCR array. This array includes genes related to the IL-1R and toll-like receptor (TLR) signaling pathways including IL-1R and TLR genes involved in the detection of pathogens.

HEKa cells (Life Technologies) are seeded at 6.0 × 10⁵ cells/well in 6-well plates and incubated in culture medium Epilife with defined Growth Supplement (EDGS, Life Technologies), at 37°C in a water-saturated atmosphere of 95% air and 5% CO₂. After 24hours of incubation under these conditions, medium is removed and cells are incubated with 50 µg/ml (w/v) of a peptide of the invention (see Table 16) in fresh culture medium keeping two wells where HEKa cells are incubated with medium only (basal conditions) at 37°C and 5% CO₂.

After 24h of incubation, the cells are lysed directly in the wells following the protocol described on the RNeasy Mini kit (Qiagen) according to the manufacturer's protocol. The lysed cells are homogenized and the RNases are inactivated. The genomic DNA is removed from the samples by using gDNA Eliminator spin columns (Qiagen). Then the samples are passed through special RNA binding columns (Qiagen) and, after several microcentrifugation washes to eliminate contaminants and impurities, the purified RNA is eluted with 50 µl of ultrapure water. Quantification and analysis of purity of RNA samples are performed after RNA elution with a biophotometer (Eppendorf). 1 µg of high quality RNA is retrotranscribed with iScript advanced (BioRad) in a final volume of 20 µl. Complete reaction mix is incubated in a thermal cycler (Eppendorf) at 42°C for 30 minutes, and the reactions are stopped at 85°C for 5 minutes. Complementary DNA is amplified by qPCR in a real-time PCR thermocycler (BioRad) using SYBRgreen supermix (BioRad) and a customized plate for use with SYBR^{®} Green (BioRad). SYBR Green binds to double-stranded DNA molecules and emits fluorescence which is quantified and is proportional to the amount of the product in the PCR reaction. Cycling conditions in BioRad CFX96 instrument are 95°C for 3 minutes, followed by 40 cycles of denaturing at 95°C for 5 seconds, annealing and elongation at 60°C for 30 seconds. GAPDH (Glyceraldehyde 3-phosphate dehydrogenase), TBP (TATA box binding protein) and HRPT1 (hypoxanthine phosphoribosyltransferase 1) are used as endogenous controls. Fold change relative to the expression of the sample genes and reference genes is calculated using normalized expression (ΔΔ(Ct)) method with default threshold values using CFX Manager Software (BioRad).

Fold induction of genes involved in innate immune response respect to basal conditions are shown in Table 16. Genes analyzed are Chemokine (C-C motif) Ligand 5 (CCL5), Myeloid Differentiation primary response gene 88 (MyD88), Nuclear Factor Kappa B subunit 1 (NFK81) and Interferon Regulatory Factor 3 (IRF3).

| Table 16 | | | | |
|---|---|---|---|---|
| **Peptides** | **CCL5** | **MyD88** | **NFKB1** | **IRF3** |
| Ac-Glu-Glu-Met-Gln-Arg-Arg-NH₂* | 4.21 | 3.16 | 2.59 | 1.96 |
| Ac-Glu-Glu-Met-Gln-Arg-Arg-Ala-NH₂ | 8.52 | 3.58 | 2.19 | 2.37 |
| Ac-Glu-Glu-Met-Gln-Arg-Arg-Ala-Asp-NH₂ | 2.70 | 2.27 | 2.04 | 1.80 |
| Ac-Glu-Glu-Met-Gln-Arg-Arg-Ala-Asp-OH | 5.59 | 3.11 | 2.55 | 2.71 |
| Myr-Glu-Glu-Met-Gln-Arg-Arg-NH₂* | 7.57 | 3.47 | 2.64 | 2.26 |
| Palm-Glu-Glu-Met-Gln-Arg-Arg-NH₂ * | 5.37 | 3.66 | 2.62 | 2.40 |
| Ac-Glu-Glu-Met-Gln-Arg-Arg-NHC₁₆H₃₃* | 3.71 | 4.95 | 3.09 | 2.47 |
| Ac-Asp-Glu-Met-Gln-Arg-Arg-Ala-Asp-NH₂ | 5.89 | 1.99 | 2.04 | 1.58 |
| Ac-Gln-Glu-Met-Gln-Arg-Arg-Ala-Asp-NH₂ | 4.26 | 3.71 | 2.66 | 2.19 |
| Ac-Glu-Glu-Leu-Gln-Arg-Arg-Ala-Asp-NH₂ | 7.17 | 3.38 | 2.46 | 2.06 |
| Ac-Glu-Glu-Met-Glu-Arg-Arg-Ala-Asp-NH₂ | 6.95 | 3.65 | 2.63 | 1.90 |
| Ac-Glu-Glu-Met-Asn-Arg-Arg-Ala-Asp-NH₂ | 6.11 | 4.02 | 2.58 | 2.03 |
| Ac-Glu-Glu-Met-Asp-Arg-Arg-Ala-Asp-NH₂ | 6.58 | 3.98 | 2.60 | 2.04 |
| Ac-Glu-Glu-Met-Gln-Arg-Lys-Ala-Asp-NH₂ | 4.32 | 3.92 | 2.59 | 2.12 |
| Ac-Glu-Glu-Met-Gln-Arg-Arg-Ser-Asp-NH₂ | 4.24 | 3.68 | 2.57 | 2.18 |
| Ac-Glu-Glu-Met-Gln-Arg-Arg-Gly-Asp-NH₂ | 4.24 | 3.53 | 2.47 | 1.83 |
| Ac-Glu-Glu-Met-Gln-Arg-Arg-Ala-Glu-NH₂ | 5.85 | 3.62 | 2.32 | 2.00 |
| Ac-Glu-Glu-Met-Gln-Arg-Arg-Ala-Asn-NH₂ | 4.55 | 3.86 | 2.69 | 2.12 |
| Ac-Glu-Glu-Met-Gln-Arg-Arg-Ala-Gln-NH₂ | 5.86 | 3.90 | 2.73 | 2.02 |
| Ac-Glu-Glu-Met-Gln-Arg-His-Ala-Asp-NH₂ | 8.18 | 3.95 | 2.71 | 2.19 |
| Ac-Glu-Glu-Met-Gln-Arg-Arg-Thr-Asp-NH₂ | 6.18 | 3.52 | 2.39 | 1.99 |
| Ac-Glu-Glu-Met-Gln-Arg-Arg-Pro-Asp-NH₂ | 3.18 | 3.46 | 2.69 | 1.86 |
| Ac-Glu-Glu-Met-Gln-Arg-Arg-Glu-Asp-NH₂ | 6.37 | 3.75 | 2.58 | 1.97 |
| Ac-Glu-Glu-Met-Gln-Arg-Arg-Lys-Asp-NH₂ | 3.96 | 3.78 | 2.46 | 1.70 |
| H-Glu-Glu-Met-Gln-Arg-Arg-Ala-Asn-NH₂ | 4.94 | 3.72 | 2.31 | 1.91 |
| H-Glu-Glu-Met-Gln-Arg-Arg-Ala-Glu-NH₂ | 2.60 | 3.49 | 2.55 | 2.01 |
| Palm-Glu-Glu-Met-Gln-Arg-Arg-Ala-Asp-NH₂ | 4.42 | 3.62 | 2.76 | 2.05 |
| Palm-Glu-Glu-Met-Gln-Arg-Arg-Ala-Asn-NH₂ | 1.50 | 3.24 | 2.65 | 1.87 |
| Myr-Glu-Glu-Met-Gln-Arg-Arg-Ala-Glu-NH₂ | 6.31 | 4.28 | 2.72 | 1.99 |
| Ac-Glu-Glu-Met-Gln-Arg-Arg-Ala-Glu-OH | 5.60 | 4.33 | 2.53 | 2.02 |
| Ac-Glu-Glu-Met-Glu-Arg-Arg-Ala-Asp-OH | 7.87 | 2.73 | 2.24 | 1.75 |
| Ac-Gln-Glu-Met-Gln-Arg-Arg-Ala-Asp-OH | 3.08 | 4.37 | 2.56 | 2.00 |
| Palm-Glu-Glu-Met-Gln-Arg-Arg-Ala-Asn-OH | 1.79 | 4.02 | 2.63 | 2.06 |
| Ac-Glu-Glu-Met-Gln-Arg-Arg-Alg-Asp-NHC₆H₁₃ | 4.83 | 4.37 | 2.59 | 1.96 |
| Ac-Glu-Glu-Met-Gln-Arg-Arg-Ala-Asp-NHC₁₆H₃₃ | 2.84 | 2.64 | 2.79 | 1.68 |
| H-Glu-Glu-Met-Gln-Arg-Arg-Ala-Asp-NHC₆H₁₃ | 7.94 | 3.92 | 2.53 | 1.88 |
| Ac-Glu-Glu-Met-Gln-Arg-Arg-Ser-NH₂ | 5.49 | 4.48 | 2.88 | 2.12 |
| Ac-Glu-Glu-Met-Gln-Arg-Arg-Gly-NH₂ | 4.97 | 3.52 | 2.40 | 1.99 |
| Ac-Glu-Glu-Met-Glu-Arg-Arg-Ala-NH₂ | 5.91 | 4.45 | 2.62 | 2.14 |
| Ac-Asp-Glu-Met-Glu-Arg-Arg-Ala-NH₂ | 9.77 | 3.80 | 2.55 | 2.69 |
| Ac-Glu-Glu-Met-Gln-Arg-Arg-Thr-NH₂ | 10.54 | 4.45 | 2.71 | 2.48 |
| Ac-Glu-Glu-Met-Asp-Arg-Arg-Ala-NH₂ | 5.58 | 3.96 | 2.70 | 2.49 |
| Ac-Asn-Glu-Met-Gln-Arg-Arg-Ala-NH₂ | 7.48 | 3.29 | 2.56 | 2.14 |
| Ac-Glu-Glu-Met-Gln-Arg-Arg-Val-NH₂ | 6.09 | 1.51 | 1.24 | 2.07 |
| Ac-Glu-Glu-Met-Gln-Arg-Lys-NH₂ * | 6.67 | 2.77 | 2.21 | 2.27 |
| Ac-Glu-Glu-Met-Gln-Lys-Arg-NH₂ * | 2.04 | 2.75 | 2.03 | 1.51 |
| Ac-Glu-Glu-Met-Glu-Arg-Arg-NH₂ * | 4.76 | 2.42 | 2.12 | 1.88 |
| Ac-Glu-Glu-Met-Asn-Arg-Arg-NH₂ * | 1.96 | 1.86 | 1.48 | 1.28 |
| Ac-Glu-Asp-Met-Gln-Arg-Arg-NH₂ * | 5.87 | 1.47 | 1.55 | 1.22 |
| Ac-Glu-Gln-Met-Gln-Arg-Arg-NH₂ * | 5.80 | 2.41 | 2.25 | 2.25 |
| Ac-Gln-Glu-Met-Gln-Arg-Arg-NH₂ * | 3.36 | 3.53 | 2.57 | 2.48 |
| Ac-Glu-Glu-Met-Gln-Arg-His-NH₂ * | 6.94 | 5.31 | 3.37 | 2.68 |
| Ac-Asp-Glu-Met-Gln-Arg-Arg-NH₂ * | 5.21 | 3.15 | 2.41 | 2.58 |
| Ac-Glu-Glu-Met-Gln-Lys-Lys-NH₂ * | 5.12 | 2.45 | 2.15 | 2.52 |
| Ac-Glu-Glu-Ile-Glu-Arg-Arg-NH₂ * | 9.75 | 4.90 | 3.39 | 2.82 |
| H-Glu-Glu-Met-Gln-Arg-Arg-NH₂ * | 5.89 | 2.02 | 1.90 | 1.94 |
| H-Glu-Glu-Met-Gln-Arg-His-NH₂ * | 7.40 | 2.67 | 2.49 | 2.49 |
| Palm-Glu-Glu-Met-Gln-Arg-His-NH₂ * | 2.66 | 2.93 | 1.87 | 2.62 |
| Palm-Glu-Glu-Met-Gln-Arg-Lys-NH₂ * | 2.72 | 2.81 | 1.77 | 1.53 |
| Myr-Glu-Glu-Met-Gln-Arg-Lys-NH₂ * | 6.10 | 1.28 | 1.26 | 1.28 |
| Ac-Glu-Glu-Met-Gln-Arg-Arg-OH * | 10.85 | 2.08 | 1.71 | 1.73 |
| Ac-Glu-Glu-Met-Gln-Arg-Lys-OH * | 7.32 | 1.58 | 1.40 | 1.66 |
| Ac-Glu-Glu-Leu-Gln-Arg-Arq-OH * | 5.02 | 2.12 | 1.50 | 1.97 |
| Ac-Glu-Glu-Met-Gln-Arg-Arg-NHC₆H₁₃ * | 5.99 | 2.55 | 1.75 | 2.28 |
| H-Glu-Glu-Met-Gln-Arg-Arg-NHC₆H₁₃ * | 8.22 | 3.27 | 2.41 | 3.00 |

| | | | | |
|---|---|---|---|---|
| *not of the invention | | | | |

The results show that the peptides of the invention are able to modulate the expression level of genes involved in innate immune response, respect to basal conditions in primary human epidermal keratinocytes isolated from adult skin and at the tested concentration.

### EXAMPLE 24

### Assessment of adherence of Staphylococcus epidermidis in keratinocytes (HaCaT)

Microbiota is subdivided into two subgroups, the resident microbes and transient microbes. Resident microbes are a relatively fixed group of organisms and species, such as *Staphylococcus epidermidis,* that are almost always found in human epidermis contributing to skin homeostasis and which are able of reestablishing themselves after a perturbation). The transient microbes are species that arise from the environment and remain in the skin only for short periods of time such as *Staphylococcus aureus.* The competition amongst microbes is constant as they battle for space and nutrition on the skin's surface, and the possibility to avoid colonization by some undesired species presents itself as a defense mechanism of the skin. Therefore, the main objective of this experiment is to study the ability of the peptides of the invention to improve adhesion of *Staphylococcus epidermidis* in keratinocytes in the presence of bioparticles of *Staphylococcus aureus.*

HaCaT cells (keratinocyte cell line, Deutsches Krebsforschungszentrum) are seeded (8000 cells/well, 4 wells per condition) in 96-well plates for 24 hours at 37°C in a water-saturated atmosphere of 95% air and 5% CO₂. After those 24 hours, medium is removed and cells are incubated with a 50 µg/ml solution of one of the peptides shown in Table 17 in medium or with medium alone (basal conditions) for 24h at 37°C in a water-saturated atmosphere of 95% air and 5% CO₂. After that treatment, cells are washed with phosphate-buffered saline (PBS, Sigma) and are then incubated with a solution of *Staphylococcus epidermidis* (ATCC^{®}12228^{™}, Culture Collections) treated with green-fluorescence calcein AM (Life technologic) and Alexa Fluor 594 Bioparticles Staphylococcus aureus (Thermofisher) in PBS for 30 minutes at 37°C and protected from light. At the end of the incubation period, cells are washed with PBS and fixed in 4% (v/v) paraformaldehyde (PFA, Sigma) for 30 minutes. After rinsing with PBS, the nuclei are stained with Hoechst 33342, trihydrochloride trihydrate (Thermofisher) in PBS for 10 minutes before analysis.

Cellular imaging is performed by fluorescence microscopy using the Operetta^{®} High Content Imaging System (PerkinElmer). From each fluorescence image, *Staphylococcus epidermidis* is quantified as a number of spots and normalized against the number of keratinocyte nuclei and the total bacteria number (understood as the addition of detected S. *epidermidis* spots and S. *aureus* bioparticles). Each value obtained is normalized respect to basal conditions (non- treated keratinocytes). An image is taken in three different fields per well. 12 representative images from each condition are analyzed with Harmony software (PerkinElmer).

The normalized percentage of *Staphylococcus epidermidis* respect to basal conditions in treated keratinocytes is represented in Table 17.

| Table 17 | |
|---|---|
| **Treatment** | ***S*. *epidermidis* (%)** |
| Basal conditions | 0 |
| Ac-Glu-Glu-Met-Gln-Arg-Arg-NH₂ * | 13.63. |
| Ac-Glu-Glu-Met-Gln-Arg-Arg-Ala-NH₂ | 24.69 |
| Ac-Glu-Glu-Met-Gln-Arg-Arg-Ala-Asp-NH₂ | 11.46 |
| Ac-Glu-Glu-Met-Gln-Arg-Arg-Ala-Asp-OH | 25.36 |
| Myr-Glu-Glu-Met-Gln-Arg-Arg-NH₂ * | 27.52 |
| Palm-Glu-Glu-Met-Gln-Arg-Arg-NH₂ * | 29.75 |
| Ac-Glu-Glu-Met-Gln-Arg-Arg-NHC₁₆H₃₃* | 11.43 |
| Ac-Asp-Glu-Met-Gln-Arg-Arg-Ala-Asp-NH₂ | 20.45 |
| Ac-Gln-Glu-Met-Gln-Arg-Arg-Ala-Asp-NH₂ | 21.36 |
| Ac-Glu-Glu-Leu-Gln-Arg-Arg-Ala-Asp-NH₂ | 11.54 |
| Ac-Glu-Glu-Met-Glu-Arg-Arg-Ala-Asp-NH₂ | 4.24 |
| Ac-Glu-Glu-Met-Asn-Arg-Arg-Ala-Asp-NH₂ | 14.00 |
| Ac-Glu-Glu-Met-Asp-Arg-Arg-Ala-Asp-NH₂ | 21.79 |
| Ac-Glu-Glu-Met-Gln-Arg-Lys-Ala-Asp-NH₂ | 22.18 |
| Ac-Glu-Glu-Met-Gln-Arg-Arg-Ser-Asp-NH₂ | 15.10 |
| Ac-Glu-Glu-Met-Gln-Arg-Arg-Gly-Asp-NH₂ | 51.11 |
| Ac-Glu-Glu-Met-Gln-Arg-Arg-Ala-Glu-NH₂ | 11.33 |
| Ac-Glu-Glu-Met-Gln-Arg-Arg-Ala-Asn-NH₂ | 26.35 |
| Ac-Glu-Glu-Met-Gln-Arg-Arg-Ala-Gln-NH₂ | 81.69 |
| Ac-Glu-Glu-Met-Gln-Arg-His-Ala-Asp-NH₂ | 16.66 |
| Ac-Glu-Glu-Met-Gln-Arg-Arg-Thr-Asp-NH₂ | 16.21 |
| Ac-Glu-Glu-Met-Gln-Arg-Arg-Pro-Asp-NH₂ | 81.90 |
| Ac-Glu-Glu-Met-Gln-Arg-Arg-Glu-Asp-NH₂ | 20.10 |
| Ac-Glu-Glu-Met-Gln-Arg-Arg-Lys-Asp-NH₂ | 10.41 |
| H-Glu-Glu-Met-Gln-Arg-Arg-Ala-Glu-NH₂ | 18.10 |
| Palm-Glu-Glu-Met-Gln-Arg-Arg-Ala-Asp-NH₂ | 27.62 |
| Palm-Glu-Glu-Met-Gln-Arg-Arg-Ala-Asn-NH₂ | 39.68 |
| Myr-Glu-Glu-Met-Gln-Arg-Arg-Ala-Glu-NH₂ | 33.02 |
| Ac-Glu-Glu-Met-Glu-Arg-Arg-Ala-Asp-OH | 29.41 |
| Ac-Gln-Glu-Met-Gln-Arg-Arg-Ala-Asp-OH | 13.89 |
| Palm-Glu-Glu-Met-Gln-Arg-Arg-Ala-Asn-OH | 58.74 |
| Ac-Glu-Glu-Met-Gln-Arg-Arg-Ala-Asp-NHC₆H₁₃ | 61.59 |
| Ac-Glu-Glu-Met-Gln-Arg-Arg-Ala-Asp-NHC₁₆H₃₃ | 90.22 |
| H-Glu-Glu-Met-Gln-Arg-Arg-Ala-Asp-NHC₆H₁₃ | 29.93 |
| Ac-Glu-Glu-Met-Gln-Arg-Arg-Ser-NH₂ | 7.49 |
| Ac-Glu-Glu-Met-Gln-Arg-Arg-Gly-NH₂ | 49.00 |
| Ac-Glu-Glu-Met-Glu-Arg-Arg-Ala-NH₂ | 71.97 |
| Ac-Asp-Glu-Met-Glu-Arg-Arg-Ala-NH₂ | 20.56 |
| Ac-Glu-Glu-Met-Gln-Arg-Arg-Thr-NH₂ | 35.69 |
| Ac-Glu-Glu-Met-Asp-Arg-Arg-Ala-NH₂ | 12.36 |
| Ac-Asn-Glu-Met-Gln-Arg-Arg-Ala-NH₂ | 30.17 |
| Ac-Glu-Glu-Met-Gln-Arg-Lys-NH_{2 *} | 5.48 |
| Ac-Glu-Glu-Met-Gln-Lys-Arg-NH_{2 *} | 3.97 |
| Ac-Glu-Glu-Met-Glu-Arg-Arg-NH_{2 *} | 2.91 |
| Ac-Glu-Glu-Met-Asn-Arg-Arg-NH_{2 *} | 18.02 |
| Ac-Glu-Glu-Leu-Gln-Arg-Arg-NH₂ * | 45.38 |
| Ac-Glu-Asp-Met-Gln-Arg-Arg-NH₂ * | 5.86 |
| Ac-Glu-Gln-Met-Gln-Arg-Arg-NH₂ * | 4.14 |
| Ac-Asp-Glu-Met-Gln-Arg-Arg-NH_{2 *} | 65.15 |
| Ac-Glu-Glu-Met-Gln-Lys-Lys-NH_{2 *} | 10.91 |
| Ac-Glu-Glu-Ile-Glu-Arg-Arg-NH_{2 *} | 2.41 |
| H-Glu-Glu-Met-Gln-Arg-Arg-NH_{2 *} | 15.75 |
| H-Glu-Glu-Met-Gln-Arg-His-NH_{2 *} | 13.57 |
| Palm-Glu-Glu-Met-Gln-Arg-Lys-NH_{2 *} | 4.63 |
| Myr-Glu-Glu-Met-Gln-Arg-Lys-NH_{2 *} | 24.84 |
| Ac-Glu-Glu-Met-Gln-Arq-Arg-OH _{*} | 3.08 |
| Ac-Glu-Glu-Met-Gln-Arg-Lys-OH _{*} | 6.78 |
| H-Glu-Glu-Met-Gln-Arg-Arg-NHC₁₆H_{33 *} | 14.91 |

| | |
|---|---|
| *not of the invention | |

The results show that the peptides of the invention are able to enhance adherence of Staphylococcus epidermidis in keratinocytes in competition with Staphylococcus aureus.

### EXAMPLE 25

*In vivo study with the composition of Example 16, for the assessment of the desquamation reduction efficacy in female volunteers.*

The study is carried out during 7 days with measurements at initial time, and after 7 days of product application. 20 female volunteers aged over 18 years old showing dry skin tendency in legs are included. Subjects apply the composition described in example 16 on the lower part of one leg (left or right) and a placebo cream described in example 17 on the lower part of the other leg. Both creams are applied twice a day (morning and night). The subjects serve as their own reference and results obtained at different times are compared with those obtained at initial time. Moreover, results obtained with the composition described in example 16 are compared with those obtained with the placebo composition described in example 17.

The efficacy of the product is assessed by:
Skin stripping to take corneocytes layers from the stratum corneum, staining with fluorescein green dye and fluorescence reading. Results are shown in table 18.

**Table 18. Percentage variations respect initial time of fluorescence reading after days of product application.**

| | **Variations (%) [(T+7 days - T0)/T0]x100** |
|---|---|
| **Fluorescence - Composition of Example 16** | **-18.7%** |
| **Fluorescence - Placebo Cream (Example 17)** | **-3.8%** |

The results shown in table 18 demonstrate that, over a treatment period of 7 days, the composition described in example 16 is effective in reducing the desquamation of skin. The decrease observed in desquamation indicates that an increase in skin moisturization takes place by use of the composition described in example 16, which also points to an improvement of the skin barrier function due to the application of said composition.

### EXAMPLE 26

*In vivo* study for assessment of the efficacy in protecting the skin barrier in Caucasian skin type female volunteers.

The aim of this study is to demonstrate the capacity of the peptides of the invention to improve the barrier protection of the skin against hazardous agents (i.e. irritating agents).

The study is carried out over 11 days. Twenty (20) Caucasian female volunteers are included in the study. Subjects apply either the composition described in Example 16, a placebo cream described in Example 17, a positive control (Zinc oxide suspension 500mg/ml) or a negative control (deionised water, type II water) on designated areas of the volar part of the forearm. All the compositions and controls are applied twice a day (morning and night) during the first 7 days of the study. After that, a skin irritation with an aqueous solution of sodium lauryl sulphate at 1% (w/v) is induced. Then, the induced skin's irritation is covered with a patch for 24 hours. The integrity of skin barrier is assessed instrumentally by measuring Transepidermal Water Loss (TEWL) with a Tewameter^{®} TM 300. TEWL is measured before inducting skin's irritation and 30 minutes and 48 hours after patch removal. The subjects serve as their own reference and results obtained at 48 hours after patch removal are compared with those obtained before irritating volunteers' skin. Moreover, results obtained with the composition of Example 16 are compared with those obtained with placebo cream.

Results are shown in Table 19.

**Table 19. Changes in TEWL of positive control, negative control, placebo cream or composition of Example 16 after skin irritation.**

| | % TEWL decrease |
|---|---|
| Positive control (Zinc oxide suspension 500mg/ml) | -28.18 |
| negative control (deionised water, type II water) | -17.96 |
| Placebo cream of Example 17 | -18.10 |
| Composition of Example 16 | -27.84 |

The results demonstrate that, after 7 days of application of the composition of Example 16, the treated skin exhibits a significant TEWL decrease, and therefore the composition of the invention is effective in enhancing the protection afforded to the skin by the skin barrier.

### EXAMPLE 27

*In vivo* study for the assessment of the efficacy in accelerating the reparation of the skin barrier in Caucasian skin type female volunteers.

The aim of this study is to demonstrate the capacity of compositions comprising the peptides of the invention to improve the capacity of the skin barrier to be repaired. To verify the repairing efficacy of the composition, the 'tape stripping' method, which causes damage to the skin, is used.

Twenty (20) Caucasian female volunteers are included in the study. At the beginning of the study, tape stripping is performed on each testing site using Corneofix^{®} tapes until the skin barrier becomes compromised (i.e, when the skin presents a glistening layer or a value of Transepidermal Water Loss (TWEL) between 30-70 g/h/m²). The study is conducted for 4 hours, with measurements at initial time (after tape stripping) and 4 hours after the application of the composition described in Example 16 or the placebo cream described in Example 17 on designated areas of the volar part of the forearm. The subjects serve as their own reference and results obtained at 4 hours after the application of the composition described in Example 16 are compared with those obtained at initial time (after tape stripping). Moreover, results obtained with the composition of Example 16 are compared with those obtained with placebo cream described in Example 17.

The integrity of skin barrier is assessed instrumentally by measuring Transepidermal Water Loss (TEWL) with a Tewameter^{®} TM 300 immediately after tape stripping and 4 hours after the application of the products. Results are shown in Table 20.

**Table 20. Variations of TEWL expressed in % at 4 hours after application of composition of Examples 16 (active) and 17 (placebo) versus initial time (after tape stripping)**

| | % TEWL mean decrease |
|---|---|
| Placebo cream of Example 17 | - 35.62 |
| Composition of Example 16 | - 39.81 |

The results shown in Table 20 demonstrate that after 4 hours of application of the composition of Example 16 there is a decrease of TEWL mean values, and therefore the composition of the invention enhances the reparation of the skin barrier.

### EXAMPLE 28

*In vivo* study for the assessment of skin's microbiota improvement in Caucasian skin type female volunteers.

The capacity of the peptides of the invention to modulate the skin microbiome was assessed on volunteers by means of metagenomic study of skin microbiota. Particularly, the objective was to demonstrate an improvement of microbiota diversity (i.e. Shannon index), the presence of relevant microbial phylum and changes in microbiome functional profile.

Twenty-one (21) Caucasian female volunteers were enrolled in the study, which was conducted for 7 days. Subjects apply the composition described in Example 16 on cubital fossa (left or right) and a placebo cream described in Example 17 on the other cubital fossa. Composition of Example 16 and placebo are applied during 7 days twice a day (morning and night). The subjects serve as their own reference and results obtained at time 7 days are compared with those obtained at initial time. Moreover, results obtained with the composition of Example 16 are compared with those obtained with placebo cream.

Samples for microbiota analysis are obtained using swabs which are rubbed for one minute in each area. Swabs are preserved in a solution of 0.1% Triton (Sigma) in Phosphate Buffered Saline (PBS, Sigma) solution and are kept at -80°C until DNA extraction and purification. Microbial DNA extraction and purification is performed with Tissue DNA GPSpin Kit (Genetic PCR Solutions) according to manufacturer's instructions. In order to perform a metagenomic analysis of the volunteer's skin microbiota, 16S region are amplified with 16S Ion Metagenomics Kit (Life Technologies). The amplified region 16S is a gene coding for a ribosomal RNA used in reconstructing phylogenies due to its slow rate of evolution. Two separate PCR reactions are performed using two different primer sets. At the end of the PCR reaction, equal volumes of the two PCR reactions are combined and processed to make a DNA library using Ion Plus Fragment Library Kit (Thermo-Fisher) and Ion Xpress Barcodes Adapters (Thermo-Fisher). The combination of the two primer pools enables broad-range, sequence based identification of bacteria from complex mixed populations.

Sequencing was performed by an Ion Personal Genome Machine (PGM) using 400-bp kit and seven 318 chip. Base calling and run demultiplexing were performed by Torrent Suite version 5.0 (Life Technologies) with default parameters. FileExporter plugin was used to generate demultiplexed fastq files for each sample. Mean read length for forward and reverse reads ranged between 235bp to 238bp for all samples. Data obtained is analysed performing three different analysis:
- Ion reporter Analysis: the sequencing results are uploaded to Ion Reporter Server 5.2 (Thermo-Fisher). This software enables the rapid identification of microbes present in complex polybacterial research samples, using both curated Greengenes1and premium curated MicroSEQ^{®} ID 16S rRNA reference databases. The sequences (complete amplicons) flanked by the corresponding primers, are extracted in both directions (forward and reverse). Readings less than 150 bp are rejected. The primers are removed from the extracted sequences. Finally, identical sequences are grouped, and those groups with fewer than 10 sequences are deleted. Blast assigned the taxonomy of each sequence group, using the GreenGenes and MicroSEQ^{®} ID 16S rRNA databases and relative abundance at phylum and family level is evaluated. The recommendations of the Clinical and Laboratory Standards Institute (CLSI) guidelines are followed, which require <97% identity for the family level.
- QIIME analysis: a second analysis is performed using the QIIME2 v1.9.1 (Quantitative Insights Into Microbial Ecology) software package, only for the V3 amplicon. The sequences (complete amplicons) flanked by the corresponding primers V3 are extracted in both directions (forward and reverse). Readings less than 150 bp and / or with an average quality index Q less than 20 are rejected. Finally, the primers are removed from the extracted sequences. The sequences of these primers, although unknown, are easily obtained after analyzing the raw data. The analysis consists of several parts: In a closed-reference OTU picking process, reads are clustered against a reference sequence collection and assigned to OTUs and then the assigned taxonomy to OTUS is performed by GreenGenes V13-8 reference database. Bacterial community diversity is measured by the Shannon diversity index, an ecological measure of microbial communities that considers the following: (1) richness, or the total number of bacterial types, and (2) evenness, or the relative proportion of these bacterial types. This index reflects the diversity within the sample based on the abundance of various taxa within a community
- Metagenome functional contect prediction: A third analysis is done using PICRUSt³ (phylogenetic investigation of communities by reconstruction of unobserved states), a computational approach to predict the functional composition of a metagenome using marker gene data (16S) and a database of reference genomes. PICRUSt uses an extended ancestral-state reconstruction algorithm to predict which gene families are present and then combines gene families to estimate the composite metagenome using 16S information. From the OTU table (closed reference) of the previous analysis (V3 amplicon) the prediction of the functional composition is performed following the next steps:
   - *Normalization of the OTU Table:* normalizes the OTU table by dividing each OTU by the known/predicted 16S copy number abdundance.
   - *Predict Functions For Metagenome:* creates the final metagenome functional predictions. It multiplies each normalized OTU abundance by each predicted functional trait abundance to produce a table of functions by samples.
   - *Collapse predictions into pathways:* Collapses the thousands of predicted functions into higher categories (e.g KOs into KEGG Pathways).

Table 21 shows changes of diversity (i.e. Shannon index) and abundance of microbial Phylums after treatment with either placebo cream or composition of Example 16 (final values - initial values).

**Table 21. Changes in microbial diversity (T-final vs T-initial) for all volunteers enrolled in the study, only those of less than 35 years old (y.o.) and those 35 years old or older.**

| | | **n** | **PLACEBO** | **COMPOSITION OF EXAMPLE 16** |
|---|---|---|---|---|
| Diversitiy Shannon index | All volunteers | 21 | 0.69 | 1.08 |
| | < 35 y.o. | 11 | 0.36 | 0.81 |
| | ≥ 35 y.o. | 10 | 1.07 | 1.39 |

**Table 22. Changes in abundance of Phylums (T-final vs T-initial). N=21.**

| | | **PLACEBO** | **COMPOSITION OF EXAMPLE 16** |
|---|---|---|---|
| Abundance of Phylums | Firmicutes | -8.93 | -21.34 |
| | Actinobacteria | 2.18 | 5.76 |
| | Proteobacteria | 6.66 | 15.24 |
| | Bacteroidetes | -0.04 | 0.12 |
| | Cyanobacteria | 0.08 | 0.19 |

The Shannon index takes into account the richness (i.e. amount of taxonomic groups). A low Shannon index value indicates a low microbial diversity, which is commonly associated with a less healthy skin. After the treatment with the active, the Shannon index value of the skin showed a higher increment compared to that of skin treated with the placebo, indicating a better efficacy in enhancing microbial diversity, which in turn suggests an improvement in skin health. This effect was observed in both groups (< 35 y.o. and ≥ 35 y.o), demonstrating that it not only improves diversity in the group of < 35 y.o. but also in the group of ≥ 35 y.o.

The composition of the invention improved the microbial balance of the skin by increasing the relative abundance of Proteobacteria (15%) and reducing the abundance of Firmicutes (21%).

Table 23 shows the capacity of the composition comprising the peptide of the invention to modulate pathways that contribute to enhance the growth and health of skin microbiota.

**Table 23: Changes in predictive microbial functional profile obtained and represented by KEGG pathway (level 3). N=21**

| | **KEGG** | **Relative abundance (%)** | |
|---|---|---|---|
| Environmental information | TRANSPORT | Placebo | Composition of Example 16 |
| | Environmental Information Processing; Membrane Transport; Transporters | 0.169 | 0.287 |
| | Environmental Information Processing; Membrane Transport; Secretion system | 0.045 | 0.082 |
| | Environmental Information Processing; Membrane Transport; ABC transporters | 0.106 | 0.163 |
| Cellular processes | MOTILITY | | |
| | Cellular Processes; Cell Motility; Bacterial motility proteins | 0.162 | 0.285 |
| | Cellular Processes; Cell Motility; Bacterial chemotaxis | 0.054 | 0.110 |
| | Cellular Processes; Cell Motility; Flagellar assembly | 0.072 | 0.119 |
| Metabolism | METABOLISM_XENOBIOTICS | | |
| | Metabolism; Xenobiotics Biodegradation and Metabolism; Benzoate degradation | -0.022 | 0.032 |
| | METABOLISM_LIPID | | |
| | Metabolism; Lipid Metabolism; Biosynthesis of unsaturated fatty acids | 0.007 | 0.026 |
| | Metabolism; Lipid Metabolism; Fatty acid metabolism | -0.003 | 0.053 |
| | Metabolism; Lipid Metabolism; Sphingolipid metabolism | 0.006 | 0.014 |
| | METABOLlSM_AA | | |
| | Metabolism; Metabolism of Amino Acids; Glutathione metabolism | 0.011 | 0.037 |
| | ENERGY | | |
| | Metabolism; Energy Metabolism; Methane metabolism | -0.002 | 0.010 |
| | Metabolism; Energy Metabolism; Oxidative phosphorylation | 0.039 | 0.056 |
| | METABOLISM_VITAMINS | | |
| | Metabolism; Metabolism of Cofactors and Vitamins; Biotin metabolism | 0.005 | 0.011 |

The active ingredient modulates pathways associated with the transport or uptake of food and bacterial movement that promote the growth of the bacteria on the skin. Besides, it also modulates several metabolic pathways related to energy, lipids, amino acids, vitamins and xenobiotics degradation that may in turn contribute to improve the skin.

### References

1 Feingold K, Elias P. Role of lipids in the formation and maintenance of the cutaneous permeability barrier. Biochimica et Biophysica Acta. 2014 Mar; 1841(3):280-294
2 Jan̊ušová B, Zbytovská J, Lorenc P, Vavrysová H, Palát K, Hrabálek A, Vávrová K. Effect of ceramide acyl chain length on skin permeability and thermotropic phase behavior of model stratum corneum lipid membranes. Biochim Biophys Acta. 2011 Mar; 1811(3):129-37
3 Boireau-Adamezyk E, Baillet-Guffroy A, Stamatas GN. Age-dependent changes in stratum corneum barrier function. Skin Res Technol. 2014 Nov; 20 (4):409-15
4 *Š*kolová B, Janůšová 8, Zbytovská J, Gooris G, Bouwstra J, Slepička P, Berka P, Roh J, Palát K, Hrabálek A, Vávrová K. Ceramides in the skin lipid membranes: length matters. Langmuir. 2013 Dec; 29(50):15624-33
5 Quatresooz P. et al, The riddle of genuine skin microrelief and wrinkles., International Journal of Cosmetic Science, 2006, 28, 389-395
5a Sato J, Yanai M, Hirao T, Denda M. Water content and thickness of the stratum corneum contribute to skin surface morphology. Arch Dermatol Res. 2000 Aug;292(8):412-7
6 O'Neill CA, Garrod D. Tight junction proteins and the epidermis. Exp Dermatol. 2011 Feb; 20(2):88-91
7 Tokumasu R, Yamaga K, Yamazaki Y, Murota H, Suzuki K, Tamura A, Bando K, Furuta Y, Katayama I, Tsukita S. Dose-dependent role of claudin-1 in vivo in orchestrating features of atopic dermatitis. Proc Natl Acad Sci U S A. 2016 Jul 12; 113(28):E4061-8
8 Grice EA, Segre JA. The skin microbiome. Nat Rev Microbiol. 2011 Apr; 9(4):244-53
9 Wanke I, Steffen H, Christ C, Krismer 8, Götz F, Peschel A, Schaller M, Schittek B. Skin commensals amplify the innate immune response to pathogens by activation of distinct signaling pathways. J Invest Dermatol. 2011 Feb; 131(2):382-90*;* Sanford JA, Gallo RL. Functions of the skin microbiota in health and disease. Semin Immunol. 2013 Nov 30; 25(5):370-7
10 Iram N, Mildner M, Prior M, Petzelbauer P, Fiala C, Hacker S, Schöppl A, Tschachler E, Elbe-Bürger A. Age-related changes in expression and function of Toll-like receptors in human skin. Development. 2012 Nov; 139(22):4210-9
11 Akira S. Toll-like receptor signaling. J Biol Chem. 2003 Oct 3;278(40):38105-8
12 Iram N, Mildner M, Prior M, Petzelbauer P, Fiala C, Hacker S, Schöppl A, Tschachler E, Elbe-Bürger A. Age-related changes in expression and function of Toll-like receptors in human skin. Development. 2012 Nov; 139(22):4210-9
13 Yuki T, Yoshida H, Akazawa Y, Komiya A, Sugiyama Y, Inoue S. Activation of TLR2 enhances tight junction barrier in epidermal keratinocytes. J Immunol. 2011 Sep 15; 187(6):3230-7
14 Dréno 8, Araviiskaia E, Berardesca E, Gontijo G, Sanchez Viera M, Xiang LF, Martin R, Bieber T. Microbiome in healthy skin, update for dermatologists. J Eur Acad Dermatol Venereol. 2016 Oct 13
15 Wang Y, Kuo S, Shu M, Yu J, Huang S, Dai A, Two A, Gallo RL, Huang CM. Staphylococcus epidermidis in the human skin microbiome mediates fermentation to inhibit the growth of Propionibacterium acnes: implications of probiotics in acne vulgaris. Appl Microbiol Biotechnol. 2014 Jan; 98(1):411-24
16 Chiller K, Selkin BA, Murakawa GJ. Skin microflora and bacterial infections of the skin. J Investig Dermatol Symp Proc. 2001 Dec; 6(3): 170-4
17 Williams MR, Gallo RL. The role of the skin microbiome in atopic dermatitis. Curr Allergy Asthma Rep. 2015 Nov; 15(11): 65

## Claims

1. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the maintenance and/or improvement of the physical barrier function of the skin and the immunological barrier function of the skin.

2. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the maintenance and/or improvement of the physical barrier function of the skin.

3. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the reduction or the loss of water and electrolytes from the skin; the maintenance and/or improvement of skin moisturization; the prevention or reduction of desquamation of the skin; and/or the prevention or reduction desquamation of the skin in a subject suffering from dry skin or having a tendency towards dry skin.

4. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the maintenance and/or improvement of skin microrelief; and/or the maintenance and/or improvement of skin softness and/or the skin's velvety aspect.

5. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the protection of the skin from environmental hazards including chemical irritants, pollutants and pathogens.

6. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the maintenance and/or improvement of the innate immune defense mechanism of the skin.

7. Use of a compound of formula (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and Ile;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
in the cosmetic, non-therapeutic treatment and/or care of the skin, hair, nails and/or mucous membranes, wherein said cosmetic, non-therapeutic treatment and/or care is the maintenance of the microbial equilibrium of the skin.

8. A cosmetic, non-therapeutic method of treatment and/or care of the skin, hair, nails and/or mucous membranes of a subject comprising administering a cosmetically effective amount of a compound of formula (I),
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
to the subject, wherein the treatment and/or care is: the maintenance and/or improvement of the physical barrier function of the skin and the immunological barrier function of the skin; the reduction of the loss of water and electrolytes from the skin; the maintenance and/or improvement of skin hydration, i.e. the maintenance and/or improvement of skin moisturization; the prevention, the maintenance and/or improvement of skin microrelief; the maintenance and/or improvement of skin softness and/or the skin's velvety aspect; the protection of the skin from environmental hazards including chemical irritants, pollutants and pathogens; the maintenance and/or improvement of the innate immune defense mechanism of the skin; and/or the maintenance of the microbial equilibrium of the skin.

9. Use according to any one of claims 1 to 7 or a method according to claim 8, wherein 0 or 1 of AA₁ to AA₆ is replaced.

10. Use according to any one of claims 1 to 7 or 9, or a method according to claim 8 or claim 9, wherein a = 1 and b = 0 and, preferably, (i) when 1 of AA₁ to AA₆ is replaced, AA₁ is replaced by Asp; or AA₄ is replaced by Asp; and (ii) when 2 of AA₁ to AA₆ are replaced, AA₁ is replaced by Asp and AA₄ is replaced by Glu.

11. Use according to any one of claims 1 to 7 or 9, or a method according to claim 8 or claim 9, wherein a = 1 and b = 1 and, preferably, (i) when 1 of AA₁ to AA₆ is replaced, AA₁ is replaced by Asp or Gin; or AA₃ is replaced by Leu; or AA₄ is replaced by Glu, Asp or Asn; or AA₆ is replaced by Lys or His.

12. Use according to any one of claims 1 to 7 or a method according to claim 8, wherein the compound is RᵣGlu-Glu-Met-Gln-Arg-Arg-Ala -R₂.

13. Use according to any one of claims 1 to 7 or 9 to 12, or a method according to claim 8, claim 9, claim 10, claim 11 or claim 12, wherein R₁ is selected from the group consisting of H and R₅-CO-, wherein R₅ is selected from the group consisting of C₁-C₁₈ alkyl, C₂-C₂₄ alkenyl, C₃-C₂₄ cycloalkyl; and R₂ is -NR₃R₄ or -OR₃ wherein R₃ and R₄ are independently selected from the group consisting of H and C₁-C₁₆ alkyl.

14. Compound of formula (I),
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
its stereoisomers and/or its cosmetically or pharmaceutically acceptable salts, wherein:
AA₁ is Glu; AA₂ is Glu; AA₃ is Met; AA₄ is Gin; AA₅ is Arg; AA₆ is Arg; AA₇ is selected from the group consisting of Ala, Ser, Gly, Thr, Pro, Glu, Lys and Val;
AA₈ is selected from the group consisting of Asp, Glu, Asn and Gin; and
0, 1 or 2 of AA₁, AA₂, AA₃, AA₄, AA₅ and AA₆ are replaced providing that:
when AA₁ is replaced, it is replaced by an amino acid selected from the group formed by Asp, Gln and Asn;
when AA₂ is replaced, it is replaced by an amino acid selected from the group formed by Asp and Gin;
when AA₃ is replaced, it is replaced by an amino acid selected from the group formed by Leu and lie;
when AA₄ is replaced, it is replaced by an amino acid selected from the group formed by Glu, Asn and Asp;
when AA₅ is replaced, it is replaced by Lys; and
when AA₆ is replaced, it is replaced by an amino acid selected from the group formed by Lys and His;
a is 1 and b is 0 or 1;
W, X, Y and Z are each independently an amino acid;
m, n, p and q are each independently 0 or 1;
m+n+p+q is less than or equal to 2;
R₁ is selected from the group consisting of H, a polymer derived from polyethylene glycol, a non-cyclic aliphatic group, alicyclyl, heterocyclyl, heteroarylalkyl, aryl, aralkyl and R₅-CO-, wherein R₅ is selected from the group consisting of H, a non-cyclic aliphatic group, alicyclyl, aryl, aralkyl, heterocyclyl and heteroarylalkyl;
R₂ is selected from the group consisting of -NR₃R₄, -OR₃, -SR₃, wherein R₃ and R₄ are independently selected from a group consisting of H, a polymer derived from polyethylene glycol, a non-cyclic aliphatic group, alicyclyl, heterocyclyl, heteroarylalkyl, aryl and aralkyl; and
R₁ and R₂ are not amino acids,
for use in the prevention or treatment of atopic dermatitis, contact dermatitis, eczema, psoriasis and/or acne vulgaris.

15. Compound for use according to claim 14, wherein the compound is R₁-Glu-Glu-Met-Gln-Arg-Arg-Ala-R₂.

## Patentansprüche

1. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gin besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gin und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege die Aufrechterhaltung und/oder Verbesserung der physikalischen Barrierefunktion der Haut und der immunologischen Barrierefunktion der Haut ist.

2. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gin besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gin und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege die Aufrechterhaltung und/oder Verbesserung der physikalischen Barrierefunktion der Haut ist.

3. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gin besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gin und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege die Verringerung des Verlusts von Wasser und Elektrolyten aus der Haut; die Aufrechterhaltung und/oder Verbesserung der Hautfeuchtigkeit; die Vorbeugung oder Verringerung der Abschuppung der Haut; und/oder die Vorbeugung oder Verringerung der Abschuppung der Haut bei einem Subjekt ist, das unter trockener Haut leidet oder zu trockener Haut neigt.

4. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gin besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gin und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege die Aufrechterhaltung und/oder Verbesserung der Verbesserung des Mikroreliefs der Haut; und/oder die Aufrechterhaltung und/oder Verbesserung der Hautweichheit und/oder des samtigen Erscheinungsbildes der Haut ist.

5. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gln besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gln und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege der Schutz der Haut vor Umweltgefahren, einschließlich chemischen Reizstoffen, Schadstoffen und Krankheitserregern ist.

6. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gln besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gln und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege die Aufrechterhaltung und/oder Verbesserung des angeborenen Immunabwehrmechanismus der Haut ist.

7. Verwendung einer Verbindung der Formel (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gln besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gln und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
bei der kosmetischen, nicht-therapeutischen Behandlung und/oder Pflege der Haut, Haaren, Nägeln und/oder Schleimhäuten, wobei die kosmetische, nichttherapeutische Behandlung und/oder Pflege die Aufrechterhaltung des mikrobiellen Gleichgewichts der Haut ist.

8. Kosmetisches, nicht-therapeutisches Verfahren zur Behandlung und/oder Pflege der Haut, Haare, Nägel und/oder Schleimhäute eines Subjekts, umfassend das Verabreichen einer kosmetisch wirksamen Menge einer Verbindung der Formel (I),
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gln besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gln und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
an dem Subjekt, wobei die Behandlung und/oder Pflege ist: Die Aufrechterhaltung und/oder Verbesserung der physikalischen Barrierefunktion der Haut und der immunologischen Barrierefunktion der Haut; die Verringerung des Verlusts von Wasser und Elektrolyten der Haut; die Aufrechterhaltung und/oder Verbesserung der Hautfeuchtigkeit, d. h. die Aufrechterhaltung und/oder Verbesserung der Hautfeuchtigkeit; die Vorbeugung, die Aufrechterhaltung und/oder Verbesserung des Mikroreliefs der Haut; die Aufrechterhaltung und/oder Verbesserung der Hautweichheit und/oder des samtigen Erscheinungsbildes der Haut; der Schutz der Haut vor Umweltgefahren, einschließlich chemischen Reizstoffen, Schadstoffen und Krankheitserregern; die Aufrechterhaltung und/oder Verbesserung des angeborenen Immunabwehrmechanismus der Haut; und/oder die Aufrechterhaltung des mikrobiellen Gleichgewichts der Haut.

9. Verwendung nach einem der Ansprüche 1 bis 7 oder ein Verfahren nach Anspruch 8, wobei 0 oder 1 von AA₁ bis AA₆ ersetzt ist.

10. Verwendung nach einem der Ansprüche 1 bis 7 oder 9 oder ein Verfahren nach Anspruch 8 oder Anspruch 9, wobei a = 1 und b = 0 und vorzugsweise (i) wenn 1 von AA₁ bis AA₆ ersetzt ist, AA₁ durch Asp ersetzt ist; oder AA₄ durch Asp ersetzt ist; und (ii) wenn 2 von AA₁ bis AA₆ ersetzt sind, AA₁ durch Asp und AA₄ durch Glu ersetzt ist.

11. Verwendung nach einem der Ansprüche 1 bis 7 oder 9 oder ein Verfahren nach Anspruch 8 oder Anspruch 9, wobei a = 1 und b = 1 und vorzugsweise (i) wenn 1 von AA₁ bis AA₆ ersetzt ist, AA₁ durch Asp oder Gln ersetzt ist; oder AA₃ durch Leu ersetzt ist; oder AA₄ durch Glu, Asp oder Asn ersetzt ist; oder AA₆ durch Lys oder His ersetzt ist.

12. Verwendung nach einem der Ansprüche 1 bis 7 oder ein Verfahren nach Anspruch 8, wobei die Verbindung R₁-Glu-Glu-Met-Gln-Arg-Arg-Ala -R₂ ist.

13. Verwendung nach einem der Ansprüche 1 bis 7 oder 9 bis 12 oder ein Verfahren nach Anspruch 8, Anspruch 9, Anspruch 10, Anspruch 11 oder Anspruch 12, wobei R₁ ausgewählt ist aus der Gruppe, die aus H und R₅-CO-besteht, wobei R₅ ausgewählt ist aus der Gruppe, die aus C₁-C₁₈-Alkyl, C₂-C₂₄-Alkenyl, C₃-C₂₄-Cycloalkyl besteht; und R₂ -NR₃R₄ oder -OR₃ ist, wobei R₃ und R₄ unabhängig voneinander ausgewählt sind aus der Gruppe, die aus H und C₁-C₁₆-Alkyl besteht.

14. Verbindung der Formel (I),
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ihrer Stereoisomere und/oder ihrer kosmetisch oder pharmazeutisch unbedenklichen Salze, wobei:
AA₁ Glu ist; AA₂ Glu ist; AA₃ Met ist; AA₄ Gln ist; AA₅ Arg ist; AA₆ Arg ist; AA₇ ausgewählt ist aus der Gruppe, die aus Ala, Ser, Gly, Thr, Pro, Glu, Lys und Val besteht;
AA₈ ausgewählt ist aus der Gruppe, die aus Asp, Glu, Asn und Gln besteht; und
0, 1 oder 2 von AA₁, AA₂, AA₃, AA₄, AA₅ und AA₆ ersetzt sind, mit der Maßgabe, dass:
wenn AA₁ ersetzt ist, es durch eine Aminosäure ersetzt ist, die aus der Gruppe ausgewählt ist, die durch Asp, Gln und Asn gebildet wird;
wenn AA₂ ersetzt ist, es durch eine Aminosäure ersetzt ist, die aus der Gruppe ausgewählt ist, die durch Asp und Gln gebildet wird;
wenn AA₃ ersetzt ist, es durch eine Aminosäure ersetzt ist, die aus der Gruppe ausgewählt ist, die durch Leu und Ile gebildet wird;
wenn AA₄ ersetzt ist, es durch eine Aminosäure ersetzt ist, die aus der Gruppe ausgewählt ist, die durch Glu, Asn und Asp gebildet wird;
wenn AA₅ ersetzt ist, es durch Lys ersetzt ist; und
wenn AA₆ ersetzt ist, es durch eine Aminosäure ersetzt ist, die aus der Gruppe ausgewählt ist, die durch Lys und His gebildet wird;
a 1 ist und b 0 oder 1 ist;
W, X, Y und Z jeweils unabhängig eine Aminosäure sind;
m, n, p und q jeweils unabhängig 0 oder 1 sind;
m+n+p+q kleiner als oder gleich 2 ist;
R₁ aus der Gruppe ausgewählt ist, die aus H, einem von Polyethylenglycol abgeleiteten Polymer, einer nicht cyclischen aliphatischen Gruppe, Alicyclyl, Heterocyclyl, Heteroarylalkyl, Aryl, Aralkyl und R₅-CO- besteht, wobei R₅ aus der Gruppe ausgewählt ist, die aus H, einer nicht cyclischen aliphatischen Gruppe, Alicyclyl, Aryl, Aralkyl, Heterocyclyl und Heteroarylalkyl besteht;
R₂ aus der Gruppe ausgewählt ist, die aus -NR₃R₄, -OR₃, -SR₃ besteht, wobei R₃ und R₄ unabhängig aus einer Gruppe ausgewählt sind, die aus H, einem von Polyethylenglycol abgeleiteten Polymer, einer nicht cyclischen aliphatischen Gruppe, Alicyclyl, Heterocyclyl, Heteroarylalkyl, Aryl und Aralkyl besteht; und
R₁ und R₂ keine Aminosäuren sind,
zur Verwendung bei
der Vorbeugung oder Behandlung von atopischer Dermatitis, Kontaktdermatitis, Ekzemen, Schuppenflechte und/oder Akne vulgaris.

15. Verbindung zur Verwendung nach Anspruch 14, wobei die Verbindung R₁-Glu-Glu-Met-Gln-Arg-Arg-Ala-R₂ ist.

## Revendications

1. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gln ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gin ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne sont pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont le maintien et/ou l'amélioration de la fonction de barrière physique de la peau et de la fonction de barrière immunologique de la peau.

2. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (1),
ses stéréoisomères et/ou ses sels cosmétiquement ou pharmaceutiquement acceptables, où :
AA₁ représente Glu ; AA₂ représente Glu ; AA₃ représente Met ; AA₄ représente Gin ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gin ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont le maintien et/ou l'amélioration de la fonction de barrière physique de la peau.

3. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gin ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gin ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont la réduction ou la perte d'eau et d'électrolytes de la peau ; le maintien et/ou l'amélioration de l'humidification de la peau ; la prévention ou la réduction de la desquamation de la peau ; et/ou la prévention ou la réduction de la desquamation de la peau chez un sujet souffrant de peau sèche ou ayant une tendance à la peau sèche.

4. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gln ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gin ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont le maintien et/ou l'amélioration du microrelief de la peau ; et/ou le maintien et/ou l'amélioration de la douceur de la peau et/ou de l'aspect velouté de la peau.

5. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gin ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gin ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont la protection de la peau contre les risques environnementaux, y compris les irritants chimiques, les polluants et les agents pathogènes.

6. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gin ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gln ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont le maintien et/ou l'amélioration du mécanisme de défense immunitaire inné de la peau.

7. Utilisation d'un composé de formule (I)
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gin ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gln ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
dans le traitement cosmétique, non thérapeutique et/ou les soins cosmétiques, non thérapeutiques de la peau, des cheveux, des ongles et/ou des muqueuses, dans laquelle ledit traitement cosmétique, non thérapeutique et/ou lesdits soins cosmétiques, non thérapeutiques sont le maintien de l'équilibre microbien de la peau.

8. Procédé cosmétique, non thérapeutique de traitement et/ou de soins de la peau, des cheveux, des ongles et/ou des muqueuses d'un sujet comprenant l'administration d'une quantité cosmétiquement efficace d'un composé de formule (I),
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gln ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gln ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
au sujet, dans lequel le traitement et/ou les soins sont : le maintien et/ou l'amélioration de la fonction de barrière physique de la peau et de la fonction de barrière immunologique de la peau ; la réduction de la perte d'eau et d'électrolytes par la peau ; le maintien et/ou l'amélioration de l'hydratation de la peau, c'est-à-dire le maintien et/ou l'amélioration de l'humidification de la peau ; la prévention, le maintien et/ou l'amélioration du microrelief de la peau ; le maintien et/ou l'amélioration de la douceur de la peau et/ou de l'aspect velouté de la peau ; la protection de la peau contre les risques environnementaux, y compris les irritants chimiques, les polluants et les agents pathogènes ; le maintien et/ou l'amélioration du mécanisme de défense immunitaire inné de la peau ; et/ou le maintien de l'équilibre microbien de la peau.

9. Utilisation selon l'une quelconque des revendications 1 à 7 ou procédé selon la revendication 8, dans lequel 0 ou 1 des AA₁ à AA₆ est remplacé.

10. Utilisation selon l'une quelconque des revendications 1 à 7 ou 9, ou procédé selon la revendication 8 ou la revendication 9, dans lequel a = 1 et b = 0 et, de préférence, (i) lorsque 1 des AA₁ à AA₆ est remplacé, AA₁ est remplacé par Asp ; ou AA₄ est remplacé par Asp ; et (ii) lorsque 2 des AA₁ à AA₆ sont remplacés, AA₁ est remplacé par Asp et AA₄ est remplacé par Glu.

11. Utilisation selon l'une quelconque des revendications 1 à 7 ou 9, ou procédé selon la revendication 8 ou la revendication 9, dans lequel a = 1 et b = 1 et, de préférence, (i) lorsque 1 des AA₁ à AA₆ est remplacé, AA₁ est remplacé par Asp ou Gln ; ou AA₃ est remplacé par Leu ; ou AA₄ est remplacé par Glu, Asp ou Asn ; ou AA₆ est remplacé par Lys ou His.

12. Utilisation selon l'une quelconque des revendications 1 à 7 ou procédé selon la revendication 8, dans lequel le composé est R₁-Glu-Glu-Met-Gln-Arg-Arg-Ala-R₂.

13. Utilisation selon l'une quelconque des revendications 1 à 7 ou 9 à 12, ou procédé selon la revendication 8, la revendication 9, la revendication 10, la revendication 11 ou la revendication 12, dans lequel R₁ est choisi dans le groupe constitué par H et Rs-CO-, où R₅ est choisi dans le groupe constitué par alkyle en C₁ à C₁₈, alcényle en C₂ à C₂₄, cycloalkyle en C₃ à C₂₄ ; et R₂ est -NR₃R₄ ou -OR₃, où R₃ et R₄ sont choisis indépendamment dans le groupe constitué de H et alkyle en C₁ à C₁₆.

14. Composé de formule (I),
R₁-Wₘ-Xₙ-AA₁-AA₂-AA₃-AA₄-AA₅-AA₆-(AA₇)ₐ-(AA₈)_{b}-Yₚ-Z_{q}-R₂ (I),
ses stéréoisomères et/ou ses sels cosmétiquement ou pharmaceutiquement acceptables, où :
AA₁ représente Glu ; AA₂ représente Glu ; AA₃ représente Met ; AA₄ représente Gln ; AA₅ représente Arg ; AA₆ représente Arg ; AA₇ est choisi dans le groupe constitué par Ala, Ser, Gly, Thr, Pro, Glu, Lys et Val ;
AA₈ est choisi dans le groupe constitué par Asp, Glu, Asn et Gln ; et
0, 1 ou 2 de AA₁, AA₂, AA₃, AA₄, AA₅ et AA₆ sont remplacés à condition que :
lorsque AA₁ est remplacé, il est remplacé par un acide aminé choisi dans le groupe formé par Asp, Gln et Asn ;
lorsque AA₂ est remplacé, il est remplacé par un acide aminé choisi dans le groupe formé par Asp et Gln ;
lorsque AA₃ est remplacé, il est remplacé par un acide aminé choisi dans le groupe formé par Leu et Ile ;
lorsque AA₄ est remplacé, il est remplacé par un acide aminé choisi dans le groupe formé par Glu, Asn et Asp ;
lorsque AA₅ est remplacé, il est remplacé par Lys ; et
lorsque AA₆ est remplacé, il est remplacé par un acide aminé choisi dans le groupe formé par Lys et His ;
a représente 1 et b représente 0 ou 1 ;
W, X, Y et Z représentent chacun indépendamment un acide aminé ;
m, n, p et q représentent chacun indépendamment 0 ou 1 ;
m + n + p + q est inférieur ou égal à 2 ;
R₁ est choisi dans le groupe constitué par H, un polymère dérivé du polyéthylèneglycol, un groupe aliphatique non cyclique, alicyclyle, hétérocyclyle, hétéroarylalkyle, aryle, aralkyle et R₅-CO-, où R₅ est choisi dans le groupe constitué par H, un groupe aliphatique non cyclique, alicyclyle, aryle, aralkyle, hétérocyclyle et hétéroarylalkyle ;
R₂ est choisi dans le groupe constitué par -NR₃R₄, -OR₃, -SR₃, où R₃ et R₄ sont indépendamment choisis dans un groupe constitué par H, un polymère dérivé du polyéthylèneglycol, un groupe aliphatique non cyclique, alicyclyle, hétérocyclyle, hétéroarylalkyle, aryle et aralkyle ; et
R₁ et R₂ ne représentent pas des acides aminés,
à utiliser dans
la prévention ou le traitement de la dermatite atopique, de la dermatite de contact, de l'eczéma, du psoriasis et/ou de l'acné vulgaire.

15. Composé à utiliser selon la revendication 14, dans lequel le composé est R₁-Glu-Glu-Met-Gln-Arg-Arg-Ala-R₂.
